# EUROPEAN PATENT APPLICATION

(11) **EP 3 460 053 A1**
(43) Date of publication of application: **27.03.2019**
(21) Application number: 17306260.5
(22) Date of filing: 25.09.2017
(51) Int. Cl.: C12N 7/00, C07K 14/005

(54) **PORCINE ASTROVIRUSES AND THE USES THEREOF**

(71) Applicant: Ceva Santé Animale, 33500 Libourne (FR)
(72) Inventor: BOROS, Akos, 7633 PECS (HU); REUTER, Gabor Kamillo, 7632 PECS (HU); ALBERT, Mihaly, 1107 BUDAPEST (HU); PANKOVICS, Peter, 7673 KOVAGOSZOLOS (HU)
(74) Representative: Cabinet Becker et Associés

(57) **Abstract**

The present invention relates to a novel type of porcine astroviruses and the uses thereof. The invention also relates to parts and subunits and variants of said viruses, compositions comprising the same, their manufacture, and their uses e.g., for the design and production of vaccines for treating pigs. The invention also concerns methods for treating and/or preventing astrovirus-associated diseases, as well as methods and materials for detecting an infection.

SEQUENCE LISTING

<110>  CEVA SANTE ANIMALE

<120>  PORCINE ASTROVIRUSES AND THE USES THEREOF

<130>  B2589

<160>  25

<170>  PatentIn version 3.3

<210>  1
<211>  6393
<212>  DNA
<213>  Artificial

<220>
<223>  genomic DNA

<400>  1
ccaaatagag ctacccgttg gtccggccct atggcgttgc cattcgacga cactcttaaa      60

tttggctcag ccacggcccg tatgaagggt cgtcggttgg acatggttgc acgcaccaag     120

ttaaaagact tacttggtga tggtccttac tactatgact ttggacccct ggaagttgtc     180

gactcttctt ctaagcagtc aactgttaaa atgacttccg tgcaaactgt ttatgtgtcg     240

tcggttgttg aggacaactc ctacgtaacc tacaagtttg tgcctggcgt caatgattgg     300

gttgaaactg aacctgttct tcataaaccc acggctttgg ttggtgtcct atggcgtgag     360

tataaacgct acaaggatag aactgaaact cttacacaag agctttccca actcaggctt     420

gaacattcat tattgcgcca tgattatgaa cgtgtacgtc ctcagcaacc agtagctcct     480

aagggccggt tagggttcat cacaaagtta ctcattggct tacttattgg ctctctgttg     540

gctcactcta ctcttgcaca taagttacct gggactggct taatgggtga atgtttagac     600

actgatgtta ttgatggtaa gcaggtgtgt gtgaatttta tccctcgaaa tgataccgag     660

gctgactcac cagataaacc tgctaccact gtctgggatc ctgaaggtct tcttgagttg     720

cttattctag tggctcctta tttagtgtct tggcccatga ttgcaacaat ggttggcttt     780

ttctatgttg ctacagctga acagcctgcc tatatgctag tatctttact cttagcaaca     840

tactcaaaag cccaggtctt agcccttgct ggccttccct tcatggatat gccatcagtt     900

gtcacgctct gggtttctat gcttatccat tcatatgatg ctgttctttc attatgggtg     960

gctctgctcc tggccgcgtt catcttgtct gctggcgtct tcatgcccga tgtacgctac    1020

tctgagttgg tgcgtggtca ggtggtagtc ttccttgtcc ttgtcctgaa ctaccttgtt    1080

gtaatgatgt ctttgcccaa ctggcttgtc ttctcccttg tcgttggcta caggatactg    1140

cgtgttttaa catttcttgt ggctgagaaa gttgaagtcc gtggtcctga tggcaaggtt    1200

gtagagacac gtacttccat gcctgcatgg attaacaagg cgtcaaattt ctttcagact    1260

aggtttcatc aaaagataag gacaggtatc tcgcccacag cacgggtgat tccaaatggg 1320
gtcatcgtta ttgaaactca ggatggttca ggaacgggtt ttaggtgtcg aaactacctt 1380
gtcactgcag gacatgttgt gacaggtacg gaaacaccaa aaatccagtg ggctggtgtc 1440
actgcttact caaaagttgt ccatcgtgta tcagataaag atattgcttt tcttgccatt 1500
cctcaagaac ttcaagattt accttcttat cgcttggcta agaaggttga ggatggtccc 1560
gtggttatta catccctaga ggattcaggt gctctgtcag ttgctgtcac cgaaggtgtc 1620
atagtgactg attctataac ttatgccgtc cagacacgga atggcatgag tggttcacct 1680
gttactaacc ttgatggtag gattcttgcg gtgcatcaaa ccaacactgg tttcactggg 1740
ggcgctgtga tactatctga tgaagacttt ccacagccta agaaatcagc ccgtgaacaa 1800
caacttgagg cacgtattaa ggaacttgag gccgcaatga atcagtcttc tggccatgag 1860
gacattgtgg agcttgtgag agccgcagtg gcccgtgaat tcaaagtgct gcgcactgaa 1920
ctttctgcca ccacttttac tcaggcaaaa ggtaagaata agaaaaatca tcgtcggcgt 1980
ggtggtaaga aacgccgcgc tgtctggtca gaagaagaat ataaggaact cctagagaaa 2040
ggcttttcca aatcacaact acgtgagatg gctgaggttc ttagatctca tgaggaagac 2100
ccgtttggct ctgacactga atcggaaggg ggtttccccg agtggtccga tattagtgat 2160
actgagtcaa ttgaacggga gtggtttggc cagtcctggg aagactgtaa gcctgttaag 2220
gaagagcccc aggacacact tcctgtgcac cttaaagata aatacacctt ggatgcctat 2280
gtcattagta aggaagagtt gaaatctttt gccaaggagt tcaaagacta tgttgacaag 2340
gttgaagcat taattgataa aactgtccaa cagggtaagt ggttgccatc agttaatccc 2400
acagccataa tcgaggagct taatgactta tggtttggcc taaatatgtt gatgtgggag 2460
aaaggcttgg ttccttttac acaaaggaag aaaatcaagc gtaaggttca aaaaaactcc 2520
aagggggccc ccaagagggg cccattacct actccatgat ggacctcaaa gcatggcggc 2580
gctccctgtg tgaggttgag cgccgcttag taccagaggg ttacccgctt attggtgggg 2640
tgcctctgga caggcctatc tctgattggg atgaacctat tgatgactta ctcctaatgt 2700
taccagcatt taaagagaac cttaactaca ctcctgctgt ctgggggcct gaggcctatg 2760
ctaaatcatt tgataagttc acttatagag agccttgccc taacatagcc gccacgtatc 2820
cacgtgagtg gtcttttgca acaaaagctt tgaagaggga gtactctttc cttgatggct 2880
ctgtcctgac cgatattact gccacatcca aaaatgctga ctccacccct gcttatccta 2940
agtctcttta ctggaaaact gaagcagact atcttggtga gcggggctat caggattaca 3000
ttaagcaatt ggacgccatc tactctgggg aacgcccaga tgttttatgg tacctcttct 3060
tgaagaagga aatcctgaaa gtggaaaaag tcgagcaggc cgacattaga caaattgttt 3120
gttctgaccc tatatttgca cgtataggtt gtcactttga ggaggatcaa aacatcagga 3180

tgaagaacca tactaggaca cggtctggtc agtgtggctg gtcgcctttc tttggtggct 3240

tcaatgacag gatcaaacgc ttatgcgctt gtgaccctga ttgttacata gaatttgatt 3300

ggacacgctt tgatggtact atacctaatg aagtgtttat gcacatcaag aaagtccgct 3360

actctttctt cgcgaaggag tttaggacga agcgtgttaa agaagcctac cagtggtact 3420

gcgaacagct cctgtacagg cacgtcgtgc tcccttcggg tgaggtaaca ttgcagaccc 3480

gtggaaaccc ctctggacaa atctccacca ctatggacaa caacatggtg aatgtttttc 3540

ttcaagcatt tgagtacgcc tacttacacc cagattgtac agatgaggaa ctcgaggcaa 3600

attggtctcg tatggacacc ctggtttatg gtgatgatag attatccgca tatattggct 3660

tgcccgccaa ttatgtggag aaggttgtaa taatgtactc aaatgttttt ggtatgtggg 3720

tgaaaccaga aaaagttatc gtttccccca gcattatagg tctcaccttc tgtggtctca 3780

gaattaccag cgacggtacc atctttttac ctgttccagc agagactaac aagcttattt 3840

ctgcgctcat taagccaacc aagaaacttc aagatgtgat gaccctctat gggaaactcc 3900

tttgctaccg catccttggc cacaacctcc ctgatgacaa caagtttaag gcctacattc 3960

tggtggcctt ggaggttgtt gctaggcaca tcagaacacg ttgtggtgac gagccactcc 4020

gctttactga tgagatgctt gaccggcttt ggagggaggg accaaaagtc ggatatggct 4080

ggtgataagc ccaatgcgtc tgccaaagcg gcacctcttg ctaaggaggt tgccaaggag 4140

gttgttaagg aggagaagag aaatcaggca cgtagtcgga agtggcgtaa gcctcgccga 4200

cagcaaaatc aacctcaaca atcaaaacaa cgagccgttg tgactaggca agttacaaag 4260

aaattaaagc aagagggctt ggaaggacca cgaccccgct tttctgtacg agtgtcagct 4320

accattggaa agattgggcc caataaaaaa caggggcccg aacttcaaat ttcaactttc 4380

ttacgcccat ctcttatgaa ggaaccaaat gatggcacca attttgggcc tttgcaagca 4440

gcctcagcac agtggggtct gtggcgtctt gcatacttaa agatacgctg tacaccctta 4500

gttgggccat cggcggttac aggctcagtc tataggcttt ccctaaatct cactcagtcg 4560

cccggcaata cctcttgggg tggcctaggt gcacgcaaac atattgatgt accagtggga 4620

aagcaatcag cttggagcct tcacaggggc gaccttgctg gccctcgcca aacctggtgg 4680

atgactgaca ctaatgaaga aggaggccaa tcttgtggac ctatggttga gattcatggc 4740

ttgggaaaga cttcctccac ttataaagac gccgactggg ttggcgattt gtttatagtg 4800

gaggtggtag gacgttggga gtttaccaat tacaatgcta agcccgccct cggcacctta 4860

gatcgtgtga ccgaggatgt ccaagccggc attgaggttg gttcagatgg gatcatgact 4920

atgacccttc ctcaacgctc caacctagct aagcacatgt caacagctta tgaaaaatta 4980

ccattaaatg cccttacagt gggtgagacc atttggcaga tagttgatga aggtgctggc 5040

cttatctcat ctgcagtacc tccgcctttt ggctggctca ttaaaggtgg ctggtggttt 5100

gttaagaagg ccattggtag gtctaataca gatgagacct attatgttta tgcctctctt 5160

gcagatgcgc aaaacaataa acctgttgag gcgactccct tctcacgtat acctgtccaa 5220

accaccattg catccaccca gattaatgcg cctaacactg ggccctcctc ttctctggtc 5280

atttccacgc cttctggctt gttaccagag gatcttccat ctactgctac cacctttgag 5340

gtcgttggct atgtgtcagc tatgttttca gttaatcaga ccacaccgct ttctccagtt 5400

attaataatg tgttcccagt tctggggcag agtgcaagtg attttgcatt ttccatcatg 5460

caaaattcta cacagggggc tgggaaaggc atgggttgcc taatctttca gtccgagccg 5520

gtgttcctta ggctaaatct agatacatca tcatctacag ttgataacat cttcttgctt 5580

gatgataccc ctggtattgg ctggactgtt aacttcaatc ttccagctag tccaatcacc 5640

aactgtgcag atgtcttggc gtacaagcat gaaaaatggg gtgataacct caagttttca 5700

tcttggcttg tccgctttac aaaagtaatc cctgagtcag ctggcaacta ctattgtctt 5760

gtttctgctc cagttatttc agctgtgaac caggctccag ttactatagt ctttgataaa 5820

gtggcaactc caaagttgtc tacttcttct tcattgtcac agatgcgtgt ttctatctca 5880

tctgtgcagc ctggtacgta tggtgttatc tatacccttg gcagttataa tgtgactgat 5940

gccactggag tagctactgc accgaccctg acaggccccg ttaatgacag ggtggtgcct 6000

ggcccctttc gtgcagtaac tgggattaca cctatctcgc tcacaaccca gctgccgaca 6060

tcttttcctt tgtgggcaaa ctttttcagg ccggcttccc ggccgagcat gattgacact 6120

cttatggagc aaatccaaca gaggtttggt ctgaagccaa ttgaacctga ctataattca 6180

gatgatgaga gcgatgtaga gcctacagct gcttctaaat ttgccaagca tggcatgtat 6240

gatgctctcc gtgattctga ctgggatcac gttgatgccg acgctcttat ccatttcatt 6300

tcaaaggcat ccaatcgccc ccgaggccac gccgagtagg aacgagggta caggggctca 6360

gatctctttt aaattgatct ttctctttca ggc 6393

<210> 2
<211> 6393
<212> DNA
<213> Artificial

<220>
<223> genomic DNA

<400> 2
ccaaatagag ctacccgttg gtccggccct atggcgttgc cattcgacga cactcttaaa 60

tttggctcag ccacggcccg tatgaagggt cgtcggttgg acatggttgc acgcaccaag 120

ttaaaagact tacttggtga tggtccttac tactatgact ttggacccct ggaagttgtc 180

gactcttctt ctaagcagtc aactgttaaa atgacttccg tgcaaactgt ttatgtgtcg 240

tcggttgttg aggacaactc ctacgtaacc tacaagtttg tgcctggcgt caatgattgg 300

gttgaaactg aacctgttct tcataaaccc acggctttgg ttggtgtcct atggcgtgag 360

tataaacgct acaaggatag aactgaaact cttacacaag agctttccca actcaagctt 420

gaacattcat tattgcgcca tgattatgaa cgtgtacgtc ctcagcaacc agtagctcct 480

aagggccggt tagggttcat cacaaagtta ctcattggct tacttattgg ctctctgttg 540

gctcactcta ctcttgcaca taagttacct gggactggct taatgggtga atgtttagac 600

actgatgtta ttgatggtaa gcaggtgtgt gtgaatttta tccctcgaaa tgataccgag 660

gctgactcac cagataaacc tgctaccact gcctgggatc ctgaaggtct tcttgagttg 720

cttattctag tggctcctta tttagtgtct tggcccatga ttgcaacaat ggttggcttt 780

ttctatgttg ctacagctga acagcctgcc tatatgctag tatctttact cttagcaaca 840

tactcaaaag cccaggtctt agcccttgct ggccttccct tcatggatat gccatcagtt 900

gtcacgctct gggtttctat gcttatccat tcatatgatg ctgttctttc attatgggtg 960

gctctgctcc tggccgcatt catcttgtct gctggcgtct tcatgcccga tgtacgctac 1020

tctgagttgg tgcgtggtca ggtggtagtc ttccttgtcc ttgtcctgaa ctaccttgtt 1080

gtaatgatgt ctttgcccaa ctggcttgtc ttctcccttg tcgttggcta caggatactg 1140

cgtgttttaa catttcttgt ggctgagaaa gttgaagtcc gtggtcctga tggcaaggtt 1200

gtagagacac gtacttccat gcctgcatgg attaacaagg cgtcaaattt ctttcagact 1260

aggtttcatc aaaagataag gacaggtatc tcgcccacag cacgggtgat tccaaatggg 1320

gtcatcgtta ttgaaactca ggatggttca ggaacgggtt ttaggtgtcg aaactacctt 1380

gtcactgcag gacatgttgt gacaggtacg gaaacaccaa aaatccagtg ggctggtgtc 1440

actgcttact caaaagttgt ccatcgtgta tcagataaag atattgcttt tcttgctatt 1500

cctcaagaac ttcaagattt accttcttac cgcttggcta agaaggttga ggatggtccc 1560

gtggttatta catccctaga ggattcaggt gctctgtcag ttgctgtcac cgaaggtgtc 1620

atagtgactg attctataac ttatgccgtc cagacacgga atggcatgag tggttcacct 1680

gttactaacc ttgatggtag gattcttgca gtgcatcaaa ccaacactgg tttcactggg 1740

ggcgctgtga tactatctga tgaagacttt ccacagccta agaaatcagc ccgtgaacaa 1800

caacttgagg cacgtattaa ggaacttgag gccgcaatga atcagtcttc tggccatgag 1860

gacattgtgg agcttgtgag agccgcagtg gcccgtgaat tcaaagtgct gcgcactgaa 1920

ctttctgcca ccacttttac tcaggcaaaa ggcaagaata agaaaaatca tcgtcggcgt 1980

ggtggtaaga aacgccgcgc tgtctggtca gaagaagaat ataaggaact cctagagaaa 2040

ggcttttcca aatcacaact acgtgaaatg gctgaggttc ttaggtctca tgaggaagac 2100

ccgtttggct ctgacactga atcggaaggg ggtttccccg agtggtccga tattagtgat 2160

actgagtcaa ttgaacggga gtggtttggc cagtcctggg aagactgtaa gcctgttaag 2220

gaagagcccc aggacacact tcctgtgcac cttaaagata aatacacctt ggatgcctat 2280

gtcattagta aggaagagtt gaaatctttt gccaaggagt tcaaagacta tgttgacaag 2340

gttgaagcac taattgataa aactgtccaa cagggtaagt ggttgccatc agttaatccc 2400

acagccataa tcgaggagct taatgactta tggtttggcc taaatatctt gatgtgggag 2460

aaaggcttgg ttccttttac acaaaggaag aaaatcaagc gtaaggttca aaaaaactcc 2520

aagggggccc ccaagagggg cccattacct actccatgat ggacctcaaa gcatggcggc 2580

gctccctgtg tgaggttgag cgccgcttag taccagaggg ttacccgctt attggtgggg 2640

tgcctctgga caggcctatc tctgattggg atgaacctat tgatgactta ctcctaatgt 2700

taccagcatt taaagagaat cttaactaca ctcctgctgt ttgggggcct gaggcctatg 2760

ctaaatcatt tgataagttc acttatagag agccttgccc taacatagcc gccacgtatc 2820

cacgtgagtg gtcttttgca acaaaagctt tgaagaggga gtactctttc cttgatggct 2880

ctgtcctgac cgatattact gccacatcca aaaatgctga ctccacccct gcttatccta 2940

agtctcttta ctggaaaact gaagcagact atcttggtga gcggggctat caggattaca 3000

ttaagcaatt ggacgccatc tactctgggg aacgcccaga tgttttatgg tacctcttct 3060

tgaagaagga aatcctgaaa gtggaaaaag tcgagcaggc cgacattaga caaattgttt 3120

gttctgaccc tatatttgca cgtataggtt gtcactttga ggaggatcaa aacatcagga 3180

tgaagaacca tactaggaca cggtctggtc agtgtggctg gtcgcctttc tttggtggct 3240

tcaatgacag gatcaaacgc ttatgcgctt gtgaccctga ttgttacata gaatttgatt 3300

ggacacgctt tgatggtact atacctaatg aagtgtttat gcacatcaag aaagtccgtt 3360

actctttctt cgcgaaggag tttaggacga agcgtgttaa agaagcctac cagtggtact 3420

gcgaacagct cctgtacagg cacgtcgtgc tcccttcggg tgaggtaaca ttgcagaccc 3480

gtggaaaccc ctctggacaa atctccacca ctatggacaa caacatggtg aatgtttttc 3540

ttcaagcatt tgagtacgcc tacttacacc cagattgtac agatgaggaa ctcgaggcaa 3600

attggtctcg tatggatacc ctggtttatg gtgatgatag attatccgca tatattggct 3660

tgcccgccaa ttatgtggag aaggttgtaa taatgtactc aaatgttttt ggtatgtggg 3720

tgaaaccaga aaaagttatc gtttccccca gcattatagg tctcaccttc tgtggtctca 3780

gaattaccag tgacggtacc atctttttac ctgttccagc agagactaac aagcttattt 3840

ctgcgctcat taagccaacc aagaaacttc aagatgtgat gaccctctat gggaaactcc 3900

tttgctaccg catccttggc cacaacctcc ctgatgacaa caagtttaag gcctacattc 3960

tggtggcctt ggaggttgtt gctaggcaca tcagaacacg ttgtggtgac gagccactcc 4020

gctttactga tgagatgctt gaccggcttt ggaggggagg accaaaagtc ggatatggct 4080
ggtgataagc ccaatgcgtc tgccaaagcg gcacctcttg ctaaggaggt tgccaaggag 4140
gttgttaagg aggagaagag aaatcaggca cgtagtcgaa agtggcgtaa gcctcgccga 4200
cagcaaaatc aacctcaaca atcaaaacaa cgagccgttg tgactaggca agttacaaag 4260
aaattaaagc aagagggctt ggaaggacca cgaccccgct tttctgtgcg agtgtcagct 4320
accattggaa agattgggcc caataaagaa caggggcctg aacttcaaat ttcaactttc 4380
ttacatccat ctcttatgaa ggaaccaaat gatggcacca attttgggcc tttgcaagca 4440
gcctcagcac agtggggtct gtggcgtctt gcagacttaa agatatgctg tacaccctta 4500
gttgggccat cggcagttac aggctcagtc tataggcttt ccctaaatct cactcaatca 4560
cccggcaaca cctcttgggg tggcctaggt gcacgcaaac atattgatgt accagtggga 4620
aagcaatcaa cttggagcct tcacaggggc gaccttgctg gccctcgcca aacctggtgg 4680
atgactgaca ctaatgaaga aggaggccaa tcttgtggac ctatggttga gattcatggc 4740
ttgggaaaga cttcctccac ttataaagac gccgactggg ttggcgattt gtttatagtg 4800
gaggtggtag gacgttggga gtttaccaat tacaatgcta agcccgccct cggcacctta 4860
gatcgtgtga ccgaggatgt tcaagccggc attgaggttg gttcagatgg gatcatgact 4920
atgacccttc ctcaacgctc caacctagct aagcacatgt caacagctta tgaaaaatta 4980
ccattaaatg cccttacagt gggtgagacc atttggcaga tagttgatga aggtgctggc 5040
cttatctcat ctgcagtacc tccgcctttt ggctggctca ttaaaggtgg ctggtggttc 5100
gttaagaagg ccattggtag gtctaataca gatgagacct attatgttta tgcctctctt 5160
gcagatgcgc aaaacaataa acctgttgag gcgactccct tctcacgtat acctgtccaa 5220
accaccattg catccaccca gattaatgcg cctaacactg ggccctcctc ttctctggtc 5280
atttccacgc cttctggctt gttaccagag gatcttccat ctactgctac cacctttgag 5340
gttgttggct atgtgtcagc tatgttttca gtcaatcaga ccacaccgct ttctccagtt 5400
attaacaatg tgttcccagt tctggggcag agtgcaagtg attttgcatt ttccatcatg 5460
caaaattcta cacagggggc tgggaaaggc atgggttgcc taatttttca gtccgagccg 5520
gtgttcctta ggctaaatct agatacatca tcatctacag ttgataacat cttcttgctt 5580
gatgataccc ctggtattgg ctggactgtt aacttcaatc ttccagctag tccaatcacc 5640
aactgtgcag atgtcttggc gtacaagcat gaaaaatggg gtgacaacct caagttttca 5700
tcttggcttg tccgctttac aaaagtaatc cctgagtcag ctggcaacta ctattgcctt 5760
gtttctgctc cagttatttc agctgtgaac caggctccag ttactatagt ctttgataaa 5820
gtggcaactc caaagttgtc tacttcttct tcattgtcac acatgcgtgt ttctatctca 5880

tctgtgcagc ctggtacgta tggtgttatc tatacccttg gcagttataa tgtgactgat 5940

gccactggag tagctactgc accgaccctg acaggccccg ttaatgacag ggtggtgcct 6000

ggcccctttc gtgcagtaac tgggattaca cctatctcgc tcacaaccca gctgccgaca 6060

tcttttcctt tgtgggcaaa ctttttcagg ccggcttccc ggccgagcat gattgacact 6120

cttatggagc aaatccaaca gaggtttggt ctgaagccaa ttgaacctga ctataattca 6180

gatgatgaga gcgatgtaga gcctacagct gcttctaaat ttgccaagca tagcatgtat 6240

gatgctctcc gtgattctga ctgggatcac gttgatgccg acgctcttat ccatttcatt 6300

tcaaaggcat ccaatcgccc ccgaggccac gccgagtagg aacgagggta caggggctca 6360

gatctctttt aaattgatct ttctctttca ggc 6393

<210> 3
<211> 6394
<212> DNA
<213> Artificial

<220>
<223> genomic DNA

<400> 3
ccaaatagag ctacccgttg gtccggccct atggcgttgc cattcgacga cactcttaaa 60

tttggctcag ccacggcccg tatgaagggt cgtcggttgg acatggttgc acgcaccaag 120

ttaaaagact tacttggtga tggtccttac tactatgact ttggacccct ggaagttgtc 180

gactcttctt ctaagcagtc aactgttaaa atgacttccg tgcaaactgt ttatgtgtcg 240

tcggttgttg aggacaactc ctacgtaacc tacaagtttg tgcctggcgt caatgattgg 300

gttgaaactg aacctgttct tcataaaccc acggctttgg ttggtgtcct atggcgtgag 360

tataaacgct acaaggatag aactgaaact cttacacaag agctttccca actcaagctt 420

gaacattcat tattgcgcca tgattatgaa cgtgtacgtc ctcagcaacc aatagctcct 480

aagggccggt tagggttcat cacaaagtta ctcattggct tacttattgg ctctctgttg 540

gctcactcta ctcttgcaca taagttacct gggactggct taatgggtga atgtttagac 600

actgatgtta ttgatggtaa gcaggtgtgt gtgaatttta tccctcgaaa tgataccgag 660

gctgactcac aagataaacc tgctaccact gtctgggatc ctgaaggtct tcttgagttg 720

cttattctag tggctcctta tttagtgtct tggcccatga ttgcaacaat ggttggcttt 780

ttctatgttg ctacagctga acagcctgcc tatatgctag tatctttact cttagcaaca 840

tactcaaaag cccaggtctt agcccttgct ggccttccct tcatggatat gccatcagtt 900

gtcacgctct gggtttctat gcttatccat tcatatgatg ctgttctttc attatgggtg 960

gctctgctcc tggccgcgtt catcttgtct gctggcgtct tcatgcccga tgtacgctac 1020

tctgagttgg tgcgtggtca ggtggtagtc ttccttgtcc ttgtcctgaa ctaccttgtt 1080

gtaatgatgt ctttgcccaa ctggcttgtc ttctcccttg tcgttggcta caggatactg 1140
cgtgttttaa catttcttgt ggctgagaaa gttgaagtcc gtggtcctga tggcaaggtt 1200
gtagagacac gtacttccat gcctgcatgg attaacaagg cgtcaaattt ctttcagact 1260
aggtttcatc aaaagataag gacaggtatc tcgcccacag cacgggtgat tccaaatggg 1320
gtcatcgtta ttgaaactca ggatggttca ggaacgggtt ttaggtgtcg aaactacctt 1380
gtcactgcag gacatgttgt gacaggtacg gaaacaccaa aaatccagtg ggctggtgtc 1440
actgcttact caaaagttgt ccatcgtgta tcagataaag atattgcttt tcttgccatt 1500
cctcaagaac ttcaagattt accttcttac cgcttggcta agaaggttga ggatggtccc 1560
gtggttatta catccctaga ggattcaggt gctctgtcag ttgctgtcac cgaaggtgtc 1620
atagtgactg attctataac ttatgccgtc cagacacgga atggcatgag tggttcacct 1680
gttactaacc ttgatggtag gattcttgca gtgcatcaaa ccaacactgg tttcactggg 1740
ggcgctgtga tactatctga tgaagacttt ccacagccta agaaatcagc ccgtgaacaa 1800
caacttgagg cacgtattaa ggaacttgag gccgcaatga atcagtcttc tggccatgag 1860
gacattgtgg agcttgtgag agccgcagtg gcccgtgaat tcaaagtgct gcgcactgaa 1920
ctttctgcca ccacttttac tcaggcaaaa ggcaagaata agaaaaatca tcgtcggcgt 1980
ggtggtaaga aacgccgcgc tgtctggtca gaagaagaat ataaggaact cctagagaaa 2040
ggcttttcca aatcacaact acgtgaaatg gctgaggttc ttagatctca tgaggaagac 2100
ccgtttggct ctgacactga ctcggaaggg ggtttccccg agtggtccga tattagtgat 2160
actgagtcaa ttgaacggga gtggtttggc cagtcctggg aagactgtaa gcctgttaag 2220
gaagagcccc aggacacact tcctgtgcac cttaaagata aatacacctt ggatgcctat 2280
gtcattagta aggaagagtt gaaatctttt gccaaggagt tcaaagacta tgttgacaag 2340
gttgaagcat taattgataa aactgtccaa cagggtaagt ggttgccatc agttaatccc 2400
acagccataa tcgaggagct taatgactta tggtttggcc taaatatgtt gatgtgggag 2460
aaaggcttgg ttccttttac acaaaggaag aaaatcaagc gtaaggttca aaaaaactcc 2520
aagggggccc ccaagagggg cccattacct actccatgat ggacctcaaa gcatggcggc 2580
gctccctgtg tgaggttgag cgccgcttag taccagaggg ttacccgctt attggtgggg 2640
tgcctctgga caggcctatc tctgattggg atgaacctat tgatgactta ctcctaatgt 2700
taccagcatt taaagagaat cttaactaca ctcctgctgt ttgggggcct gaggcctatg 2760
ctaaatcatt tgataagttc acttatagag agccttgccc taacatagcc gccacgtatc 2820
cacgtgagtg gtcttttgca acaaaagctt tgaagaggga gtactctttc cttgatggct 2880
ctgtcctgac cgatattact gccacatcca aaaatgctga ctccacccct gcttatccta 2940

agtctcttta ctggaaaact gaagcagact atcttggtga gcggggctat caggattaca 3000
ttaagcaatt ggacgccatc tactctgggg aacgcccaga tgttttatgg tacctcttct 3060
tgaagaagga aatcctgaaa gtggaaaaag tcgagcaggc cgacattaga caaattgttt 3120
gttctgaccc tatatttgca cgtataggtt gtcactttga ggaggatcaa aacatcagga 3180
tgaagaacca tactaggaca cggtctggtc agtgtggctg gtcgcctttc tttggtggct 3240
tcaatgacag gatcaaacgc ttatgcgctt gtgaccctga ttgttacata gaatttgatt 3300
ggacacgctt tgatggtact atacctaatg aagtgtttat gcacatcaag aaagtccgct 3360
actctttctt cgcgaaggag tttaggacga agcgtgttaa agaagcctac cagtggtact 3420
gcgaacagct cctgtacagg cacgtcgtgc tcccttcggg tgaggtaaca ttgcagaccc 3480
gtggaaaccc ctctggacaa atctccacca ctatggacaa caacatggta aatgtttttc 3540
ttcaagcatt tgagtacgcc tacttacacc cagattgtac agatgaggaa ctcgaggcaa 3600
attggtctcg tatggatacc ctggtttatg gtgatgatag attatccgca tatattggct 3660
tgcccgccaa ttatgtggag aaggttgtaa taatgtactc aaatgttttt ggtatgtggg 3720
tgaaaccaga aaaagttatc gtttccccca gcattatagg tctcaccttc tgtggtctca 3780
gaattaccag cgacggtacc atctttttac ctgttccagc agagactaac aagcttattt 3840
ctgcgctcat taagccaacc aagaaacttc aagatgtgat gaccctctat gggaaactcc 3900
tttgctaccg catccttggc cacaacctcc ctgatgacaa caagtttaag gcctatattc 3960
tggtggcctt ggaggttgtt gctaggcaca tcagaacacg ttgtggtgac gagccactcc 4020
gctttactga tgagatgctt gaccggcttt ggaggggagg accaaaagtc ggatatggct 4080
ggtgataagc ccaatgcgtc tgccaaagcg gcacctcttg ctaaggaggt tgccaaggag 4140
gttgttaagg aggagaagag aaatcaggca cgtagtcgga agtggcgtaa gcctcgccga 4200
cagcaaaatc aacctcaaca atcaaaacaa cgagccgttg tgactaggca agttacaaag 4260
aaattaaagc aagagggctt ggaaggacca cgaccccgct tttctgtacg agtgtcagct 4320
accattggaa agattgggcc caataaagaa caggggcccg aacttcaaat ttcaactttc 4380
ttacacccat ctcttatgaa ggaaccaaat gatggcacca attttgggcc tttgcaagca 4440
gcctcagcac agtggggtct gtggcgtctt gcagacttaa agatacgctg tacaccctta 4500
gttgggccat cggcagttac aggctcagtc tataggcttt ccctaaatct cactcaatca 4560
cccggcaata cctcttgggg tggcctaggt gcacgcaaac atattgatgt accagtggga 4620
aagcaatcaa cttggagcct tcacaggggc gaccttgctg gccctcgcca aacctggtgg 4680
atgactgaca ctaatgaaga aggaggccaa tcttgtggac ctatggttga gattcatggc 4740
ttgggaaaga cttcctccac ttataaagac gccgactggg ttggcgattt gtttatagtg 4800
gaggtggtag gacgttggga gtttaccaat tacaatgcta agcccgccct cggcacctta 4860

gatcgtgtga ccgaggatgt ccaagccggc attgaggttg gttcagatgg gatcatgact     4920

atgacccttc ctcaacgctc caacctagct aagcacatgt caacagctta tgaaaaatta     4980

ccattaaatg cccttacagt gggtgagacc atttggcaga tagttgatga aggtgctggc     5040

cttatctcat ctgcagtacc tccgcctttt ggctggctca ttaaaggtgg ctggtggttt     5100

gttaagaagg ccattggtag gtctaataca gatgagacct attatgttta tgcctctctt     5160

gcagatgcgc aaaacaataa acctgttgag gcgactccct tctcacgtat acctgtccaa     5220

accaccattg catccaccca gattaatgcg cctaacactg ggccctcctc ttctctggtc     5280

atttccacgc cttctggctt gttaccagag gatcttccat ctactgctac cacctttgag     5340

gtcgttggct atgtgtcagc tatgttttca gttaatcaga ccacaccgct ttctccagtt     5400

attaataatg tgttcccagt tctggggcag agtgcaagtg attttgcatt ttccatcatg     5460

caaaattcta cacagggggc tgggaaaggc atgggttgcc taatctttca gtccgagccg     5520

gtgttcctta ggctaaatct agatacatca tcatctacag ttgataacat cttcttgctt     5580

gatgataccc ctggtattgg ctggactgtt aacttcaatc ttccagctag tccaatcacc     5640

aactgtgcag atgtcttggc gtacaagcat gaaaaatggg gtgataacct caagttttca     5700

tcttggcttg tccgcttaac aaaagtaatc cctgagtcag ctggcaacta ctattgcctt     5760

gttcctgctc cagttatttc acctgtgtac caggctccag ttactatagt ctttgataaa     5820

gtggcaactc caaagttgtc tacttcttct tcattgtcac agatgcgtgt ttctatctca     5880

tctgtgcagc ctggtacgta tggtgttatc tatacccttg gcagttataa tgtgactgat     5940

gccactggag tagctactgc accgaccctg acaggccccg ttaatgacag ggtggtgcct     6000

ggcccctttc gtgcagtaac tgggattaca cctatctcgc tcacaaccca gctgccgaca     6060

tcttttcctt tgtgggcaaa ctttttcagg ccggcttccc ggccgagcat gattgacact     6120

cttatggagc aaatccaaca gaggtttggt ctgaagccaa ttgaacctga ctataattca     6180

gatgatgaga gcgatgtaga gcctacagct gcttctaaat ttgccaagca tagcatgtat     6240

gatgctctcc gtgattctga ctgggatcac gttgatgccg acgctcttat ccatttcatt     6300

tcaaaggcat ccaatcgccc ccgaggccac gccgagtagg aacgagggta caggggctca     6360

gatctctttc aaattgatct ttctcttttc aggc                                 6394

<210>  4
<211>  2265
<212>  DNA
<213>  Artificial

<220>
<223>  ORF2 gene

<400>  4

atggctggtg ataagcccaa tgcgtctgcc aaagcggcac ctcttgctaa ggaggttgcc 60
aaggaggttg ttaaggagga gaagagaaat caggcacgta gtcggaagtg gcgtaagcct 120
cgccgacagc aaaatcaacc tcaacaatca aaacaacgag ccgttgtgac taggcaagtt 180
acaaagaaat taaagcaaga gggcttggaa ggaccacgac cccgcttttc tgtacgagtg 240
tcagctacca ttggaaagat tgggcccaat aaaaaacagg ggcccgaact tcaaatttca 300
actttcttac gcccatctct tatgaaggaa ccaaatgatg gcaccaattt tgggcctttg 360
caagcagcct cagcacagtg gggtctgtgg cgtcttgcat acttaaagat acgctgtaca 420
cccttagttg ggccatcggc ggttacaggc tcagtctata ggctttccct aaatctcact 480
cagtcgcccg gcaatacctc ttggggtggc ctaggtgcac gcaaacatat tgatgtacca 540
gtgggaaagc aatcagcttg gagccttcac aggggcgacc ttgctggccc tcgccaaacc 600
tggtggatga ctgacactaa tgaagaagga ggccaatctt gtggacctat ggttgagatt 660
catggcttgg gaaagacttc ctccacttat aaagacgccg actgggttgg cgatttgttt 720
atagtggagg tggtaggacg ttgggagttt accaattaca atgctaagcc cgccctcggc 780
accttagatc gtgtgaccga ggatgtccaa gccggcattg aggttggttc agatgggatc 840
atgactatga cccttcctca acgctccaac ctagctaagc acatgtcaac agcttatgaa 900
aaattaccat taaatgccct tacagtgggt gagaccattt ggcagatagt tgatgaaggt 960
gctggcctta tctcatctgc agtacctccg ccttttggct ggctcattaa aggtggctgg 1020
tggtttgtta agaaggccat tggtaggtct aatacagatg agacctatta tgtttatgcc 1080
tctcttgcag atgcgcaaaa caataaacct gttgaggcga ctcccttctc acgtatacct 1140
gtccaaacca ccattgcatc cacccagatt aatgcgccta acactgggcc ctcctcttct 1200
ctggtcattt ccacgccttc tggcttgtta ccagaggatc ttccatctac tgctaccacc 1260
tttgaggtcg ttggctatgt gtcagctatg ttttcagtta atcagaccac accgctttct 1320
ccagttatta ataatgtgtt cccagttctg gggcagagtg caagtgattt tgcattttcc 1380
atcatgcaaa attctacaca gggggctggg aaaggcatgg gttgcctaat ctttcagtcc 1440
gagccggtgt tccttaggct aaatctagat acatcatcat ctacagttga taacatcttc 1500
ttgcttgatg atacccctgg tattggctgg actgttaact tcaatcttcc agctagtcca 1560
atcaccaact gtgcagatgt cttggcgtac aagcatgaaa aatggggtga taacctcaag 1620
ttttcatctt ggcttgtccg ctttacaaaa gtaatccctg agtcagctgg caactactat 1680
tgtcttgttt ctgctccagt tatttcagct gtgaaccagg ctccagttac tatagtcttt 1740
gataaagtgg caactccaaa gttgtctact tcttcttcat tgtcacagat gcgtgtttct 1800
atctcatctg tgcagcctgg tacgtatggt gttatctata cccttggcag ttataatgtg 1860
actgatgcca ctggagtagc tactgcaccg accctgacag gccccgttaa tgacagggtg 1920

gtgcctggcc cctttcgtgc agtaactggg attacaccta tctcgctcac aacccagctg 1980

ccgacatctt ttcctttgtg ggcaaacttt ttcaggccgg cttcccggcc gagcatgatt 2040

gacactctta tggagcaaat ccaacagagg tttggtctga agccaattga acctgactat 2100

aattcagatg atgagagcga tgtagagcct acagctgctt ctaaatttgc caagcatggc 2160

atgtatgatg ctctccgtga ttctgactgg gatcacgttg atgccgacgc tcttatccat 2220

ttcatttcaa aggcatccaa tcgcccccga ggccacgccg agtag 2265

<210> 5
<211> 754
<212> PRT
<213> Artificial

<220>
<223> ORF2 polypeptide

<400> 5

Met Ala Gly Asp Lys Pro Asn Ala Ser Ala Lys Ala Ala Pro Leu Ala
1               5                   10                  15

Lys Glu Val Ala Lys Glu Val Val Lys Glu Glu Lys Arg Asn Gln Ala
            20                  25                  30

Arg Ser Arg Lys Trp Arg Lys Pro Arg Arg Gln Gln Asn Gln Pro Gln
        35                  40                  45

Gln Ser Lys Gln Arg Ala Val Val Thr Arg Gln Val Thr Lys Lys Leu
    50                  55                  60

Lys Gln Glu Gly Leu Glu Gly Pro Arg Pro Arg Phe Ser Val Arg Val
65                  70                  75                  80

Ser Ala Thr Ile Gly Lys Ile Gly Pro Asn Lys Lys Gln Gly Pro Glu
                85                  90                  95

Leu Gln Ile Ser Thr Phe Leu Arg Pro Ser Leu Met Lys Glu Pro Asn
            100                 105                 110

Asp Gly Thr Asn Phe Gly Pro Leu Gln Ala Ala Ser Ala Gln Trp Gly
        115                 120                 125

Leu Trp Arg Leu Ala Tyr Leu Lys Ile Arg Cys Thr Pro Leu Val Gly
    130                 135                 140

Pro Ser Ala Val Thr Gly Ser Val Tyr Arg Leu Ser Leu Asn Leu Thr
145                 150                 155                 160

Gln Ser Pro Gly Asn Thr Ser Trp Gly Gly Leu Gly Ala Arg Lys His
                165                 170                 175

Ile Asp Val Pro Val Gly Lys Gln Ser Ala Trp Ser Leu His Arg Gly
            180                 185                 190

Asp Leu Ala Gly Pro Arg Gln Thr Trp Trp Met Thr Asp Thr Asn Glu
        195                 200                 205

Glu Gly Gly Gln Ser Cys Gly Pro Met Val Glu Ile His Gly Leu Gly
    210                 215                 220

Lys Thr Ser Ser Thr Tyr Lys Asp Ala Asp Trp Val Gly Asp Leu Phe
225                 230                 235                 240

Ile Val Glu Val Val Gly Arg Trp Glu Phe Thr Asn Tyr Asn Ala Lys
                245                 250                 255

Pro Ala Leu Gly Thr Leu Asp Arg Val Thr Glu Asp Val Gln Ala Gly
            260                 265                 270

Ile Glu Val Gly Ser Asp Gly Ile Met Thr Met Thr Leu Pro Gln Arg
        275                 280                 285

Ser Asn Leu Ala Lys His Met Ser Thr Ala Tyr Glu Lys Leu Pro Leu
    290                 295                 300

Asn Ala Leu Thr Val Gly Glu Thr Ile Trp Gln Ile Val Asp Glu Gly
305                 310                 315                 320

Ala Gly Leu Ile Ser Ser Ala Val Pro Pro Pro Phe Gly Trp Leu Ile
                325                 330                 335

Lys Gly Gly Trp Trp Phe Val Lys Lys Ala Ile Gly Arg Ser Asn Thr
            340                 345                 350

Asp Glu Thr Tyr Tyr Val Tyr Ala Ser Leu Ala Asp Ala Gln Asn Asn
        355                 360                 365

Lys Pro Val Glu Ala Thr Pro Phe Ser Arg Ile Pro Val Gln Thr Thr
    370                 375                 380

Ile Ala Ser Thr Gln Ile Asn Ala Pro Asn Thr Gly Pro Ser Ser Ser
385                 390                 395                 400

Leu Val Ile Ser Thr Pro Ser Gly Leu Leu Pro Glu Asp Leu Pro Ser
                405                 410                 415

Thr Ala Thr Thr Phe Glu Val Val Gly Tyr Val Ser Ala Met Phe Ser
        420                 425                 430

Val Asn Gln Thr Thr Pro Leu Ser Pro Val Ile Asn Asn Val Phe Pro
    435                 440                 445

Val Leu Gly Gln Ser Ala Ser Asp Phe Ala Phe Ser Ile Met Gln Asn
    450                 455                 460

Ser Thr Gln Gly Ala Gly Lys Gly Met Gly Cys Leu Ile Phe Gln Ser
465                 470                 475                 480

Glu Pro Val Phe Leu Arg Leu Asn Leu Asp Thr Ser Ser Ser Thr Val
                485                 490                 495

Asp Asn Ile Phe Leu Leu Asp Asp Thr Pro Gly Ile Gly Trp Thr Val
            500                 505                 510

Asn Phe Asn Leu Pro Ala Ser Pro Ile Thr Asn Cys Ala Asp Val Leu
        515                 520                 525

Ala Tyr Lys His Glu Lys Trp Gly Asp Asn Leu Lys Phe Ser Ser Trp
    530                 535                 540

Leu Val Arg Phe Thr Lys Val Ile Pro Glu Ser Ala Gly Asn Tyr Tyr
545                 550                 555                 560

Cys Leu Val Ser Ala Pro Val Ile Ser Ala Val Asn Gln Ala Pro Val
                565                 570                 575

Thr Ile Val Phe Asp Lys Val Ala Thr Pro Lys Leu Ser Thr Ser Ser
            580                 585                 590

Ser Leu Ser Gln Met Arg Val Ser Ile Ser Ser Val Gln Pro Gly Thr
        595                 600                 605

Tyr Gly Val Ile Tyr Thr Leu Gly Ser Tyr Asn Val Thr Asp Ala Thr
    610                 615                 620

Gly Val Ala Thr Ala Pro Thr Leu Thr Gly Pro Val Asn Asp Arg Val
625                 630                 635                 640

Val Pro Gly Pro Phe Arg Ala Val Thr Gly Ile Thr Pro Ile Ser Leu
                645                 650                 655

Thr Thr Gln Leu Pro Thr Ser Phe Pro Leu Trp Ala Asn Phe Phe Arg

660                 665                 670

Pro Ala Ser Arg Pro Ser Met Ile Asp Thr Leu Met Glu Gln Ile Gln
        675                 680                 685

Gln Arg Phe Gly Leu Lys Pro Ile Glu Pro Asp Tyr Asn Ser Asp Asp
    690                 695                 700

Glu Ser Asp Val Glu Pro Thr Ala Ala Ser Lys Phe Ala Lys His Gly
705                 710                 715                 720

Met Tyr Asp Ala Leu Arg Asp Ser Asp Trp Asp His Val Asp Ala Asp
                725                 730                 735

Ala Leu Ile His Phe Ile Ser Lys Ala Ser Asn Arg Pro Arg Gly His
            740                 745                 750

Ala Glu

<210>  6
<211>  754
<212>  PRT
<213>  Artificial

<220>
<223>  ORF2 polypeptide

<400>  6

Met Ala Gly Asp Lys Pro Asn Ala Ser Ala Lys Ala Ala Pro Leu Ala
1               5                   10                  15

Lys Glu Val Ala Lys Glu Val Val Lys Glu Glu Lys Arg Asn Gln Ala
            20                  25                  30

Arg Ser Arg Lys Trp Arg Lys Pro Arg Arg Gln Gln Asn Gln Pro Gln
        35                  40                  45

Gln Ser Lys Gln Arg Ala Val Val Thr Arg Gln Val Thr Lys Lys Leu
    50                  55                  60

Lys Gln Glu Gly Leu Glu Gly Pro Arg Pro Arg Phe Ser Val Arg Val
65                  70                  75                  80

Ser Ala Thr Ile Gly Lys Ile Gly Pro Asn Lys Glu Gln Gly Pro Glu
                85                  90                  95

Leu Gln Ile Ser Thr Phe Leu His Pro Ser Leu Met Lys Glu Pro Asn
            100                 105                 110

Asp Gly Thr Asn Phe Gly Pro Leu Gln Ala Ala Ser Ala Gln Trp Gly
        115                 120                 125

Leu Trp Arg Leu Ala Asp Leu Lys Ile Arg Cys Thr Pro Leu Val Gly
    130                 135                 140

Pro Ser Ala Val Thr Gly Ser Val Tyr Arg Leu Ser Leu Asn Leu Thr
145                 150                 155                 160

Gln Ser Pro Gly Asn Thr Ser Trp Gly Gly Leu Gly Ala Arg Lys His
                165                 170                 175

Ile Asp Val Pro Val Gly Lys Gln Ser Thr Trp Ser Leu His Arg Gly
            180                 185                 190

Asp Leu Ala Gly Pro Arg Gln Thr Trp Trp Met Thr Asp Thr Asn Glu
        195                 200                 205

Glu Gly Gly Gln Ser Cys Gly Pro Met Val Glu Ile His Gly Leu Gly
    210                 215                 220

Lys Thr Ser Ser Thr Tyr Lys Asp Ala Asp Trp Val Gly Asp Leu Phe
225                 230                 235                 240

Ile Val Glu Val Val Gly Arg Trp Glu Phe Thr Asn Tyr Asn Ala Lys
                245                 250                 255

Pro Ala Leu Gly Thr Leu Asp Arg Val Thr Glu Asp Val Gln Ala Gly
            260                 265                 270

Ile Glu Val Gly Ser Asp Gly Ile Met Thr Met Thr Leu Pro Gln Arg
        275                 280                 285

Ser Asn Leu Ala Lys His Met Ser Thr Ala Tyr Glu Lys Leu Pro Leu
    290                 295                 300

Asn Ala Leu Thr Val Gly Glu Thr Ile Trp Gln Ile Val Asp Glu Gly
305                 310                 315                 320

Ala Gly Leu Ile Ser Ser Ala Val Pro Pro Phe Gly Trp Leu Ile
                325                 330                 335

Lys Gly Gly Trp Trp Phe Val Lys Lys Ala Ile Gly Arg Ser Asn Thr
                340                 345                 350

Asp Glu Thr Tyr Tyr Val Tyr Ala Ser Leu Ala Asp Ala Gln Asn Asn

            355                 360                 365

Lys Pro Val Glu Ala Thr Pro Phe Ser Arg Ile Pro Val Gln Thr Thr
        370                 375                 380

Ile Ala Ser Thr Gln Ile Asn Ala Pro Asn Thr Gly Pro Ser Ser Ser
385 390 395 400

Leu Val Ile Ser Thr Pro Ser Gly Leu Leu Pro Glu Asp Leu Pro Ser
405 410 415

Thr Ala Thr Thr Phe Glu Val Val Gly Tyr Val Ser Ala Met Phe Ser
                420                 425                 430

Val Asn Gln Thr Thr Pro Leu Ser Pro Val Ile Asn Asn Val Phe Pro
            435                 440                 445

Val Leu Gly Gln Ser Ala Ser Asp Phe Ala Phe Ser Ile Met Gln Asn
        450                 455                 460

Ser Thr Gln Gly Ala Gly Lys Gly Met Gly Cys Leu Ile Phe Gln Ser
465 470 475 480

Glu Pro Val Phe Leu Arg Leu Asn Leu Asp Thr Ser Ser Ser Thr Val
485 490 495

Asp Asn Ile Phe Leu Leu Asp Asp Thr Pro Gly Ile Gly Trp Thr Val
                500                 505                 510

Asn Phe Asn Leu Pro Ala Ser Pro Ile Thr Asn Cys Ala Asp Val Leu
            515                 520                 525

Ala Tyr Lys His Glu Lys Trp Gly Asp Asn Leu Lys Phe Ser Ser Trp
        530                 535                 540

Leu Val Arg Phe Thr Lys Val Ile Pro Glu Ser Ala Gly Asn Tyr Tyr
545 550 555 560

Cys Leu Val Ser Ala Pro Val Ile Ser Ala Val Asn Gln Ala Pro Val
565 570 575

Thr Ile Val Phe Asp Lys Val Ala Thr Pro Lys Leu Ser Thr Ser Ser
                580                 585                 590

Ser Leu Ser Gln Met Arg Val Ser Ile Ser Ser Val Gln Pro Gly Thr
            595                 600                 605

Tyr Gly Val Ile Tyr Thr Leu Gly Ser Tyr Asn Val Thr Asp Ala Thr
    610                 615                 620

Gly Val Ala Thr Ala Pro Thr Leu Thr Gly Pro Val Asn Asp Arg Val
625                 630                 635                 640

Val Pro Gly Pro Phe Arg Ala Val Thr Gly Ile Thr Pro Ile Ser Leu
                645                 650                 655

Thr Thr Gln Leu Pro Thr Ser Phe Pro Leu Trp Ala Asn Phe Phe Arg
            660                 665                 670

Pro Ala Ser Arg Pro Ser Met Ile Asp Thr Leu Met Glu Gln Ile Gln
        675                 680                 685

Gln Arg Phe Gly Leu Lys Pro Ile Glu Pro Asp Tyr Asn Ser Asp Asp
    690                 695                 700

Glu Ser Asp Val Glu Pro Thr Ala Ala Ser Lys Phe Ala Lys His Gly
705                 710                 715                 720

Met Tyr Asp Ala Leu Arg Asp Ser Asp Trp Asp His Val Asp Ala Asp
                725                 730                 735

Ala Leu Ile His Phe Ile Ser Lys Ala Ser Asn Arg Pro Arg Gly His
            740                 745                 750

Ala Glu

<210>  7
<211>  754
<212>  PRT
<213>  Artificial

<220>
<223>  ORF2 polypeptide

<400>  7

Met Ala Gly Asp Lys Pro Asn Ala Ser Ala Lys Ala Ala Pro Leu Ala
1               5                   10                  15

Lys Glu Val Ala Lys Glu Val Val Lys Glu Glu Lys Arg Asn Gln Ala
            20                  25                  30

Arg Ser Arg Lys Trp Arg Lys Pro Arg Arg Gln Gln Asn Gln Pro Gln
        35                  40                  45

Gln Ser Lys Gln Arg Ala Val Val Thr Arg Gln Val Thr Lys Lys Leu

50                  55                  60

Lys Gln Glu Gly Leu Glu Gly Pro Arg Pro Arg Phe Ser Val Arg Val
65                  70                  75                  80

Ser Ala Thr Ile Gly Lys Ile Gly Pro Asn Lys Glu Gln Gly Pro Glu
                85                  90                  95

Leu Gln Ile Ser Thr Phe Leu His Pro Ser Leu Met Lys Glu Pro Asn
            100                 105                 110

Asp Gly Thr Asn Phe Gly Pro Leu Gln Ala Ala Ser Ala Gln Trp Gly
        115                 120                 125

Leu Trp Arg Leu Ala Asp Leu Lys Ile Cys Cys Thr Pro Leu Val Gly
    130                 135                 140

Pro Ser Ala Val Thr Gly Ser Val Tyr Arg Leu Ser Leu Asn Leu Thr
145                 150                 155                 160

Gln Ser Pro Gly Asn Thr Ser Trp Gly Gly Leu Gly Ala Arg Lys His
                165                 170                 175

Ile Asp Val Pro Val Gly Lys Gln Ser Thr Trp Ser Leu His Arg Gly
            180                 185                 190

Asp Leu Ala Gly Pro Arg Gln Thr Trp Trp Met Thr Asp Thr Asn Glu
        195                 200                 205

Glu Gly Gly Gln Ser Cys Gly Pro Met Val Glu Ile His Gly Leu Gly
    210                 215                 220

Lys Thr Ser Ser Thr Tyr Lys Asp Ala Asp Trp Val Gly Asp Leu Phe
225                 230                 235                 240

Ile Val Glu Val Val Gly Arg Trp Glu Phe Thr Asn Tyr Asn Ala Lys
                245                 250                 255

Pro Ala Leu Gly Thr Leu Asp Arg Val Thr Glu Asp Val Gln Ala Gly
            260                 265                 270

Ile Glu Val Gly Ser Asp Gly Ile Met Thr Met Thr Leu Pro Gln Arg
        275                 280                 285

Ser Asn Leu Ala Lys His Met Ser Thr Ala Tyr Glu Lys Leu Pro Leu
    290                 295                 300

Asn Ala Leu Thr Val Gly Glu Thr Ile Trp Gln Ile Val Asp Glu Gly
305                 310                 315                 320

Ala Gly Leu Ile Ser Ser Ala Val Pro Pro Pro Phe Gly Trp Leu Ile
                325                 330                 335

Lys Gly Gly Trp Trp Phe Val Lys Lys Ala Ile Gly Arg Ser Asn Thr
            340                 345                 350

Asp Glu Thr Tyr Tyr Val Tyr Ala Ser Leu Ala Asp Ala Gln Asn Asn
        355                 360                 365

Lys Pro Val Glu Ala Thr Pro Phe Ser Arg Ile Pro Val Gln Thr Thr
    370                 375                 380

Ile Ala Ser Thr Gln Ile Asn Ala Pro Asn Thr Gly Pro Ser Ser Ser
385                 390                 395                 400

Leu Val Ile Ser Thr Pro Ser Gly Leu Leu Pro Glu Asp Leu Pro Ser
                405                 410                 415

Thr Ala Thr Thr Phe Glu Val Val Gly Tyr Val Ser Ala Met Phe Ser
            420                 425                 430

Val Asn Gln Thr Thr Pro Leu Ser Pro Val Ile Asn Asn Val Phe Pro
        435                 440                 445

Val Leu Gly Gln Ser Ala Ser Asp Phe Ala Phe Ser Ile Met Gln Asn
    450                 455                 460

Ser Thr Gln Gly Ala Gly Lys Gly Met Gly Cys Leu Ile Phe Gln Ser
465                 470                 475                 480

Glu Pro Val Phe Leu Arg Leu Asn Leu Asp Thr Ser Ser Ser Thr Val
                485                 490                 495

Asp Asn Ile Phe Leu Leu Asp Asp Thr Pro Gly Ile Gly Trp Thr Val
            500                 505                 510

Asn Phe Asn Leu Pro Ala Ser Pro Ile Thr Asn Cys Ala Asp Val Leu
        515                 520                 525

Ala Tyr Lys His Glu Lys Trp Gly Asp Asn Leu Lys Phe Ser Ser Trp
    530                 535                 540

Leu Val Arg Phe Thr Lys Val Ile Pro Glu Ser Ala Gly Asn Tyr Tyr
545                 550                 555                 560

Cys Leu Val Ser Ala Pro Val Ile Ser Ala Val Asn Gln Ala Pro Val
                565                 570                 575

Thr Ile Val Phe Asp Lys Val Ala Thr Pro Lys Leu Ser Thr Ser Ser
            580                 585                 590

Ser Leu Ser His Met Arg Val Ser Ile Ser Ser Val Gln Pro Gly Thr
        595                 600                 605

Tyr Gly Val Ile Tyr Thr Leu Gly Ser Tyr Asn Val Thr Asp Ala Thr
    610                 615                 620

Gly Val Ala Thr Ala Pro Thr Leu Thr Gly Pro Val Asn Asp Arg Val
625                 630                 635                 640

Val Pro Gly Pro Phe Arg Ala Val Thr Gly Ile Thr Pro Ile Ser Leu
                645                 650                 655

Thr Thr Gln Leu Pro Thr Ser Phe Pro Leu Trp Ala Asn Phe Phe Arg
            660                 665                 670

Pro Ala Ser Arg Pro Ser Met Ile Asp Thr Leu Met Glu Gln Ile Gln
        675                 680                 685

Gln Arg Phe Gly Leu Lys Pro Ile Glu Pro Asp Tyr Asn Ser Asp Asp
    690                 695                 700

Glu Ser Asp Val Glu Pro Thr Ala Ala Ser Lys Phe Ala Lys His Ser
705                 710                 715                 720

Met Tyr Asp Ala Leu Arg Asp Ser Asp Trp Asp His Val Asp Ala Asp
                725                 730                 735

Ala Leu Ile His Phe Ile Ser Lys Ala Ser Asn Arg Pro Arg Gly His
            740                 745                 750

Ala Glu

<210>  8
<211>  754
<212>  PRT
<213>  Artificial

<220>
<223>  ORF2 polypeptide

<400>  8

Met Ala Gly Asp Lys Pro Asn Ala Ser Ala Lys Ala Ala Pro Leu Ala
1               5                   10                  15

Lys Glu Val Ala Lys Glu Val Val Lys Glu Glu Lys Arg Asn Gln Ala
            20                  25                  30

Arg Ser Arg Lys Trp Arg Lys Pro Arg Arg Gln Gln Asn Gln Pro Gln
        35                  40                  45

Gln Ser Lys Gln Arg Ala Val Val Thr Arg Gln Val Thr Lys Lys Leu
    50                  55                  60

Lys Gln Glu Gly Leu Glu Gly Pro Arg Pro Arg Phe Ser Val Arg Val
65                  70                  75                  80

Ser Ala Thr Ile Gly Lys Ile Gly Pro Asn Lys Glu Gln Gly Pro Glu
                85                  90                  95

Leu Gln Ile Ser Thr Phe Leu His Pro Ser Leu Met Lys Glu Pro Asn
            100                 105                 110

Asp Gly Thr Asn Phe Gly Pro Leu Gln Ala Ala Ser Ala Gln Trp Gly
        115                 120                 125

Leu Trp Arg Leu Ala Asp Leu Lys Ile Arg Cys Thr Pro Leu Val Gly
    130                 135                 140

Pro Ser Ala Val Thr Gly Ser Val Tyr Arg Leu Ser Leu Asn Leu Thr
145                 150                 155                 160

Gln Ser Pro Gly Asn Thr Ser Trp Gly Gly Leu Gly Ala Arg Lys His
                165                 170                 175

Ile Asp Val Pro Val Gly Lys Gln Ser Thr Trp Ser Leu His Arg Gly
            180                 185                 190

Asp Leu Ala Gly Pro Arg Gln Thr Trp Trp Met Thr Asp Thr Asn Glu
        195                 200                 205

Glu Gly Gly Gln Ser Cys Gly Pro Met Val Glu Ile His Gly Leu Gly
    210                 215                 220

Lys Thr Ser Ser Thr Tyr Lys Asp Ala Asp Trp Val Gly Asp Leu Phe
225                 230                 235                 240

Ile Val Glu Val Val Gly Arg Trp Glu Phe Thr Asn Tyr Asn Ala Lys
                245                 250                 255

Pro Ala Leu Gly Thr Leu Asp Arg Val Thr Glu Asp Val Gln Ala Gly
        260                 265                 270

Ile Glu Val Gly Ser Asp Gly Ile Met Thr Met Thr Leu Pro Gln Arg
    275                 280                 285

Ser Asn Leu Ala Lys His Met Ser Thr Ala Tyr Glu Lys Leu Pro Leu
    290                 295                 300

Asn Ala Leu Thr Val Gly Glu Thr Ile Trp Gln Ile Val Asp Glu Gly
305                 310                 315                 320

Ala Gly Leu Ile Ser Ser Ala Val Pro Pro Pro Phe Gly Trp Leu Ile
                325                 330                 335

Lys Gly Gly Trp Trp Phe Val Lys Lys Ala Ile Gly Arg Ser Asn Thr
            340                 345                 350

Asp Glu Thr Tyr Tyr Val Tyr Ala Ser Leu Ala Asp Ala Gln Asn Asn
        355                 360                 365

Lys Pro Val Glu Ala Thr Pro Phe Ser Arg Ile Pro Val Gln Thr Thr
    370                 375                 380

Ile Ala Ser Thr Gln Ile Asn Ala Pro Asn Thr Gly Pro Ser Ser Ser
385                 390                 395                 400

Leu Val Ile Ser Thr Pro Ser Gly Leu Leu Pro Glu Asp Leu Pro Ser
                405                 410                 415

Thr Ala Thr Thr Phe Glu Val Val Gly Tyr Val Ser Ala Met Phe Ser
            420                 425                 430

Val Asn Gln Thr Thr Pro Leu Ser Pro Val Ile Asn Asn Val Phe Pro
        435                 440                 445

Val Leu Gly Gln Ser Ala Ser Asp Phe Ala Phe Ser Ile Met Gln Asn
    450                 455                 460

Ser Thr Gln Gly Ala Gly Lys Gly Met Gly Cys Leu Ile Phe Gln Ser
465                 470                 475                 480

Glu Pro Val Phe Leu Arg Leu Asn Leu Asp Thr Ser Ser Ser Thr Val
                485                 490                 495

Asp Asn Ile Phe Leu Leu Asp Asp Thr Pro Gly Ile Gly Trp Thr Val
            500                 505                 510

Asn Phe Asn Leu Pro Ala Ser Pro Ile Thr Asn Cys Ala Asp Val Leu
        515                 520                 525

Ala Tyr Lys His Glu Lys Trp Gly Asp Asn Leu Lys Phe Ser Ser Trp
    530                 535                 540

Leu Val Arg Leu Thr Lys Val Ile Pro Glu Ser Ala Gly Asn Tyr Tyr
545                 550                 555                 560

Cys Leu Val Pro Ala Pro Val Ile Ser Pro Val Tyr Gln Ala Pro Val
                565                 570                 575

Thr Ile Val Phe Asp Lys Val Ala Thr Pro Lys Leu Ser Thr Ser Ser
            580                 585                 590

Ser Leu Ser Gln Met Arg Val Ser Ile Ser Ser Val Gln Pro Gly Thr
        595                 600                 605

Tyr Gly Val Ile Tyr Thr Leu Gly Ser Tyr Asn Val Thr Asp Ala Thr
    610                 615                 620

Gly Val Ala Thr Ala Pro Thr Leu Thr Gly Pro Val Asn Asp Arg Val
625                 630                 635                 640

Val Pro Gly Pro Phe Arg Ala Val Thr Gly Ile Thr Pro Ile Ser Leu
                645                 650                 655

Thr Thr Gln Leu Pro Thr Ser Phe Pro Leu Trp Ala Asn Phe Phe Arg
            660                 665                 670

Pro Ala Ser Arg Pro Ser Met Ile Asp Thr Leu Met Glu Gln Ile Gln
        675                 680                 685

Gln Arg Phe Gly Leu Lys Pro Ile Glu Pro Asp Tyr Asn Ser Asp Asp
    690                 695                 700

Glu Ser Asp Val Glu Pro Thr Ala Ala Ser Lys Phe Ala Lys His Ser
705                 710                 715                 720

Met Tyr Asp Ala Leu Arg Asp Ser Asp Trp Asp His Val Asp Ala Asp
                725                 730                 735

Ala Leu Ile His Phe Ile Ser Lys Ala Ser Asn Arg Pro Arg Gly His
            740                 745                 750

Ala Glu

<210>  9
<211>  842
<212>  PRT
<213>  Artificial

<220>
<223>  ORF1a polypeptide

<400>  9

Met Ala Leu Pro Phe Asp Asp Thr Leu Lys Phe Gly Ser Ala Thr Ala
1               5                   10                  15

Arg Met Lys Gly Arg Arg Leu Asp Met Val Ala Arg Thr Lys Leu Lys
            20                  25                  30

Asp Leu Leu Gly Asp Gly Pro Tyr Tyr Tyr Asp Phe Gly Pro Leu Glu
        35                  40                  45

Val Val Asp Ser Ser Ser Lys Gln Ser Thr Val Lys Met Thr Ser Val
    50                  55                  60

Gln Thr Val Tyr Val Ser Ser Val Val Glu Asp Asn Ser Tyr Val Thr
65                  70                  75                  80

Tyr Lys Phe Val Pro Gly Val Asn Asp Trp Val Glu Thr Glu Pro Val
                85                  90                  95

Leu His Lys Pro Thr Ala Leu Val Gly Val Leu Trp Arg Glu Tyr Lys
            100                 105                 110

Arg Tyr Lys Asp Arg Thr Glu Thr Leu Thr Gln Glu Leu Ser Gln Leu
        115                 120                 125

Arg Leu Glu His Ser Leu Leu Arg His Asp Tyr Glu Arg Val Arg Pro
    130                 135                 140

Gln Gln Pro Val Ala Pro Lys Gly Arg Leu Gly Phe Ile Thr Lys Leu
145                 150                 155                 160

Leu Ile Gly Leu Leu Ile Gly Ser Leu Leu Ala His Ser Thr Leu Ala
                165                 170                 175

His Lys Leu Pro Gly Thr Gly Leu Met Gly Glu Cys Leu Asp Thr Asp
            180                 185                 190

Val Ile Asp Gly Lys Gln Val Cys Val Asn Phe Ile Pro Arg Asn Asp
        195                 200                 205

Thr Glu Ala Asp Ser Pro Asp Lys Pro Ala Thr Thr Val Trp Asp Pro
    210                 215                 220

Glu Gly Leu Leu Glu Leu Leu Ile Leu Val Ala Pro Tyr Leu Val Ser
225                 230                 235                 240

Trp Pro Met Ile Ala Thr Met Val Gly Phe Phe Tyr Val Ala Thr Ala
                245                 250                 255

Glu Gln Pro Ala Tyr Met Leu Val Ser Leu Leu Leu Ala Thr Tyr Ser
            260                 265                 270

Lys Ala Gln Val Leu Ala Leu Ala Gly Leu Pro Phe Met Asp Met Pro
        275                 280                 285

Ser Val Val Thr Leu Trp Val Ser Met Leu Ile His Ser Tyr Asp Ala
    290                 295                 300

Val Leu Ser Leu Trp Val Ala Leu Leu Leu Ala Ala Phe Ile Leu Ser
305                 310                 315                 320

Ala Gly Val Phe Met Pro Asp Val Arg Tyr Ser Glu Leu Val Arg Gly
                325                 330                 335

Gln Val Val Val Phe Leu Val Leu Val Leu Asn Tyr Leu Val Val Met
            340                 345                 350

Met Ser Leu Pro Asn Trp Leu Val Phe Ser Leu Val Val Gly Tyr Arg
        355                 360                 365

Ile Leu Arg Val Leu Thr Phe Leu Val Ala Glu Lys Val Glu Val Arg
    370                 375                 380

Gly Pro Asp Gly Lys Val Val Glu Thr Arg Thr Ser Met Pro Ala Trp
385                 390                 395                 400

Ile Asn Lys Ala Ser Asn Phe Phe Gln Thr Arg Phe His Gln Lys Ile
                405                 410                 415

Arg Thr Gly Ile Ser Pro Thr Ala Arg Val Ile Pro Asn Gly Val Ile
            420                 425                 430

Val Ile Glu Thr Gln Asp Gly Ser Gly Thr Gly Phe Arg Cys Arg Asn
        435                 440                 445

Tyr Leu Val Thr Ala Gly His Val Val Thr Gly Thr Glu Thr Pro Lys

450                 455                 460

Ile Gln Trp Ala Gly Val Thr Ala Tyr Ser Lys Val Val His Arg Val
465                 470                 475                 480

Ser Asp Lys Asp Ile Ala Phe Leu Ala Ile Pro Gln Glu Leu Gln Asp
                485                 490                 495

Leu Pro Ser Tyr Arg Leu Ala Lys Lys Val Glu Asp Gly Pro Val Val
            500                 505                 510

Ile Thr Ser Leu Glu Asp Ser Gly Ala Leu Ser Val Ala Val Thr Glu
        515                 520                 525

Gly Val Ile Val Thr Asp Ser Ile Thr Tyr Ala Val Gln Thr Arg Asn
    530                 535                 540

Gly Met Ser Gly Ser Pro Val Thr Asn Leu Asp Gly Arg Ile Leu Ala
545                 550                 555                 560

Val His Gln Thr Asn Thr Gly Phe Thr Gly Gly Ala Val Ile Leu Ser
                565                 570                 575

Asp Glu Asp Phe Pro Gln Pro Lys Lys Ser Ala Arg Glu Gln Gln Leu
            580                 585                 590

Glu Ala Arg Ile Lys Glu Leu Glu Ala Ala Met Asn Gln Ser Ser Gly
        595                 600                 605

His Glu Asp Ile Val Glu Leu Val Arg Ala Ala Val Ala Arg Glu Phe
    610                 615                 620

Lys Val Leu Arg Thr Glu Leu Ser Ala Thr Thr Phe Thr Gln Ala Lys
625                 630                 635                 640

Gly Lys Asn Lys Lys Asn His Arg Arg Arg Gly Gly Lys Lys Arg Arg
                645                 650                 655

Ala Val Trp Ser Glu Glu Glu Tyr Lys Glu Leu Leu Glu Lys Gly Phe
            660                 665                 670

Ser Lys Ser Gln Leu Arg Glu Met Ala Glu Val Leu Arg Ser His Glu
        675                 680                 685

Glu Asp Pro Phe Gly Ser Asp Thr Glu Ser Glu Gly Gly Phe Pro Glu
    690                 695                 700

Trp Ser Asp Ile Ser Asp Thr Glu Ser Ile Glu Arg Glu Trp Phe Gly
705                 710                 715                 720

Gln Ser Trp Glu Asp Cys Lys Pro Val Lys Glu Glu Pro Gln Asp Thr
                725                 730                 735

Leu Pro Val His Leu Lys Asp Lys Tyr Thr Leu Asp Ala Tyr Val Ile
            740                 745                 750

Ser Lys Glu Glu Leu Lys Ser Phe Ala Lys Glu Phe Lys Asp Tyr Val
        755                 760                 765

Asp Lys Val Glu Ala Leu Ile Asp Lys Thr Val Gln Gln Gly Lys Trp
    770                 775                 780

Leu Pro Ser Val Asn Pro Thr Ala Ile Ile Glu Glu Leu Asn Asp Leu
785                 790                 795                 800

Trp Phe Gly Leu Asn Met Leu Met Trp Glu Lys Gly Leu Val Pro Phe
                805                 810                 815

Thr Gln Arg Lys Lys Ile Lys Arg Lys Val Gln Lys Asn Ser Lys Gly
            820                 825                 830

Ala Pro Lys Arg Gly Pro Leu Pro Thr Pro
        835                 840

<210>  10
<211>  842
<212>  PRT
<213>  Artificial

<220>
<223>  ORF1a polypeptide

<400>  10

Met Ala Leu Pro Phe Asp Asp Thr Leu Lys Phe Gly Ser Ala Thr Ala
1               5                   10                  15

Arg Met Lys Gly Arg Arg Leu Asp Met Val Ala Arg Thr Lys Leu Lys
            20                  25                  30

Asp Leu Leu Gly Asp Gly Pro Tyr Tyr Tyr Asp Phe Gly Pro Leu Glu
        35                  40                  45

Val Val Asp Ser Ser Ser Lys Gln Ser Thr Val Lys Met Thr Ser Val
    50                  55                  60

Gln Thr Val Tyr Val Ser Ser Val Val Glu Asp Asn Ser Tyr Val Thr

65                  70                  75                  80

Tyr Lys Phe Val Pro Gly Val Asn Asp Trp Val Glu Thr Glu Pro Val
                85                  90                  95

Leu His Lys Pro Thr Ala Leu Val Gly Val Leu Trp Arg Glu Tyr Lys
            100                 105                 110

Arg Tyr Lys Asp Arg Thr Glu Thr Leu Thr Gln Glu Leu Ser Gln Leu
        115                 120                 125

Lys Leu Glu His Ser Leu Leu Arg His Asp Tyr Glu Arg Val Arg Pro
    130                 135                 140

Gln Gln Pro Val Ala Pro Lys Gly Arg Leu Gly Phe Ile Thr Lys Leu
145                 150                 155                 160

Leu Ile Gly Leu Leu Ile Gly Ser Leu Leu Ala His Ser Thr Leu Ala
                165                 170                 175

His Lys Leu Pro Gly Thr Gly Leu Met Gly Glu Cys Leu Asp Thr Asp
            180                 185                 190

Val Ile Asp Gly Lys Gln Val Cys Val Asn Phe Ile Pro Arg Asn Asp
        195                 200                 205

Thr Glu Ala Asp Ser Pro Asp Lys Pro Ala Thr Thr Ala Trp Asp Pro
    210                 215                 220

Glu Gly Leu Leu Glu Leu Leu Ile Leu Val Ala Pro Tyr Leu Val Ser
225                 230                 235                 240

Trp Pro Met Ile Ala Thr Met Val Gly Phe Phe Tyr Val Ala Thr Ala
                245                 250                 255

Glu Gln Pro Ala Tyr Met Leu Val Ser Leu Leu Leu Ala Thr Tyr Ser
            260                 265                 270

Lys Ala Gln Val Leu Ala Leu Ala Gly Leu Pro Phe Met Asp Met Pro
        275                 280                 285

Ser Val Val Thr Leu Trp Val Ser Met Leu Ile His Ser Tyr Asp Ala
    290                 295                 300

Val Leu Ser Leu Trp Val Ala Leu Leu Leu Ala Ala Phe Ile Leu Ser
305                 310                 315                 320

Ala Gly Val Phe Met Pro Asp Val Arg Tyr Ser Glu Leu Val Arg Gly
                325                 330                 335

Gln Val Val Val Phe Leu Val Leu Val Leu Asn Tyr Leu Val Val Met
            340                 345                 350

Met Ser Leu Pro Asn Trp Leu Val Phe Ser Leu Val Val Gly Tyr Arg
        355                 360                 365

Ile Leu Arg Val Leu Thr Phe Leu Val Ala Glu Lys Val Glu Val Arg
    370                 375                 380

Gly Pro Asp Gly Lys Val Val Glu Thr Arg Thr Ser Met Pro Ala Trp
385                 390                 395                 400

Ile Asn Lys Ala Ser Asn Phe Phe Gln Thr Arg Phe His Gln Lys Ile
                405                 410                 415

Arg Thr Gly Ile Ser Pro Thr Ala Arg Val Ile Pro Asn Gly Val Ile
            420                 425                 430

Val Ile Glu Thr Gln Asp Gly Ser Gly Thr Gly Phe Arg Cys Arg Asn
        435                 440                 445

Tyr Leu Val Thr Ala Gly His Val Val Thr Gly Thr Glu Thr Pro Lys
    450                 455                 460

Ile Gln Trp Ala Gly Val Thr Ala Tyr Ser Lys Val Val His Arg Val
465                 470                 475                 480

Ser Asp Lys Asp Ile Ala Phe Leu Ala Ile Pro Gln Glu Leu Gln Asp
                485                 490                 495

Leu Pro Ser Tyr Arg Leu Ala Lys Lys Val Glu Asp Gly Pro Val Val
            500                 505                 510

Ile Thr Ser Leu Glu Asp Ser Gly Ala Leu Ser Val Ala Val Thr Glu
        515                 520                 525

Gly Val Ile Val Thr Asp Ser Ile Thr Tyr Ala Val Gln Thr Arg Asn
    530                 535                 540

Gly Met Ser Gly Ser Pro Val Thr Asn Leu Asp Gly Arg Ile Leu Ala
545                 550                 555                 560

Val His Gln Thr Asn Thr Gly Phe Thr Gly Gly Ala Val Ile Leu Ser
                565                 570                 575

Asp Glu Asp Phe Pro Gln Pro Lys Lys Ser Ala Arg Glu Gln Gln Leu
        580                 585                 590

Glu Ala Arg Ile Lys Glu Leu Glu Ala Ala Met Asn Gln Ser Ser Gly
    595                 600                 605

His Glu Asp Ile Val Glu Leu Val Arg Ala Ala Val Ala Arg Glu Phe
    610                 615                 620

Lys Val Leu Arg Thr Glu Leu Ser Ala Thr Thr Phe Thr Gln Ala Lys
625                 630                 635                 640

Gly Lys Asn Lys Lys Asn His Arg Arg Arg Gly Gly Lys Lys Arg Arg
                645                 650                 655

Ala Val Trp Ser Glu Glu Glu Tyr Lys Glu Leu Leu Glu Lys Gly Phe
            660                 665                 670

Ser Lys Ser Gln Leu Arg Glu Met Ala Glu Val Leu Arg Ser His Glu
        675                 680                 685

Glu Asp Pro Phe Gly Ser Asp Thr Glu Ser Glu Gly Gly Phe Pro Glu
    690                 695                 700

Trp Ser Asp Ile Ser Asp Thr Glu Ser Ile Glu Arg Glu Trp Phe Gly
705                 710                 715                 720

Gln Ser Trp Glu Asp Cys Lys Pro Val Lys Glu Glu Pro Gln Asp Thr
                725                 730                 735

Leu Pro Val His Leu Lys Asp Lys Tyr Thr Leu Asp Ala Tyr Val Ile
            740                 745                 750

Ser Lys Glu Glu Leu Lys Ser Phe Ala Lys Glu Phe Lys Asp Tyr Val
        755                 760                 765

Asp Lys Val Glu Ala Leu Ile Asp Lys Thr Val Gln Gln Gly Lys Trp
    770                 775                 780

Leu Pro Ser Val Asn Pro Thr Ala Ile Ile Glu Glu Leu Asn Asp Leu
785                 790                 795                 800

Trp Phe Gly Leu Asn Ile Leu Met Trp Glu Lys Gly Leu Val Pro Phe
                805                 810                 815

Thr Gln Arg Lys Lys Ile Lys Arg Lys Val Gln Lys Asn Ser Lys Gly
            820                 825                 830

Ala Pro Lys Arg Gly Pro Leu Pro Thr Pro
        835                 840

<210>  11
<211>  842
<212>  PRT
<213>  Artificial

<220>
<223>  ORF1a polypeptide

<400>  11

Met Ala Leu Pro Phe Asp Asp Thr Leu Lys Phe Gly Ser Ala Thr Ala
1               5                   10                  15

Arg Met Lys Gly Arg Arg Leu Asp Met Val Ala Arg Thr Lys Leu Lys
            20                  25                  30

Asp Leu Leu Gly Asp Gly Pro Tyr Tyr Tyr Asp Phe Gly Pro Leu Glu
        35                  40                  45

Val Val Asp Ser Ser Ser Lys Gln Ser Thr Val Lys Met Thr Ser Val
    50                  55                  60

Gln Thr Val Tyr Val Ser Ser Val Val Glu Asp Asn Ser Tyr Val Thr
65                  70                  75                  80

Tyr Lys Phe Val Pro Gly Val Asn Asp Trp Val Glu Thr Glu Pro Val
                85                  90                  95

Leu His Lys Pro Thr Ala Leu Val Gly Val Leu Trp Arg Glu Tyr Lys
            100                 105                 110

Arg Tyr Lys Asp Arg Thr Glu Thr Leu Thr Gln Glu Leu Ser Gln Leu
        115                 120                 125

Lys Leu Glu His Ser Leu Leu Arg His Asp Tyr Glu Arg Val Arg Pro
    130                 135                 140

Gln Gln Pro Ile Ala Pro Lys Gly Arg Leu Gly Phe Ile Thr Lys Leu
145                 150                 155                 160

Leu Ile Gly Leu Leu Ile Gly Ser Leu Leu Ala His Ser Thr Leu Ala
                165                 170                 175

His Lys Leu Pro Gly Thr Gly Leu Met Gly Glu Cys Leu Asp Thr Asp
            180                 185                 190

Val Ile Asp Gly Lys Gln Val Cys Val Asn Phe Ile Pro Arg Asn Asp
        195                 200                 205

Thr Glu Ala Asp Ser Gln Asp Lys Pro Ala Thr Thr Val Trp Asp Pro
    210                 215                 220

Glu Gly Leu Leu Glu Leu Leu Ile Leu Val Ala Pro Tyr Leu Val Ser
225                 230                 235                 240

Trp Pro Met Ile Ala Thr Met Val Gly Phe Phe Tyr Val Ala Thr Ala
                245                 250                 255

Glu Gln Pro Ala Tyr Met Leu Val Ser Leu Leu Leu Ala Thr Tyr Ser
            260                 265                 270

Lys Ala Gln Val Leu Ala Leu Ala Gly Leu Pro Phe Met Asp Met Pro
        275                 280                 285

Ser Val Val Thr Leu Trp Val Ser Met Leu Ile His Ser Tyr Asp Ala
    290                 295                 300

Val Leu Ser Leu Trp Val Ala Leu Leu Leu Ala Ala Phe Ile Leu Ser
305                 310                 315                 320

Ala Gly Val Phe Met Pro Asp Val Arg Tyr Ser Glu Leu Val Arg Gly
                325                 330                 335

Gln Val Val Val Phe Leu Val Leu Val Leu Asn Tyr Leu Val Val Met
            340                 345                 350

Met Ser Leu Pro Asn Trp Leu Val Phe Ser Leu Val Val Gly Tyr Arg
        355                 360                 365

Ile Leu Arg Val Leu Thr Phe Leu Val Ala Glu Lys Val Glu Val Arg
    370                 375                 380

Gly Pro Asp Gly Lys Val Val Glu Thr Arg Thr Ser Met Pro Ala Trp
385                 390                 395                 400

Ile Asn Lys Ala Ser Asn Phe Phe Gln Thr Arg Phe His Gln Lys Ile
                405                 410                 415

Arg Thr Gly Ile Ser Pro Thr Ala Arg Val Ile Pro Asn Gly Val Ile
            420                 425                 430

Val Ile Glu Thr Gln Asp Gly Ser Gly Thr Gly Phe Arg Cys Arg Asn
        435                 440                 445

Tyr Leu Val Thr Ala Gly His Val Val Thr Gly Thr Glu Thr Pro Lys
    450                 455                 460

Ile Gln Trp Ala Gly Val Thr Ala Tyr Ser Lys Val Val His Arg Val
465                 470                 475                 480

Ser Asp Lys Asp Ile Ala Phe Leu Ala Ile Pro Gln Glu Leu Gln Asp
                485                 490                 495

Leu Pro Ser Tyr Arg Leu Ala Lys Lys Val Glu Asp Gly Pro Val Val
            500                 505                 510

Ile Thr Ser Leu Glu Asp Ser Gly Ala Leu Ser Val Ala Val Thr Glu
        515                 520                 525

Gly Val Ile Val Thr Asp Ser Ile Thr Tyr Ala Val Gln Thr Arg Asn
    530                 535                 540

Gly Met Ser Gly Ser Pro Val Thr Asn Leu Asp Gly Arg Ile Leu Ala
545                 550                 555                 560

Val His Gln Thr Asn Thr Gly Phe Thr Gly Gly Ala Val Ile Leu Ser
                565                 570                 575

Asp Glu Asp Phe Pro Gln Pro Lys Lys Ser Ala Arg Glu Gln Gln Leu
            580                 585                 590

Glu Ala Arg Ile Lys Glu Leu Glu Ala Ala Met Asn Gln Ser Ser Gly
        595                 600                 605

His Glu Asp Ile Val Glu Leu Val Arg Ala Ala Val Ala Arg Glu Phe
    610                 615                 620

Lys Val Leu Arg Thr Glu Leu Ser Ala Thr Thr Phe Thr Gln Ala Lys
625                 630                 635                 640

Gly Lys Asn Lys Lys Asn His Arg Arg Arg Gly Gly Lys Lys Arg Arg
                645                 650                 655

Ala Val Trp Ser Glu Glu Glu Tyr Lys Glu Leu Leu Glu Lys Gly Phe
            660                 665                 670

Ser Lys Ser Gln Leu Arg Glu Met Ala Glu Val Leu Arg Ser His Glu
        675                 680                 685

Glu Asp Pro Phe Gly Ser Asp Thr Asp Ser Glu Gly Gly Phe Pro Glu

690                 695                 700

Trp Ser Asp Ile Ser Asp Thr Glu Ser Ile Glu Arg Glu Trp Phe Gly
705                 710                 715                 720

Gln Ser Trp Glu Asp Cys Lys Pro Val Lys Glu Glu Pro Gln Asp Thr
                725                 730                 735

Leu Pro Val His Leu Lys Asp Lys Tyr Thr Leu Asp Ala Tyr Val Ile
            740                 745                 750

Ser Lys Glu Glu Leu Lys Ser Phe Ala Lys Glu Phe Lys Asp Tyr Val
        755                 760                 765

Asp Lys Val Glu Ala Leu Ile Asp Lys Thr Val Gln Gln Gly Lys Trp
    770                 775                 780

Leu Pro Ser Val Asn Pro Thr Ala Ile Ile Glu Glu Leu Asn Asp Leu
785                 790                 795                 800

Trp Phe Gly Leu Asn Met Leu Met Trp Glu Lys Gly Leu Val Pro Phe
                805                 810                 815

Thr Gln Arg Lys Lys Ile Lys Arg Lys Val Gln Lys Asn Ser Lys Gly
            820                 825                 830

Ala Pro Lys Arg Gly Pro Leu Pro Thr Pro
        835                 840

<210>  12
<211>  1351
<212>  PRT
<213>  Artificial

<220>
<223>  ORF1ab polypeptide

<400>  12

Met Ala Leu Pro Phe Asp Asp Thr Leu Lys Phe Gly Ser Ala Thr Ala
1               5                   10                  15

Arg Met Lys Gly Arg Arg Leu Asp Met Val Ala Arg Thr Lys Leu Lys
            20                  25                  30

Asp Leu Leu Gly Asp Gly Pro Tyr Tyr Tyr Asp Phe Gly Pro Leu Glu
        35                  40                  45

Val Val Asp Ser Ser Ser Lys Gln Ser Thr Val Lys Met Thr Ser Val
    50                  55                  60

Gln Thr Val Tyr Val Ser Ser Val Val Glu Asp Asn Ser Tyr Val Thr
65                  70                  75                  80

Tyr Lys Phe Val Pro Gly Val Asn Asp Trp Val Glu Thr Glu Pro Val
                85                  90                  95

Leu His Lys Pro Thr Ala Leu Val Gly Val Leu Trp Arg Glu Tyr Lys
            100                 105                 110

Arg Tyr Lys Asp Arg Thr Glu Thr Leu Thr Gln Glu Leu Ser Gln Leu
        115                 120                 125

Arg Leu Glu His Ser Leu Leu Arg His Asp Tyr Glu Arg Val Arg Pro
    130                 135                 140

Gln Gln Pro Val Ala Pro Lys Gly Arg Leu Gly Phe Ile Thr Lys Leu
145                 150                 155                 160

Leu Ile Gly Leu Leu Ile Gly Ser Leu Leu Ala His Ser Thr Leu Ala
                165                 170                 175

His Lys Leu Pro Gly Thr Gly Leu Met Gly Glu Cys Leu Asp Thr Asp
            180                 185                 190

Val Ile Asp Gly Lys Gln Val Cys Val Asn Phe Ile Pro Arg Asn Asp
        195                 200                 205

Thr Glu Ala Asp Ser Pro Asp Lys Pro Ala Thr Thr Val Trp Asp Pro
    210                 215                 220

Glu Gly Leu Leu Glu Leu Leu Ile Leu Val Ala Pro Tyr Leu Val Ser
225                 230                 235                 240

Trp Pro Met Ile Ala Thr Met Val Gly Phe Phe Tyr Val Ala Thr Ala
                245                 250                 255

Glu Gln Pro Ala Tyr Met Leu Val Ser Leu Leu Leu Ala Thr Tyr Ser
            260                 265                 270

Lys Ala Gln Val Leu Ala Leu Ala Gly Leu Pro Phe Met Asp Met Pro
        275                 280                 285

Ser Val Val Thr Leu Trp Val Ser Met Leu Ile His Ser Tyr Asp Ala
    290                 295                 300

Val Leu Ser Leu Trp Val Ala Leu Leu Leu Ala Ala Phe Ile Leu Ser

305                 310                 315                 320

Ala Gly Val Phe Met Pro Asp Val Arg Tyr Ser Glu Leu Val Arg Gly
                325                 330                 335

Gln Val Val Val Phe Leu Val Leu Val Leu Asn Tyr Leu Val Val Met
            340                 345                 350

Met Ser Leu Pro Asn Trp Leu Val Phe Ser Leu Val Val Gly Tyr Arg
        355                 360                 365

Ile Leu Arg Val Leu Thr Phe Leu Val Ala Glu Lys Val Glu Val Arg
    370                 375                 380

Gly Pro Asp Gly Lys Val Val Glu Thr Arg Thr Ser Met Pro Ala Trp
385                 390                 395                 400

Ile Asn Lys Ala Ser Asn Phe Phe Gln Thr Arg Phe His Gln Lys Ile
                405                 410                 415

Arg Thr Gly Ile Ser Pro Thr Ala Arg Val Ile Pro Asn Gly Val Ile
            420                 425                 430

Val Ile Glu Thr Gln Asp Gly Ser Gly Thr Gly Phe Arg Cys Arg Asn
        435                 440                 445

Tyr Leu Val Thr Ala Gly His Val Val Thr Gly Thr Glu Thr Pro Lys
    450                 455                 460

Ile Gln Trp Ala Gly Val Thr Ala Tyr Ser Lys Val Val His Arg Val
465                 470                 475                 480

Ser Asp Lys Asp Ile Ala Phe Leu Ala Ile Pro Gln Glu Leu Gln Asp
                485                 490                 495

Leu Pro Ser Tyr Arg Leu Ala Lys Lys Val Glu Asp Gly Pro Val Val
            500                 505                 510

Ile Thr Ser Leu Glu Asp Ser Gly Ala Leu Ser Val Ala Val Thr Glu
        515                 520                 525

Gly Val Ile Val Thr Asp Ser Ile Thr Tyr Ala Val Gln Thr Arg Asn
    530                 535                 540

Gly Met Ser Gly Ser Pro Val Thr Asn Leu Asp Gly Arg Ile Leu Ala
545                 550                 555                 560

Val His Gln Thr Asn Thr Gly Phe Thr Gly Gly Ala Val Ile Leu Ser
                565                 570                 575

Asp Glu Asp Phe Pro Gln Pro Lys Lys Ser Ala Arg Glu Gln Gln Leu
            580                 585                 590

Glu Ala Arg Ile Lys Glu Leu Glu Ala Ala Met Asn Gln Ser Ser Gly
        595                 600                 605

His Glu Asp Ile Val Glu Leu Val Arg Ala Ala Val Ala Arg Glu Phe
    610                 615                 620

Lys Val Leu Arg Thr Glu Leu Ser Ala Thr Thr Phe Thr Gln Ala Lys
625                 630                 635                 640

Gly Lys Asn Lys Lys Asn His Arg Arg Arg Gly Gly Lys Lys Arg Arg
                645                 650                 655

Ala Val Trp Ser Glu Glu Glu Tyr Lys Glu Leu Leu Glu Lys Gly Phe
            660                 665                 670

Ser Lys Ser Gln Leu Arg Glu Met Ala Glu Val Leu Arg Ser His Glu
        675                 680                 685

Glu Asp Pro Phe Gly Ser Asp Thr Glu Ser Glu Gly Gly Phe Pro Glu
    690                 695                 700

Trp Ser Asp Ile Ser Asp Thr Glu Ser Ile Glu Arg Glu Trp Phe Gly
705                 710                 715                 720

Gln Ser Trp Glu Asp Cys Lys Pro Val Lys Glu Glu Pro Gln Asp Thr
                725                 730                 735

Leu Pro Val His Leu Lys Asp Lys Tyr Thr Leu Asp Ala Tyr Val Ile
            740                 745                 750

Ser Lys Glu Glu Leu Lys Ser Phe Ala Lys Glu Phe Lys Asp Tyr Val
        755                 760                 765

Asp Lys Val Glu Ala Leu Ile Asp Lys Thr Val Gln Gln Gly Lys Trp
    770                 775                 780

Leu Pro Ser Val Asn Pro Thr Ala Ile Ile Glu Glu Leu Asn Asp Leu
785                 790                 795                 800

Trp Phe Gly Leu Asn Met Leu Met Trp Glu Lys Gly Leu Val Pro Phe
                805                 810                 815

Thr Gln Arg Lys Lys Ile Lys Arg Lys Val Gln Lys Lys Leu Gln Gly
            820                 825                 830

Gly Pro Gln Glu Gly Pro Ile Thr Tyr Ser Met Met Asp Leu Lys Ala
        835                 840                 845

Trp Arg Arg Ser Leu Cys Glu Val Glu Arg Arg Leu Val Pro Glu Gly
    850                 855                 860

Tyr Pro Leu Ile Gly Gly Val Pro Leu Asp Arg Pro Ile Ser Asp Trp
865                 870                 875                 880

Asp Glu Pro Ile Asp Asp Leu Leu Leu Met Leu Pro Ala Phe Lys Glu
                885                 890                 895

Asn Leu Asn Tyr Thr Pro Ala Val Trp Gly Pro Glu Ala Tyr Ala Lys
            900                 905                 910

Ser Phe Asp Lys Phe Thr Tyr Arg Glu Pro Cys Pro Asn Ile Ala Ala
        915                 920                 925

Thr Tyr Pro Arg Glu Trp Ser Phe Ala Thr Lys Ala Leu Lys Arg Glu
    930                 935                 940

Tyr Ser Phe Leu Asp Gly Ser Val Leu Thr Asp Ile Thr Ala Thr Ser
945                 950                 955                 960

Lys Asn Ala Asp Ser Thr Pro Ala Tyr Pro Lys Ser Leu Tyr Trp Lys
                965                 970                 975

Thr Glu Ala Asp Tyr Leu Gly Glu Arg Gly Tyr Gln Asp Tyr Ile Lys
            980                 985                 990

Gln Leu Asp Ala Ile Tyr Ser Gly Glu Arg Pro Asp Val Leu Trp Tyr
        995                 1000                1005

Leu Phe Leu Lys Lys Glu Ile Leu Lys Val Glu Lys Val Glu Gln
    1010                1015                1020

Ala Asp Ile Arg Gln Ile Val Cys Ser Asp Pro Ile Phe Ala Arg
    1025                1030                1035

Ile Gly Cys His Phe Glu Glu Asp Gln Asn Ile Arg Met Lys Asn
    1040                1045                1050

His Thr Arg Thr Arg Ser Gly Gln Cys Gly Trp Ser Pro Phe Phe
    1055                1060                1065

Gly Gly Phe Asn Asp Arg Ile Lys Arg Leu Cys Ala Cys Asp Pro
    1070                1075                1080

Asp Cys Tyr Ile Glu Phe Asp Trp Thr Arg Phe Asp Gly Thr Ile
    1085                1090                1095

Pro Asn Glu Val Phe Met His Ile Lys Lys Val Arg Tyr Ser Phe
    1100                1105                1110

Phe Ala Lys Glu Phe Arg Thr Lys Arg Val Lys Glu Ala Tyr Gln
    1115                1120                1125

Trp Tyr Cys Glu Gln Leu Leu Tyr Arg His Val Val Leu Pro Ser
    1130                1135                1140

Gly Glu Val Thr Leu Gln Thr Arg Gly Asn Pro Ser Gly Gln Ile
    1145                1150                1155

Ser Thr Thr Met Asp Asn Asn Met Val Asn Val Phe Leu Gln Ala
    1160                1165                1170

Phe Glu Tyr Ala Tyr Leu His Pro Asp Cys Thr Asp Glu Glu Leu
    1175                1180                1185

Glu Ala Asn Trp Ser Arg Met Asp Thr Leu Val Tyr Gly Asp Asp
    1190                1195                1200

Arg Leu Ser Ala Tyr Ile Gly Leu Pro Ala Asn Tyr Val Glu Lys
    1205                1210                1215

Val Val Ile Met Tyr Ser Asn Val Phe Gly Met Trp Val Lys Pro
    1220                1225                1230

Glu Lys Val Ile Val Ser Pro Ser Ile Ile Gly Leu Thr Phe Cys
    1235                1240                1245

Gly Leu Arg Ile Thr Ser Asp Gly Thr Ile Phe Leu Pro Val Pro
    1250                1255                1260

Ala Glu Thr Asn Lys Leu Ile Ser Ala Leu Ile Lys Pro Thr Lys
    1265                1270                1275

Lys Leu Gln Asp Val Met Thr Leu Tyr Gly Lys Leu Leu Cys Tyr
    1280                1285                1290

Arg Ile Leu Gly His Asn Leu Pro Asp Asp Asn Lys Phe Lys Ala

1295                1300                1305

Tyr Ile  Leu Val Ala Leu Glu  Val Val Ala Arg His  Ile Arg Thr
    1310                1315                1320

Arg Cys  Gly Asp Glu Pro Leu  Arg Phe Thr Asp Glu  Met Leu Asp
    1325                1330                1335

Arg Leu  Trp Arg Gly Gly Pro  Lys Val Gly Tyr Gly  Trp
    1340                1345                1350

<210>  13
<211>  1351
<212>  PRT
<213>  Artificial

<220>
<223>  ORF1ab polypeptide

<400>  13

Met Ala Leu Pro Phe Asp Asp Thr Leu Lys Phe Gly Ser Ala Thr Ala
1               5                   10                  15

Arg Met Lys Gly Arg Arg Leu Asp Met Val Ala Arg Thr Lys Leu Lys
            20                  25                  30

Asp Leu Leu Gly Asp Gly Pro Tyr Tyr Tyr Asp Phe Gly Pro Leu Glu
        35                  40                  45

Val Val Asp Ser Ser Ser Lys Gln Ser Thr Val Lys Met Thr Ser Val
    50                  55                  60

Gln Thr Val Tyr Val Ser Ser Val Val Glu Asp Asn Ser Tyr Val Thr
65                  70                  75                  80

Tyr Lys Phe Val Pro Gly Val Asn Asp Trp Val Glu Thr Glu Pro Val
                85                  90                  95

Leu His Lys Pro Thr Ala Leu Val Gly Val Leu Trp Arg Glu Tyr Lys
            100                 105                 110

Arg Tyr Lys Asp Arg Thr Glu Thr Leu Thr Gln Glu Leu Ser Gln Leu
        115                 120                 125

Lys Leu Glu His Ser Leu Leu Arg His Asp Tyr Glu Arg Val Arg Pro
    130                 135                 140

Gln Gln Pro Val Ala Pro Lys Gly Arg Leu Gly Phe Ile Thr Lys Leu
145                 150                 155                 160

Leu Ile Gly Leu Leu Ile Gly Ser Leu Leu Ala His Ser Thr Leu Ala
                165                 170                 175

His Lys Leu Pro Gly Thr Gly Leu Met Gly Glu Cys Leu Asp Thr Asp
            180                 185                 190

Val Ile Asp Gly Lys Gln Val Cys Val Asn Phe Ile Pro Arg Asn Asp
        195                 200                 205

Thr Glu Ala Asp Ser Pro Asp Lys Pro Ala Thr Thr Ala Trp Asp Pro
    210                 215                 220

Glu Gly Leu Leu Glu Leu Leu Ile Leu Val Ala Pro Tyr Leu Val Ser
225                 230                 235                 240

Trp Pro Met Ile Ala Thr Met Val Gly Phe Phe Tyr Val Ala Thr Ala
                245                 250                 255

Glu Gln Pro Ala Tyr Met Leu Val Ser Leu Leu Leu Ala Thr Tyr Ser
            260                 265                 270

Lys Ala Gln Val Leu Ala Leu Ala Gly Leu Pro Phe Met Asp Met Pro
        275                 280                 285

Ser Val Val Thr Leu Trp Val Ser Met Leu Ile His Ser Tyr Asp Ala
    290                 295                 300

Val Leu Ser Leu Trp Val Ala Leu Leu Leu Ala Ala Phe Ile Leu Ser
305                 310                 315                 320

Ala Gly Val Phe Met Pro Asp Val Arg Tyr Ser Glu Leu Val Arg Gly
                325                 330                 335

Gln Val Val Val Phe Leu Val Leu Val Leu Asn Tyr Leu Val Val Met
            340                 345                 350

Met Ser Leu Pro Asn Trp Leu Val Phe Ser Leu Val Val Gly Tyr Arg
        355                 360                 365

Ile Leu Arg Val Leu Thr Phe Leu Val Ala Glu Lys Val Glu Val Arg
    370                 375                 380

Gly Pro Asp Gly Lys Val Val Glu Thr Arg Thr Ser Met Pro Ala Trp
385                 390                 395                 400

Ile Asn Lys Ala Ser Asn Phe Phe Gln Thr Arg Phe His Gln Lys Ile

                405                 410                 415

Arg Thr Gly Ile Ser Pro Thr Ala Arg Val Ile Pro Asn Gly Val Ile
            420                 425                 430

Val Ile Glu Thr Gln Asp Gly Ser Gly Thr Gly Phe Arg Cys Arg Asn
        435                 440                 445

Tyr Leu Val Thr Ala Gly His Val Val Thr Gly Thr Glu Thr Pro Lys
    450                 455                 460

Ile Gln Trp Ala Gly Val Thr Ala Tyr Ser Lys Val Val His Arg Val
465                 470                 475                 480

Ser Asp Lys Asp Ile Ala Phe Leu Ala Ile Pro Gln Glu Leu Gln Asp
                485                 490                 495

Leu Pro Ser Tyr Arg Leu Ala Lys Lys Val Glu Asp Gly Pro Val Val
            500                 505                 510

Ile Thr Ser Leu Glu Asp Ser Gly Ala Leu Ser Val Ala Val Thr Glu
        515                 520                 525

Gly Val Ile Val Thr Asp Ser Ile Thr Tyr Ala Val Gln Thr Arg Asn
    530                 535                 540

Gly Met Ser Gly Ser Pro Val Thr Asn Leu Asp Gly Arg Ile Leu Ala
545                 550                 555                 560

Val His Gln Thr Asn Thr Gly Phe Thr Gly Gly Ala Val Ile Leu Ser
                565                 570                 575

Asp Glu Asp Phe Pro Gln Pro Lys Lys Ser Ala Arg Glu Gln Gln Leu
            580                 585                 590

Glu Ala Arg Ile Lys Glu Leu Glu Ala Ala Met Asn Gln Ser Ser Gly
        595                 600                 605

His Glu Asp Ile Val Glu Leu Val Arg Ala Ala Val Ala Arg Glu Phe
    610                 615                 620

Lys Val Leu Arg Thr Glu Leu Ser Ala Thr Thr Phe Thr Gln Ala Lys
625                 630                 635                 640

Gly Lys Asn Lys Lys Asn His Arg Arg Arg Gly Gly Lys Lys Arg Arg
                645                 650                 655

Ala Val Trp Ser Glu Glu Glu Tyr Lys Glu Leu Leu Glu Lys Gly Phe
            660                 665                 670

Ser Lys Ser Gln Leu Arg Glu Met Ala Glu Val Leu Arg Ser His Glu
        675                 680                 685

Glu Asp Pro Phe Gly Ser Asp Thr Glu Ser Glu Gly Gly Phe Pro Glu
    690                 695                 700

Trp Ser Asp Ile Ser Asp Thr Glu Ser Ile Glu Arg Glu Trp Phe Gly
705                 710                 715                 720

Gln Ser Trp Glu Asp Cys Lys Pro Val Lys Glu Glu Pro Gln Asp Thr
                725                 730                 735

Leu Pro Val His Leu Lys Asp Lys Tyr Thr Leu Asp Ala Tyr Val Ile
            740                 745                 750

Ser Lys Glu Glu Leu Lys Ser Phe Ala Lys Glu Phe Lys Asp Tyr Val
        755                 760                 765

Asp Lys Val Glu Ala Leu Ile Asp Lys Thr Val Gln Gln Gly Lys Trp
    770                 775                 780

Leu Pro Ser Val Asn Pro Thr Ala Ile Ile Glu Glu Leu Asn Asp Leu
785                 790                 795                 800

Trp Phe Gly Leu Asn Ile Leu Met Trp Glu Lys Gly Leu Val Pro Phe
                805                 810                 815

Thr Gln Arg Lys Lys Ile Lys Arg Lys Val Gln Lys Lys Leu Gln Gly
            820                 825                 830

Gly Pro Gln Glu Gly Pro Ile Thr Tyr Ser Met Met Asp Leu Lys Ala
        835                 840                 845

Trp Arg Arg Ser Leu Cys Glu Val Glu Arg Arg Leu Val Pro Glu Gly
    850                 855                 860

Tyr Pro Leu Ile Gly Gly Val Pro Leu Asp Arg Pro Ile Ser Asp Trp
865                 870                 875                 880

Asp Glu Pro Ile Asp Asp Leu Leu Leu Met Leu Pro Ala Phe Lys Glu
                885                 890                 895

Asn Leu Asn Tyr Thr Pro Ala Val Trp Gly Pro Glu Ala Tyr Ala Lys
            900                 905                 910

Ser Phe Asp Lys Phe Thr Tyr Arg Glu Pro Cys Pro Asn Ile Ala Ala
        915                 920                 925

Thr Tyr Pro Arg Glu Trp Ser Phe Ala Thr Lys Ala Leu Lys Arg Glu
    930                 935                 940

Tyr Ser Phe Leu Asp Gly Ser Val Leu Thr Asp Ile Thr Ala Thr Ser
945                 950                 955                 960

Lys Asn Ala Asp Ser Thr Pro Ala Tyr Pro Lys Ser Leu Tyr Trp Lys
                965                 970                 975

Thr Glu Ala Asp Tyr Leu Gly Glu Arg Gly Tyr Gln Asp Tyr Ile Lys
            980                 985                 990

Gln Leu Asp Ala Ile Tyr Ser Gly  Glu Arg Pro Asp Val  Leu Trp Tyr
        995                 1000                1005

Leu Phe  Leu Lys Lys Glu Ile  Leu Lys Val Glu Lys  Val Glu Gln
    1010                 1015                 1020

Ala Asp  Ile Arg Gln Ile Val  Cys Ser Asp Pro Ile  Phe Ala Arg
    1025                 1030                 1035

Ile Gly  Cys His Phe Glu Glu  Asp Gln Asn Ile Arg  Met Lys Asn
    1040                 1045                 1050

His Thr  Arg Thr Arg Ser Gly  Gln Cys Gly Trp Ser  Pro Phe Phe
    1055                 1060                 1065

Gly Gly  Phe Asn Asp Arg Ile  Lys Arg Leu Cys Ala  Cys Asp Pro
    1070                 1075                 1080

Asp Cys  Tyr Ile Glu Phe Asp  Trp Thr Arg Phe Asp  Gly Thr Ile
    1085                 1090                 1095

Pro Asn  Glu Val Phe Met His  Ile Lys Lys Val Arg  Tyr Ser Phe
    1100                 1105                 1110

Phe Ala  Lys Glu Phe Arg Thr  Lys Arg Val Lys Glu  Ala Tyr Gln
    1115                 1120                 1125

Trp Tyr  Cys Glu Gln Leu Leu  Tyr Arg His Val Val  Leu Pro Ser
    1130                 1135                 1140

Gly Glu  Val Thr Leu Gln Thr  Arg Gly Asn Pro Ser  Gly Gln Ile
    1145                 1150                 1155

Ser Thr Thr Met Asp Asn Asn Met Val Asn Val Phe Leu Gln Ala
    1160                1165                1170

Phe Glu Tyr Ala Tyr Leu His Pro Asp Cys Thr Asp Glu Glu Leu
    1175                1180                1185

Glu Ala Asn Trp Ser Arg Met Asp Thr Leu Val Tyr Gly Asp Asp
    1190                1195                1200

Arg Leu Ser Ala Tyr Ile Gly Leu Pro Ala Asn Tyr Val Glu Lys
    1205                1210                1215

Val Val Ile Met Tyr Ser Asn Val Phe Gly Met Trp Val Lys Pro
    1220                1225                1230

Glu Lys Val Ile Val Ser Pro Ser Ile Ile Gly Leu Thr Phe Cys
    1235                1240                1245

Gly Leu Arg Ile Thr Ser Asp Gly Thr Ile Phe Leu Pro Val Pro
    1250                1255                1260

Ala Glu Thr Asn Lys Leu Ile Ser Ala Leu Ile Lys Pro Thr Lys
    1265                1270                1275

Lys Leu Gln Asp Val Met Thr Leu Tyr Gly Lys Leu Leu Cys Tyr
    1280                1285                1290

Arg Ile Leu Gly His Asn Leu Pro Asp Asp Asn Lys Phe Lys Ala
    1295                1300                1305

Tyr Ile Leu Val Ala Leu Glu Val Val Ala Arg His Ile Arg Thr
    1310                1315                1320

Arg Cys Gly Asp Glu Pro Leu Arg Phe Thr Asp Glu Met Leu Asp
    1325                1330                1335

Arg Leu Trp Arg Gly Gly Pro Lys Val Gly Tyr Gly Trp
    1340                1345                1350

<210>  14
<211>  1351
<212>  PRT
<213>  Artificial

<220>
<223>  ORF1ab polypeptide

<400>  14

Met Ala Leu Pro Phe Asp Asp Thr Leu Lys Phe Gly Ser Ala Thr Ala
1               5                   10                  15

Arg Met Lys Gly Arg Arg Leu Asp Met Val Ala Arg Thr Lys Leu Lys
            20                  25                  30

Asp Leu Leu Gly Asp Gly Pro Tyr Tyr Tyr Asp Phe Gly Pro Leu Glu
        35                  40                  45

Val Val Asp Ser Ser Ser Lys Gln Ser Thr Val Lys Met Thr Ser Val
    50                  55                  60

Gln Thr Val Tyr Val Ser Ser Val Val Glu Asp Asn Ser Tyr Val Thr
65                  70                  75                  80

Tyr Lys Phe Val Pro Gly Val Asn Asp Trp Val Glu Thr Glu Pro Val
                85                  90                  95

Leu His Lys Pro Thr Ala Leu Val Gly Val Leu Trp Arg Glu Tyr Lys
            100                 105                 110

Arg Tyr Lys Asp Arg Thr Glu Thr Leu Thr Gln Glu Leu Ser Gln Leu
        115                 120                 125

Lys Leu Glu His Ser Leu Leu Arg His Asp Tyr Glu Arg Val Arg Pro
    130                 135                 140

Gln Gln Pro Ile Ala Pro Lys Gly Arg Leu Gly Phe Ile Thr Lys Leu
145                 150                 155                 160

Leu Ile Gly Leu Leu Ile Gly Ser Leu Leu Ala His Ser Thr Leu Ala
                165                 170                 175

His Lys Leu Pro Gly Thr Gly Leu Met Gly Glu Cys Leu Asp Thr Asp
            180                 185                 190

Val Ile Asp Gly Lys Gln Val Cys Val Asn Phe Ile Pro Arg Asn Asp
        195                 200                 205

Thr Glu Ala Asp Ser Gln Asp Lys Pro Ala Thr Thr Val Trp Asp Pro
    210                 215                 220

Glu Gly Leu Leu Glu Leu Leu Ile Leu Val Ala Pro Tyr Leu Val Ser
225                 230                 235                 240

Trp Pro Met Ile Ala Thr Met Val Gly Phe Phe Tyr Val Ala Thr Ala
                245                 250                 255

Glu Gln Pro Ala Tyr Met Leu Val Ser Leu Leu Leu Ala Thr Tyr Ser
            260                 265                 270

Lys Ala Gln Val Leu Ala Leu Ala Gly Leu Pro Phe Met Asp Met Pro
        275                 280                 285

Ser Val Val Thr Leu Trp Val Ser Met Leu Ile His Ser Tyr Asp Ala
    290                 295                 300

Val Leu Ser Leu Trp Val Ala Leu Leu Leu Ala Ala Phe Ile Leu Ser
305                 310                 315                 320

Ala Gly Val Phe Met Pro Asp Val Arg Tyr Ser Glu Leu Val Arg Gly
                325                 330                 335

Gln Val Val Val Phe Leu Val Leu Val Leu Asn Tyr Leu Val Val Met
            340                 345                 350

Met Ser Leu Pro Asn Trp Leu Val Phe Ser Leu Val Val Gly Tyr Arg
        355                 360                 365

Ile Leu Arg Val Leu Thr Phe Leu Val Ala Glu Lys Val Glu Val Arg
    370                 375                 380

Gly Pro Asp Gly Lys Val Val Glu Thr Arg Thr Ser Met Pro Ala Trp
385                 390                 395                 400

Ile Asn Lys Ala Ser Asn Phe Phe Gln Thr Arg Phe His Gln Lys Ile
                405                 410                 415

Arg Thr Gly Ile Ser Pro Thr Ala Arg Val Ile Pro Asn Gly Val Ile
            420                 425                 430

Val Ile Glu Thr Gln Asp Gly Ser Gly Thr Gly Phe Arg Cys Arg Asn
        435                 440                 445

Tyr Leu Val Thr Ala Gly His Val Val Thr Gly Thr Glu Thr Pro Lys
    450                 455                 460

Ile Gln Trp Ala Gly Val Thr Ala Tyr Ser Lys Val Val His Arg Val
465                 470                 475                 480

Ser Asp Lys Asp Ile Ala Phe Leu Ala Ile Pro Gln Glu Leu Gln Asp
                485                 490                 495

Leu Pro Ser Tyr Arg Leu Ala Lys Lys Val Glu Asp Gly Pro Val Val

            500                 505                 510

Ile Thr Ser Leu Glu Asp Ser Gly Ala Leu Ser Val Ala Val Thr Glu
        515                 520                 525

Gly Val Ile Val Thr Asp Ser Ile Thr Tyr Ala Val Gln Thr Arg Asn
    530                 535                 540

Gly Met Ser Gly Ser Pro Val Thr Asn Leu Asp Gly Arg Ile Leu Ala
545                 550                 555                 560

Val His Gln Thr Asn Thr Gly Phe Thr Gly Gly Ala Val Ile Leu Ser
                565                 570                 575

Asp Glu Asp Phe Pro Gln Pro Lys Lys Ser Ala Arg Glu Gln Gln Leu
            580                 585                 590

Glu Ala Arg Ile Lys Glu Leu Glu Ala Ala Met Asn Gln Ser Ser Gly
        595                 600                 605

His Glu Asp Ile Val Glu Leu Val Arg Ala Ala Val Ala Arg Glu Phe
    610                 615                 620

Lys Val Leu Arg Thr Glu Leu Ser Ala Thr Thr Phe Thr Gln Ala Lys
625                 630                 635                 640

Gly Lys Asn Lys Lys Asn His Arg Arg Arg Gly Gly Lys Lys Arg Arg
                645                 650                 655

Ala Val Trp Ser Glu Glu Glu Tyr Lys Glu Leu Leu Glu Lys Gly Phe
            660                 665                 670

Ser Lys Ser Gln Leu Arg Glu Met Ala Glu Val Leu Arg Ser His Glu
        675                 680                 685

Glu Asp Pro Phe Gly Ser Asp Thr Asp Ser Glu Gly Gly Phe Pro Glu
    690                 695                 700

Trp Ser Asp Ile Ser Asp Thr Glu Ser Ile Glu Arg Glu Trp Phe Gly
705                 710                 715                 720

Gln Ser Trp Glu Asp Cys Lys Pro Val Lys Glu Glu Pro Gln Asp Thr
                725                 730                 735

Leu Pro Val His Leu Lys Asp Lys Tyr Thr Leu Asp Ala Tyr Val Ile
            740                 745                 750

Ser Lys Glu Glu Leu Lys Ser Phe Ala Lys Glu Phe Lys Asp Tyr Val
        755                 760                 765

Asp Lys Val Glu Ala Leu Ile Asp Lys Thr Val Gln Gln Gly Lys Trp
    770                 775                 780

Leu Pro Ser Val Asn Pro Thr Ala Ile Ile Glu Glu Leu Asn Asp Leu
785                 790                 795                 800

Trp Phe Gly Leu Asn Met Leu Met Trp Glu Lys Gly Leu Val Pro Phe
                805                 810                 815

Thr Gln Arg Lys Lys Ile Lys Arg Lys Val Gln Lys Lys Leu Gln Gly
            820                 825                 830

Gly Pro Gln Glu Gly Pro Ile Thr Tyr Ser Met Met Asp Leu Lys Ala
        835                 840                 845

Trp Arg Arg Ser Leu Cys Glu Val Glu Arg Arg Leu Val Pro Glu Gly
    850                 855                 860

Tyr Pro Leu Ile Gly Gly Val Pro Leu Asp Arg Pro Ile Ser Asp Trp
865                 870                 875                 880

Asp Glu Pro Ile Asp Asp Leu Leu Leu Met Leu Pro Ala Phe Lys Glu
                885                 890                 895

Asn Leu Asn Tyr Thr Pro Ala Val Trp Gly Pro Glu Ala Tyr Ala Lys
            900                 905                 910

Ser Phe Asp Lys Phe Thr Tyr Arg Glu Pro Cys Pro Asn Ile Ala Ala
        915                 920                 925

Thr Tyr Pro Arg Glu Trp Ser Phe Ala Thr Lys Ala Leu Lys Arg Glu
    930                 935                 940

Tyr Ser Phe Leu Asp Gly Ser Val Leu Thr Asp Ile Thr Ala Thr Ser
945                 950                 955                 960

Lys Asn Ala Asp Ser Thr Pro Ala Tyr Pro Lys Ser Leu Tyr Trp Lys
                965                 970                 975

Thr Glu Ala Asp Tyr Leu Gly Glu Arg Gly Tyr Gln Asp Tyr Ile Lys
            980                 985                 990

Gln Leu Asp Ala Ile Tyr Ser Gly  Glu Arg Pro Asp Val  Leu Trp Tyr
        995                 1000                1005

Leu Phe Leu Lys Lys Glu Ile Leu Lys Val Glu Lys Val Glu Gln
    1010                1015                1020

Ala Asp Ile Arg Gln Ile Val Cys Ser Asp Pro Ile Phe Ala Arg
    1025                1030                1035

Ile Gly Cys His Phe Glu Glu Asp Gln Asn Ile Arg Met Lys Asn
    1040                1045                1050

His Thr Arg Thr Arg Ser Gly Gln Cys Gly Trp Ser Pro Phe Phe
    1055                1060                1065

Gly Gly Phe Asn Asp Arg Ile Lys Arg Leu Cys Ala Cys Asp Pro
    1070                1075                1080

Asp Cys Tyr Ile Glu Phe Asp Trp Thr Arg Phe Asp Gly Thr Ile
    1085                1090                1095

Pro Asn Glu Val Phe Met His Ile Lys Lys Val Arg Tyr Ser Phe
    1100                1105                1110

Phe Ala Lys Glu Phe Arg Thr Lys Arg Val Lys Glu Ala Tyr Gln
    1115                1120                1125

Trp Tyr Cys Glu Gln Leu Leu Tyr Arg His Val Val Leu Pro Ser
    1130                1135                1140

Gly Glu Val Thr Leu Gln Thr Arg Gly Asn Pro Ser Gly Gln Ile
    1145                1150                1155

Ser Thr Thr Met Asp Asn Asn Met Val Asn Val Phe Leu Gln Ala
    1160                1165                1170

Phe Glu Tyr Ala Tyr Leu His Pro Asp Cys Thr Asp Glu Glu Leu
    1175                1180                1185

Glu Ala Asn Trp Ser Arg Met Asp Thr Leu Val Tyr Gly Asp Asp
    1190                1195                1200

Arg Leu Ser Ala Tyr Ile Gly Leu Pro Ala Asn Tyr Val Glu Lys
    1205                1210                1215

Val Val Ile Met Tyr Ser Asn Val Phe Gly Met Trp Val Lys Pro
    1220                1225                1230

Glu Lys Val Ile Val Ser Pro Ser Ile Ile Gly Leu Thr Phe Cys
    1235                1240                1245

Gly Leu Arg Ile Thr Ser Asp Gly Thr Ile Phe Leu Pro Val Pro
    1250                1255                1260

Ala Glu Thr Asn Lys Leu Ile Ser Ala Leu Ile Lys Pro Thr Lys
    1265                1270                1275

Lys Leu Gln Asp Val Met Thr Leu Tyr Gly Lys Leu Leu Cys Tyr
    1280                1285                1290

Arg Ile Leu Gly His Asn Leu Pro Asp Asp Asn Lys Phe Lys Ala
    1295                1300                1305

Tyr Ile Leu Val Ala Leu Glu Val Val Ala Arg His Ile Arg Thr
    1310                1315                1320

Arg Cys Gly Asp Glu Pro Leu Arg Phe Thr Asp Glu Met Leu Asp
    1325                1330                1335

Arg Leu Trp Arg Gly Gly Pro Lys Val Gly Tyr Gly Trp
    1340                1345                1350

<210> 15
<211> 508
<212> PRT
<213> Artificial

<220>
<223> ORF1b polypeptide

<400> 15
   Met Asp Leu Lys Ala Trp Arg Arg Ser Leu Cys Glu Val Glu Arg Arg
   1               5                   10                  15
   Leu Val Pro Glu Gly Tyr Pro Leu Ile Gly Gly Val Pro Leu Asp Arg
               20                  25                  30
   Pro Ile Ser Asp Trp Asp Glu Pro Ile Asp Asp Leu Leu Leu Met Leu
           35                  40                  45
   Pro Ala Phe Lys Glu Asn Leu Asn Tyr Thr Pro Ala Val Trp Gly Pro
       50                  55                  60
   Glu Ala Tyr Ala Lys Ser Phe Asp Lys Phe Thr Tyr Arg Glu Pro Cys
   65                  70                  75                  80
   Pro Asn Ile Ala Ala Thr Tyr Pro Arg Glu Trp Ser Phe Ala Thr Lys
                   85                  90                  95
   Ala Leu Lys Arg Glu Tyr Ser Phe Leu Asp Gly Ser Val Leu Thr Asp
               100                 105                 110
   Ile Thr Ala Thr Ser Lys Asn Ala Asp Ser Thr Pro Ala Tyr Pro Lys
           115                 120                 125
   Ser Leu Tyr Trp Lys Thr Glu Ala Asp Tyr Leu Gly Glu Arg Gly Tyr
       130                 135                 140
   Gln Asp Tyr Ile Lys Gln Leu Asp Ala Ile Tyr Ser Gly Glu Arg Pro
   145                 150                 155                 160
   Asp Val Leu Trp Tyr Leu Phe Leu Lys Lys Glu Ile Leu Lys Val Glu
                   165                 170                 175
   Lys Val Glu Gln Ala Asp Ile Arg Gln Ile Val Cys Ser Asp Pro Ile
               180                 185                 190
   Phe Ala Arg Ile Gly Cys His Phe Glu Glu Asp Gln Asn Ile Arg Met
           195                 200                 205
   Lys Asn His Thr Arg Thr Arg Ser Gly Gln Cys Gly Trp Ser Pro Phe
       210                 215                 220
   Phe Gly Gly Phe Asn Asp Arg Ile Lys Arg Leu Cys Ala Cys Asp Pro
   225                 230                 235                 240
   Asp Cys Tyr Ile Glu Phe Asp Trp Thr Arg Phe Asp Gly Thr Ile Pro
                   245                 250                 255
   Asn Glu Val Phe Met His Ile Lys Lys Val Arg Tyr Ser Phe Phe Ala
               260                 265                 270
   Lys Glu Phe Arg Thr Lys Arg Val Lys Glu Ala Tyr Gln Trp Tyr Cys
           275                 280                 285
   Glu Gln Leu Leu Tyr Arg His Val Val Leu Pro Ser Gly Glu Val Thr
       290                 295                 300
   Leu Gln Thr Arg Gly Asn Pro Ser Gly Gln Ile Ser Thr Thr Met Asp
   305                 310                 315                 320
   Asn Asn Met Val Asn Val Phe Leu Gln Ala Phe Glu Tyr Ala Tyr Leu
                   325                 330                 335
   His Pro Asp Cys Thr Asp Glu Glu Leu Glu Ala Asn Trp Ser Arg Met
               340                 345                 350
   Asp Thr Leu Val Tyr Gly Asp Asp Arg Leu Ser Ala Tyr Ile Gly Leu
           355                 360                 365
   Pro Ala Asn Tyr Val Glu Lys Val Val Ile Met Tyr Ser Asn Val Phe
       370                 375                 380
   Gly Met Trp Val Lys Pro Glu Lys Val Ile Val Ser Pro Ser Ile Ile
   385                 390                 395                 400
   Gly Leu Thr Phe Cys Gly Leu Arg Ile Thr Ser Asp Gly Thr Ile Phe
                   405                 410                 415
   Leu Pro Val Pro Ala Glu Thr Asn Lys Leu Ile Ser Ala Leu Ile Lys
               420                 425                 430
   Pro Thr Lys Lys Leu Gln Asp Val Met Thr Leu Tyr Gly Lys Leu Leu
           435                 440                 445
   Cys Tyr Arg Ile Leu Gly His Asn Leu Pro Asp Asp Asn Lys Phe Lys
       450                 455                 460
   Ala Tyr Ile Leu Val Ala Leu Glu Val Val Ala Arg His Ile Arg Thr
   465                 470                 475                 480
   Arg Cys Gly Asp Glu Pro Leu Arg Phe Thr Asp Glu Met Leu Asp Arg
                   485                 490                 495
   Leu Trp Arg Gly Gly Pro Lys Val Gly Tyr Gly Trp
               500                 505

<210>  16
<211>  508
<212>  PRT
<213>  Artificial

<220>
<223>  ORF1b polypeptide

<400>  16
   Met Asp Leu Lys Ala Trp Arg Arg Ser Leu Cys Glu Val Glu Arg Arg
   1               5                   10                  15
   Leu Val Pro Glu Gly Tyr Pro Leu Ile Gly Gly Val Pro Leu Asp Arg
               20                  25                  30
   Pro Ile Ser Asp Trp Asp Glu Pro Ile Asp Asp Leu Leu Leu Met Leu
           35                  40                  45
   Pro Ala Phe Lys Glu Asn Leu Asn Tyr Thr Pro Ala Val Trp Gly Pro
       50                  55                  60
   Glu Ala Tyr Ala Lys Ser Phe Asp Lys Phe Thr Tyr Arg Glu Pro Cys
   65                  70                  75                  80
   Pro Asn Ile Ala Ala Thr Tyr Pro Arg Glu Trp Ser Phe Ala Thr Lys
                   85                  90                  95
   Ala Leu Lys Arg Glu Tyr Ser Phe Leu Asp Gly Ser Val Leu Thr Asp
               100                 105                 110
   Ile Thr Ala Thr Ser Lys Asn Ala Asp Ser Thr Pro Ala Tyr Pro Lys
           115                 120                 125
   Ser Leu Tyr Trp Lys Thr Glu Ala Asp Tyr Leu Gly Glu Arg Gly Tyr
       130                 135                 140
   Gln Asp Tyr Ile Lys Gln Leu Asp Ala Ile Tyr Ser Gly Glu Arg Pro
   145                 150                 155                 160
   Asp Val Leu Trp Tyr Leu Phe Leu Lys Lys Glu Ile Leu Lys Val Glu
                   165                 170                 175
   Lys Val Glu Gln Ala Asp Ile Arg Gln Ile Val Cys Ser Asp Pro Ile
               180                 185                 190
   Phe Ala Arg Ile Gly Cys His Phe Glu Glu Asp Gln Asn Ile Arg Met
           195                 200                 205
   Lys Asn His Thr Arg Thr Arg Ser Gly Gln Cys Gly Trp Ser Pro Phe
       210                 215                 220
   Phe Gly Gly Phe Asn Asp Arg Ile Lys Arg Leu Cys Ala Cys Asp Pro
   225                 230                 235                 240
   Asp Cys Tyr Ile Glu Phe Asp Trp Thr Arg Phe Asp Gly Thr Ile Pro
                   245                 250                 255
   Asn Glu Val Phe Met His Ile Lys Lys Val Arg Tyr Ser Phe Phe Ala
               260                 265                 270
   Lys Glu Phe Arg Thr Lys Arg Val Lys Glu Ala Tyr Gln Trp Tyr Cys
           275                 280                 285
   Glu Gln Leu Leu Tyr Arg His Val Val Leu Pro Ser Gly Glu Val Thr
       290                 295                 300
   Leu Gln Thr Arg Gly Asn Pro Ser Gly Gln Ile Ser Thr Thr Met Asp
   305                 310                 315                 320
   Asn Asn Met Val Asn Val Phe Leu Gln Ala Phe Glu Tyr Ala Tyr Leu
                   325                 330                 335
   His Pro Asp Cys Thr Asp Glu Glu Leu Glu Ala Asn Trp Ser Arg Met
               340                 345                 350
   Asp Thr Leu Val Tyr Gly Asp Asp Arg Leu Ser Ala Tyr Ile Gly Leu
           355                 360                 365
   Pro Ala Asn Tyr Val Glu Lys Val Val Ile Met Tyr Ser Asn Val Phe
       370                 375                 380
   Gly Met Trp Val Lys Pro Glu Lys Val Ile Val Ser Pro Ser Ile Ile
   385                 390                 395                 400
   Gly Leu Thr Phe Cys Gly Leu Arg Ile Thr Ser Asp Gly Thr Ile Phe
                   405                 410                 415
   Leu Pro Val Pro Ala Glu Thr Asn Lys Leu Ile Ser Ala Leu Ile Lys
               420                 425                 430
   Pro Thr Lys Lys Leu Gln Asp Val Met Thr Leu Tyr Gly Lys Leu Leu
           435                 440                 445
   Cys Tyr Arg Ile Leu Gly His Asn Leu Pro Asp Asp Asn Lys Phe Lys
       450                 455                 460
   Ala Tyr Ile Leu Val Ala Leu Glu Val Val Ala Arg His Ile Arg Thr
   465                 470                 475                 480
   Arg Cys Gly Asp Glu Pro Leu Arg Phe Thr Asp Glu Met Leu Asp Arg
                   485                 490                 495
   Leu Trp Arg Gly Gly Pro Lys Val Gly Tyr Gly Trp
               500                 505

<210>  17
<211>  508
<212>  PRT
<213>  Artificial

<220>
<223>  ORF1b polypeptide

<400>  17

Met Asp Leu Lys Ala Trp Arg Arg Ser Leu Cys Glu Val Glu Arg Arg
1               5                   10                  15

Leu Val Pro Glu Gly Tyr Pro Leu Ile Gly Gly Val Pro Leu Asp Arg
            20                  25                  30

Pro Ile Ser Asp Trp Asp Glu Pro Ile Asp Asp Leu Leu Leu Met Leu
        35                  40                  45

Pro Ala Phe Lys Glu Asn Leu Asn Tyr Thr Pro Ala Val Trp Gly Pro
    50                  55                  60

Glu Ala Tyr Ala Lys Ser Phe Asp Lys Phe Thr Tyr Arg Glu Pro Cys
65                  70                  75                  80

Pro Asn Ile Ala Ala Thr Tyr Pro Arg Glu Trp Ser Phe Ala Thr Lys
                85                  90                  95

Ala Leu Lys Arg Glu Tyr Ser Phe Leu Asp Gly Ser Val Leu Thr Asp
            100                 105                 110

Ile Thr Ala Thr Ser Lys Asn Ala Asp Ser Thr Pro Ala Tyr Pro Lys
        115                 120                 125

Ser Leu Tyr Trp Lys Thr Glu Ala Asp Tyr Leu Gly Glu Arg Gly Tyr
    130                 135                 140

Gln Asp Tyr Ile Lys Gln Leu Asp Ala Ile Tyr Ser Gly Glu Arg Pro
145                 150                 155                 160

Asp Val Leu Trp Tyr Leu Phe Leu Lys Lys Glu Ile Leu Lys Val Glu
                165                 170                 175

Lys Val Glu Gln Ala Asp Ile Arg Gln Ile Val Cys Ser Asp Pro Ile
            180                 185                 190

Phe Ala Arg Ile Gly Cys His Phe Glu Glu Asp Gln Asn Ile Arg Met
        195                 200                 205

Lys Asn His Thr Arg Thr Arg Ser Gly Gln Cys Gly Trp Ser Pro Phe
    210                 215                 220

Phe Gly Gly Phe Asn Asp Arg Ile Lys Arg Leu Cys Ala Cys Asp Pro
225                 230                 235                 240

Asp Cys Tyr Ile Glu Phe Asp Trp Thr Arg Phe Asp Gly Thr Ile Pro
                245                 250                 255

Asn Glu Val Phe Met His Ile Lys Lys Val Arg Tyr Ser Phe Phe Ala
            260                 265                 270

Lys Glu Phe Arg Thr Lys Arg Val Lys Glu Ala Tyr Gln Trp Tyr Cys
        275                 280                 285

Glu Gln Leu Leu Tyr Arg His Val Val Leu Pro Ser Gly Glu Val Thr
    290                 295                 300

Leu Gln Thr Arg Gly Asn Pro Ser Gly Gln Ile Ser Thr Thr Met Asp
305                 310                 315                 320

Asn Asn Met Val Asn Val Phe Leu Gln Ala Phe Glu Tyr Ala Tyr Leu
                325                 330                 335

His Pro Asp Cys Thr Asp Glu Glu Leu Glu Ala Asn Trp Ser Arg Met
            340                 345                 350

Asp Thr Leu Val Tyr Gly Asp Asp Arg Leu Ser Ala Tyr Ile Gly Leu
        355                 360                 365

Pro Ala Asn Tyr Val Glu Lys Val Val Ile Met Tyr Ser Asn Val Phe
    370                 375                 380

Gly Met Trp Val Lys Pro Glu Lys Val Ile Val Ser Pro Ser Ile Ile
385                 390                 395                 400

Gly Leu Thr Phe Cys Gly Leu Arg Ile Thr Ser Asp Gly Thr Ile Phe
                405                 410                 415

Leu Pro Val Pro Ala Glu Thr Asn Lys Leu Ile Ser Ala Leu Ile Lys
            420                 425                 430

Pro Thr Lys Lys Leu Gln Asp Val Met Thr Leu Tyr Gly Lys Leu Leu
        435                 440                 445

Cys Tyr Arg Ile Leu Gly His Asn Leu Pro Asp Asp Asn Lys Phe Lys
    450                 455                 460

Ala Tyr Ile Leu Val Ala Leu Glu Val Val Ala Arg His Ile Arg Thr
465                 470                 475                 480

Arg Cys Gly Asp Glu Pro Leu Arg Phe Thr Asp Glu Met Leu Asp Arg
                485                 490                 495

Leu Trp Arg Gly Gly Pro Lys Val Gly Tyr Gly Trp

500                 505

<210>  18
<211>  21
<212>  DNA
<213>  Artificial

<220>
<223>  primer

<400>  18
aaccytctcc acataattgg c                                                21

<210>  19
<211>  22
<212>  DNA
<213>  Artificial

<220>
<223>  primer

<400>  19
gaagtgttta tgcacatcaa ga                                               22

<210>  20
<211>  20
<212>  DNA
<213>  Artificial

<220>
<223>  primer

<400>  20
ggcgtactca aatgcttgaa                                                  20

<210>  21
<211>  20
<212>  DNA
<213>  Artificial

<220>
<223>  primer

<400>  21
gcgaaggagt ttaggacgaa                                                  20

<210>  22
<211>  20
<212>  DNA
<213>  Artificial

<220>
<223>  primer

<400>  22
ggcccagtrt taggbgcatt                                                  20

<210>  23
<211>  20
<212>  DNA
<213>  Artificial

<220>
<223>  primer

<400>  23
accatytggc agatagtkga                                                    20


<210>  24
<211>  23
<212>  DNA
<213>  Artificial

<220>
<223>  primer

<400>  24
ccagtrttag gbgcattaat ctg                                                23


<210>  25
<211>  21
<212>  DNA
<213>  Artificial

<220>
<223>  primer

<400>  25
catytggcag atagtkgatg a                                                  21

## Description

### Field of the Invention

The present invention relates to a novel type of porcine astroviruses and the uses thereof. The invention also relates to parts and subunits and variants of said viruses, compositions comprising the same, their manufacture, and their uses e.g., for the design and production of vaccines for treating pigs. The invention also concerns methods for treating and/or preventing astrovirus-associated diseases, as well as methods and materials for detecting an infection.

### Background of the Invention

Astroviruses are small, non-enveloped viruses with single stranded 6.2-7.8 kb-long RNA genome of positive polarity. The family *Astroviridae* is currently divided into two genera: the genus *Mamastrovirus* of mammal-infecting viruses and the genus *Avastrovirus* of avian viruses. The genetically heterogenic astroviruses that are widespread among mammals and avian species are generally associated with gastroenteritis. In this regard, porcine astroviruses (PoAstVs) were identified mainly from diarrhoeic faecal samples, although the etiological role of astrovirus infection in gastroenteritis or in other diseases among swine is not settled.

The applicant has succeeded in detecting, obtaining and characterizing new astrovirus strains from brain and spinal cord samples obtained from diseased pigs in Europe. These viruses were detected from samples of pigs with neurological disorders, but not in healthy pigs. The virus was isolated from pigs showing clinical signs of posterior leg weakness/paraplegia, pitching (Stage 1),; paralysis of both legs, skin pain (Stage 2), loss of consciousness, paresis and serious spastic paralysis of muscles (end stage, Stage 3) with high mortality rate among newly weaned (25-35-day-old) pigs. Affected pigs that were necropsied had encephalomyelitis and neural necrosis. The four strains (KY073229, KY073230, KY073231 and KY073232 family *Astroviridae*) were identified by RT-PCR and *in situ* hybridization (ISH) in brain and spinal cord samples in 6 cases and referred to as "Neuroinvasive porcine astroviruses". Among tissues tested by quantitative PCR, the highest viral loads were present in brainstem and spinal cord. Neuroinvasive porcine astroviruses were also detected in archived, formalin fixed paraffin embedded (FFPE) brain and spinal cord samples from other affected pigs and/or from other farms. Viral RNA was predominantly restricted to neurons, particularly in the brainstem, cortex, cerebellum (Purkinje cells), and all parts of the spinal cord. The virus was generally undetectable from faeces but present in respiratory samples indicating a possible respiratory infection. The virus was also detectable in other internal organs/tissues such as sera, lung, tonsils, hearth, submandibular lymph node and salivary glands

The applicant has sequenced the complete genomes of three strains (KY073229, KY073231 and KY073232) and the complete ORF2 of a fourth strain (KY073230). The strains exhibit a strong homology with each other at the nucleotide level, typically exceeding 99%, and much lower with previously known, essentially gastroenteric, astrovirus strains, typically below 91%. These new strains can thus be considered as being representative of a new clade of porcine astroviruses, designated in the present as neuroinvasive porcine type-3 astroviruses (NI-PoAstV-3).

The invention further discloses the sequence of proteins encoded by these viruses, including the capsid protein (ORF2) and well as regulatory proteins ORFla and ORFlab. The invention discloses a very high level of identity between the ORF proteins of this new clade of viruses, as well as distinctive sequence variations as compared to other astrovirus capsid proteins.

The invention thus reports isolation and characterization of a novel class of neuroinvasive porcine astroviruses and provides novel methods, reagents and compositions for treating, preventing or detecting such infections and related diseases.

### Summary

An object of the invention relates to isolated neuroinvasive porcine type-3 astroviruses, as well as parts, subunits and variants thereof. The viruses may be native, attenuated, or inactivated (e.g. killed). Such viruses refer to a clade of viruses having at least 91% nucleotide sequence identity over the entire genome to any of KY073229, KY073230, KY073231 or KY073232, preferably at least 95%, more preferably at least 99%, and being preferably neuroinvasive in swine.

The invention particularly relates to an isolated porcine astrovirus comprising:
(a) a nucleotide sequence as set forth in SEQ ID NO: 1 or SEQ ID NO: 2 or SEQ ID NO: 3 or SEQ ID NO: 4, or the complementary strand thereof, and/or
(b) an ORF2 nucleic acid sequence selected from nucleotide residues nucleotide residues 4075 to 6339 of anyone of SEQ ID NOs: 1 to 3, SEQ ID NO: 4, or the complementary strand thereof, and/or
(c) an ORF2 nucleic acid sequence encoding an ORF2 polypeptide comprising or consisting of the amino acid sequence of anyone of SEQ ID NOs: 5 to 8 or the complementary strand thereof, and/or
(d) an ORF1a nucleic acid sequence selected from nucleotide residues 31 to 2559 of anyone of SEQ ID NOs: 1 to 3 or the complementary strand thereof, and/or
(e) an ORFla nucleic acid sequence encoding an ORFla polypeptide comprising or consisting of the amino acid sequence of anyone of SEQ ID NOs: 9 to 11 or the complementary strand thereof, and/or
(f) an ORFlab nucleic acid sequence selected from nucleotide residues 31 to 4085 of anyone of SEQ ID NOs: 1 to 3 or the complementary strand thereof, and/or
(g) an ORFlab nucleic acid sequence encoding an ORFlab polypeptide comprising or consisting of the amino acid sequence of anyone of SEQ ID NOs: 12 to 14 or the complementary strand thereof, or
(h) an ORF1b nucleic acid sequence selected from nucleotide residues 2559 to 4085 of anyone of SEQ ID NOs: 1 to 3 or the complementary strand thereof, and/or
(i) an ORF1b nucleic acid sequence encoding an ORF1b polypeptide comprising or consisting of the amino acid sequence of anyone of SEQ ID NOs: 15 to 17 or the complementary strand thereof, or
(j) a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% to a nucleotide sequence of anyone of (a) to (i), or
(k) a variant sequence of anyone of (a) to (j) within the degeneration of the genetic code.

The invention particularly relates to isolated porcine type-3 astroviruses comprising:
(a) a nucleotide sequence as set forth in SEQ ID NO: 1 or SEQ ID NO: 2 or SEQ ID NO: 3 or the complementary strand thereof, or
(b) a nucleotide sequence having a homology of at least 91%, at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

Most preferably, such viruses are neuroinvasive in swine.

The invention also relates to isolated porcine type-3 astroviruses comprising an ORF2 nucleic acid sequence selected from:
(a) nucleotide residues 4075 to 6339 of anyone of SEQ ID NOs: 1 to 3, SEQ ID NO: 4, or the complementary strand thereof, or
(b) a nucleotide sequence having a homology of at least 87%, at least 88%, at leasdt 89%, at least 90%, at least 91%, at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORF2 nucleic acid sequence selected from:
(a) a nucleic acid sequence encoding an ORF2 polypeptide comprising or consisting of the amino acid sequence of SEQ ID NO: 5, SEQ ID NO: 6 or SEQ ID NO: 7 or SEQ ID NO: 8, or the complementary strand thereof, or
(b) a nucleotide sequence having a homology of at least 87%, at least 88%, at least89, at least 90%, at least 91%, at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORFla nucleic acid sequence selected from:
(a) nucleotide residues 31 to 2559 of anyone of SEQ ID NOs: 1 to 3 or the complementary strand thereof, or
(b) a nucleotide sequence having a homology of at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORFla nucleic acid sequence selected from:
(a) a nucleic acid sequence encoding an ORFla polypeptide comprising or consisting of the amino acid sequence of SEQ ID NO: 9 or SEQ ID NO: 10 or SEQ ID NO: 11, or the complementary strand thereof, or
(b) a nucleotide sequence having a homology of at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORFlab nucleic acid sequence selected from:
(a) nucleotide residues 31 to 4085 of anyone of SEQ ID NOs: 1 to 3 or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORFlab nucleic acid sequence selected from:
(a) a nucleic acid sequence encoding an ORFlab polypeptide comprising or consisting of the amino acid sequence of SEQ ID NO: 12 or SEQ ID NO: 13 or SEQ ID NO: 14, or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORF1b nucleic acid sequence selected from:
(a) nucleotide residues 2559 to 4085 of anyone of SEQ ID NOs: 1 to 3 or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORF1b nucleic acid sequence selected from:
(a) a nucleic acid sequence encoding an ORF1b polypeptide comprising or consisting of the amino acid sequence of SEQ ID NO: 15 or SEQ ID NO: 17 or SEQ ID NO: 17, or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

A further object of the invention is a host cell containing a virus as defined above.

Another object of the invention relates to a method of producing a porcine astrovirus, comprising seeding a competent host cell with a seed of culture of a virus as defined above, culturing or growing the cells under suitable conditions in a manner allowing production of the virus, and optionally harvesting the porcine astrovirus from said cells.

The invention also relates to a nucleic acid molecule comprising:
(a) a nucleotide sequence as set forth in SEQ ID NO: 1 or SEQ ID NO: 2 or SEQ ID NO: 3 or SEQ ID NO: 4, or
(b) a nucleotide sequence comprising or consisting of nucleotide residues 4075 to 6339 of anyone of SEQ ID NOs: 1 to 3, or nucleotide residues 31 to 2559 of anyone of SEQ ID NOs: 1 to 3, or nucleotide residues 31 to 4085 of anyone of SEQ ID NOs: 1 to 3, or nucleotide residues 2559 to 4085 of anyone of SEQ ID NOs: 1 to 3, or the complementary strand thereof, or
(c) a nucleic acid sequence encoding a polypeptide comprising or consisting of the amino acid sequence of SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15, or SEQ ID NO: 16, or SEQ ID NO: 17, or the complementary strand thereof, or
(d) a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% to a nucleotide sequence of (a), (b) or (c), or
(e) a variant sequence of any of (a) to (d) within the degeneration of the genetic code, or
(f) a fragment of a sequence of any of (a) to (e) of at least 10 consecutive nucleotides.

The invention also relates to polypeptides (e.g., proteins or peptides) encoded by or derived from such nucleic acid molecules.

A particular object of the invention is a polypeptide selected from:
(a) a polypeptide comprising or consisting of the amino acid sequence of anyone of SEQ ID NOs: 5 to 17,
(b) a polypeptide having a homology of at least 90%, at least 95%, at least 96%, at least 97%, at least 98%, or at least 99% to an amino acid sequence of (a), or
(c) a variant sequence of any of (a) or (b) containing conservative amino acid modification(s), or
(d) a fragment of a sequence of any of (a) to (c) of at least 5, preferably at least 6, at least 7, at least 8, at least 9, or at least 10 consecutive amino acids.

A further object of the invention resides in a vector, recombinant virus, plasmid or host cell comprising a nucleic acid molecule as defined above or encoding a polypeptide as defined above.

A further object of the invention is a composition or vaccine comprising a virus, a subunit thereof, a polypeptide, a nucleic acid, vector, or host cell as defined above, and a veterinary acceptable vehicle or excipient.

The invention also relates to a composition or vaccine as defined above for use for treating and/or preventing a porcine astrovirus infection or associated disease, or for inducing or stimulating an immune response against porcine astrovirus in a mammal.

The invention also provides a method of inducing or stimulating an immune response against porcine astrovirus in a mammal, comprising administering to the mammal an effective amount of a composition or vaccine as defined above.

The invention further relates to a method of treating and/or preventing a porcine astrovirus-associated disease in an animal comprising administering to said animal an effective amount of a composition or vaccine as defined above.

The invention also provides a method of treating and/or preventing encephalomyelitis or paralysis in an animal, comprising administering to said animal an effective amount of a composition or vaccine as defined above.

A further object of the invention is a container or kit comprising an effective amount a composition or vaccine as defined above. In a particular embodiment, the kit comprises the container and an instruction manual including information for the administration of the composition or vaccine into pigs for treating and/or preventing porcine astrovirus-associated diseases.

The invention also discloses and provides diagnostic kits or reagents comprising nucleic acid probes or primers or antibodies or ligands that bind to a NI-PoAst3 virus or polypeptide. Preferred primers or probes comprise a fragment of a nucleotide sequence as defined above.

The invention particularly relates to a polyclonal or monoclonal antibody, or a variant thereof, which is capable of binding to a virus or cell or polypeptide of the invention, or sub-units thereof. Preferably, the antibody preferentially binds to a virus or cell or polypeptide of the invention essentially without binding a type 1 or type 2 astrovirus, or essentially without binding a type 3 astrovirus of a different clade.

The invention also relates to a method of diagnosing the presence of a virus in an animal comprising contacting a tissue or fluid sample with the diagnosing reagent as defined above, and detecting the presence of a virus.

### Brief description of the drawings

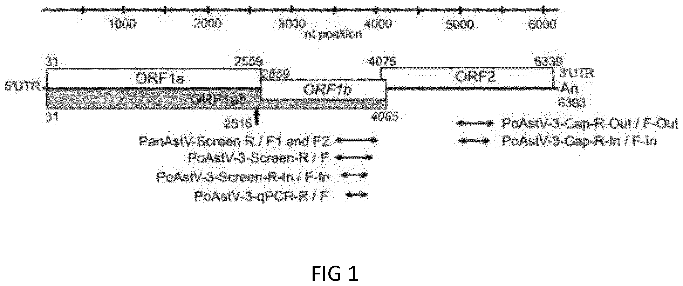

Figure 1: Genome map of the neuroinvasive porcine astrovirus strain NI-Brain/9-2016a/HUN (KY073229) together with the location of RT-PCR product used for different astrovirus screening reactions and RT-qPCR analyses. The black arrow indicates the localization of a ribosomal frame-shift during the synthesis of ORFlab peptide.

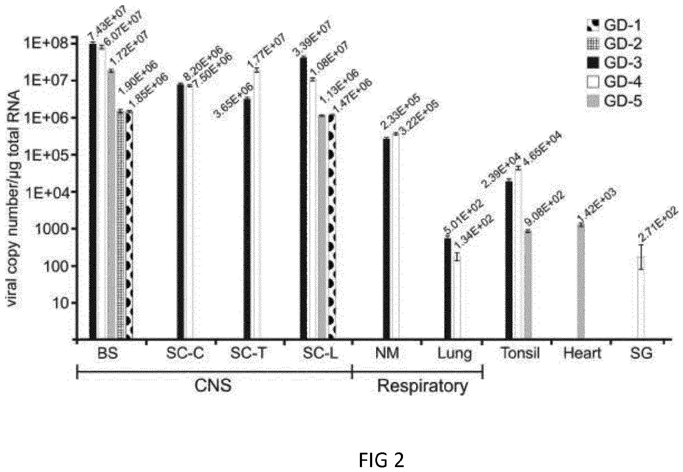

Figure 2: Logarithmic graph of the viral copy numbers of porcine astrovirus 3 in different organs determined by SYBR Green based RT-qPCR. BS: brainstem; SC-C/T/L: cervical-, thoracic-, or lumbar spinal cord; NM: nasal mucosa; SG: salivary gland; CNS: central nervous system. The analyzed samples were the same as in Table 1 and originated from five different animals with the IDs found in the top right corner of the picture. All the samples which were porcine astrovirus 3 positive only by nested RT-PCR were found negative by qPCR.

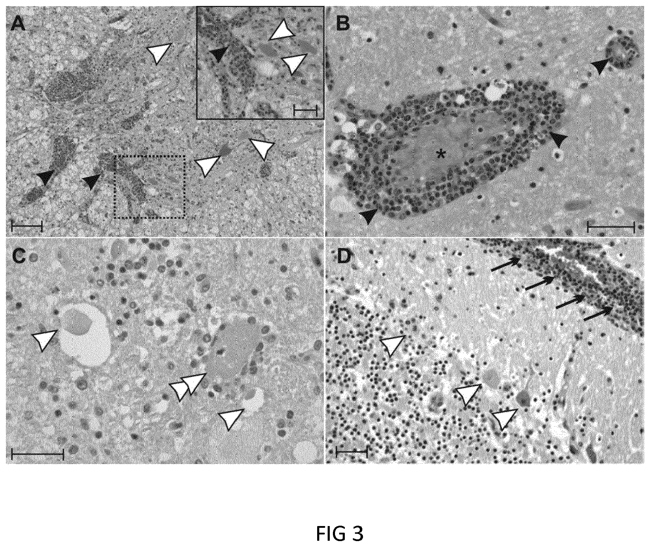

Figure 3: Tissue sections of cervical spinal cord (A), brainstem (B, C) and cerebellum (D) stained with hematoxylin and eosin from a pig shows the signs of end-stage encephalomyelitis (Stage 3). The mononuclear perivascular cuffs with vasculitis (black arrowheads) together with significant neuronal necrosis (white arrowheads), neurophagia (white double arrowheads) and multifocal microgliosis are present in all sections. The black arrows in section D indicate the signs of meningitis. Scale bars: A, D: 50µm, Insert A, B, C: 20µm.

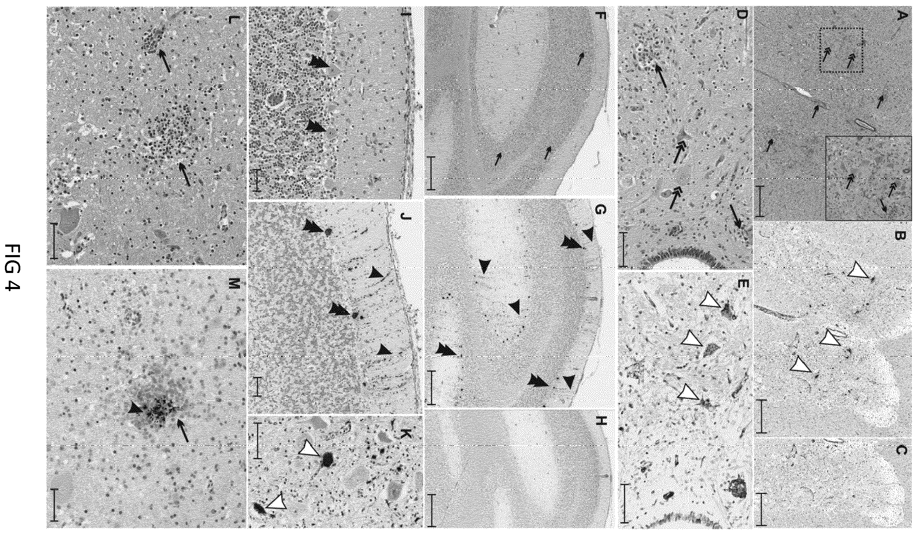

Figure 4: Sections of the cervical spinal cord (A-E), cerebellum (F-J), cortex (L, M) from the index animal (GD-1) and brainstem (K) from an additional affected Stage 1 animal (GD-7). Hematoxylin and eosin: A, D, F, I, L. Gliosis (black arrows) is multifocal within the gray matter (A,D) and in the molecular layers (F, I, L and M). Neuronal degeneration and necrosis is evident by hypereosinophilia, angular degeneration, loss of neuronal detail, and vacuolation (double arrows) (A, D). Some Purkinje neurons are slightly angular with mild vacuolation (double arrowheads) (I). ISH, neuroinvasive porcine astrovirus: B, E, G, J, K, M. Hybridization of the neuroinvasive porcine astrovirus probe is restricted to neurons (white arrowheads, B, E, K) or limited to Purkinje neurons (double black arrowheads, G, J) with extension into dendritic processes that course through the molecular layer (black arrowheads G, J). Hybridization of the porcine astrovirus 3 probe (black arrowhead, M) is present in the gliosis (black arrows, L, M). C, H: Using a control probe on a serial section, no hybridization is detectable. *In situ* hybridization, DapB probe. Scale bars: A-C, F-H,: 500µm; D,E, I-M: 50µm.

### Detailed description of the invention

The invention defines a novel clade of astroviruses and provides means, compositions and methods for producing such viruses, subunits thereof, material derived therefrom, as well as detecting, preventing and treating infections associated with such viruses in mammals, particularly in pigs.

### Neuroinvasive porcine type-3 astroviruses

The invention relates to any neuroinvasive porcine astrovirus capable of being isolated from a nervous tissue sample of a pig, especially brain or spinal cord.

The present invention more particularly relates to purified or isolated preparations of NI-PoAstV-3 strains, the sequences of which were deposited at Genbank under the following numbers, as well as any neuroinvasive porcine astroviruses having a significant serological and/or structural similarity with these strains. The sequences of the deposited strains are detailed below:
Strain NI-Brain/9-2016a/HUN, Genbank No. KY073229: SEQ ID NOs: 1 (genome), 5 (ORF2 protein), 9 (ORF1a protein), 12 (ORFlab protein) and 15 (ORF1b protein);
Strain NI-SC/9-2016a/HUN, Genbank No. KY073230: SEQ ID NOs: 4 (ORF2 nucleotides) and 6 (ORF2 protein);
Strain NI-Brain/173-2016a/HUN, Genbank No. KY073231: SEQ ID NOs: 2 (genome), 7 (ORF2 protein), 10 (ORF1a protein), 13 (ORFlab protein) and 16 (ORF1b protein);
Strain NI-Brain/386-2015/HUN, Genbank No. KY073232: SEQ ID NOs: 3 (genome), 8 (ORF2 protein), 11 (ORF1a protein), 14 (ORFlab protein) and 17 (ORF1b protein).

The invention particularly relates to isolated porcine type-3 astroviruses comprising
(a) a nucleotide sequence as set forth in SEQ ID NO: 1 or SEQ ID NO: 2 or SEQ ID NO: 3 or the complementary strand thereof, or
(b) a nucleotide sequence having a homology of at least 91%, at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

By comparing the entire nucleotide sequences of these viral genomes to known viral genome sequences, only about 90% sequence identity could be identified. In contrast, between each other these genomes share high level identity, typically above 98%. It is thus concluded these viruses represent a novel clade of astroviruses, which may be defined by a level of sequence identity above 91%, preferably above 95%, even more preferably above 98%.

In a particular embodiment, the astroviruses of the invention comprise SEQ ID NO: 1 or SEQ ID NO: 2 or SEQ ID NO: 3, or the complementary strand thereof, or a nucleotide sequence having at least 98% sequence identity therewith.

The applicant has further analyzed the nucleotide sequences of these viruses and identified open reading frames encoding proteins ORF2, ORF1a, ORF1b and ORFlab. These ORFs are located at nucleotide positions 31 to 4085 (ORFlab), 31 to 2559 (ORF1a), 2559 to 4085 (ORF1b) and 4075 to 6339 (ORF2). By analyzing such sequences at the nucleotide and amino acid level, it has also been found that these sequences distinguish substantially from the protein sequences of previously known astroviruses. In particular, homology levels as low as below 90% were found for the ORF2 nucleotide sequences, more specifically of 87% at the most. Such low level of identity further confirms the distinctive character of the present viruses.

The invention thus also relates to isolated porcine type-3 astroviruses comprising an ORF2 nucleic acid sequence selected from:
(a) nucleotide residues 4075 to 6339 of anyone of SEQ ID NOs: 1 to 3, or SEQ ID NO: 4, or the complementary strand thereof, or
(b) a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORF2 nucleic acid sequence selected from:
(a) a nucleic acid sequence encoding an ORF2 polypeptide comprising or consisting of the amino acid sequence of SEQ ID NO: 5 or SEQ ID NO: 6 or SEQ ID NO: 7 or SEQ ID NO: 8, or the complementary strand thereof, or
(b) a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

In a preferred embodiment, the invention relates to any isolated porcine astrovirus comprising an ORF2 nucleic acid sequence selected from an ORF2 polypeptide comprising or consisting of the amino acid sequence of SEQ ID NO: 5 or SEQ ID NO: 6 or SEQ ID NO: 7 or SEQ ID NO: 8, the complementary strand thereof, or a nucleotide sequence having a homology of at least 97%, at least 98%, or at least 99% thereto.

In a particular embodiment, the ORF2 nucleic acid has at least 87% identity with nucleotide residues 4075 to 6339 of anyone of SEQ ID NOs: 1 to 3, or with SEQ ID NO: 4, or the complementary strand thereof, and encodes at least one of the amino acid residues listed in table 3 (the position being given by reference to SEQ ID NO: 5, 6, 7 or 8), e.g., R29, S34, R38, V55, T57, T61, L439, S453, F457, Y559, A570, N572, D581, 1601, S617, T628, S678, and/or 1696.

The invention also relates to isolated porcine type-3 astroviruses comprising an ORFla nucleic acid sequence selected from:
(a) nucleotide residues 31 to 2559 of any one of SEQ ID NOs: 1 to 3 or the complementary strand thereof, or
(b) a nucleotide sequence having a homology of at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORFla nucleic acid sequence selected from:
(a) a nucleic acid sequence encoding an ORFla polypeptide comprising or consisting of the amino acid sequence of SEQ ID NO: 9 or SEQ ID NO: 10 or SEQ ID NO: 11, or the complementary strand thereof, or
(b) a nucleotide sequence having a homology of at least 93%, at least 94%, at least 95%, at least 96%, at least 97%, at least 98%, or at least 99% to a nucleotide sequence of (a), or
(c) a variant sequence of (a) or (b) within the degeneration of the genetic code.

In a preferred embodiment, the invention relates to any isolated porcine astrovirus comprising an ORFla nucleic acid sequence selected from an ORFla polypeptide comprising or consisting of the amino acid sequence of SEQ ID NO: 9 or SEQ ID NO: 10 or SEQ ID NO: 11, the complementary strand thereof, or a nucleotide sequence having a homology of at least 97%, at least 98%, or at least 99% thereto.

The invention also relates to isolated porcine type-3 astroviruses comprising an ORFlab nucleic acid sequence selected from:
(a) nucleotide residues 31 to 4085 of anyone of SEQ ID NOs: 1 to 3 or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORFlab nucleic acid sequence selected from:
(a) a nucleic acid sequence encoding an ORFlab polypeptide comprising or consisting of the amino acid sequence of SEQ ID NO: 12 or SEQ ID NO: 13 or SEQ ID NO: 14, or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

In a preferred embodiment, the invention relates to any isolated porcine astrovirus comprising an ORFlab nucleic acid sequence selected from an ORFlab polypeptide comprising or consisting of the amino acid sequence of SEQ ID NO: 12 or SEQ ID NO: 13 or SEQ ID NO: 14, the complementary strand thereof, or a nucleotide sequence having a homology of at least 97%, at least 98%, or at least 99% thereto.

The invention also relates to isolated porcine type-3 astroviruses comprising an ORF1b nucleic acid sequence selected from:
(a) nucleotide residues 2559 to 4085 of anyone of SEQ ID NOs: 1 to 3 or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

The invention also relates to isolated porcine type-3 astroviruses comprising an ORF1b nucleic acid sequence selected from:
(a) a nucleic acid sequence encoding an ORF1b polypeptide comprising or consisting of the amino acid sequence of SEQ ID NO: 15 or SEQ ID NO: 16 or SEQ ID NO: 17, or the complementary strand thereof, or
(b) a nucleotide sequence having a homology of at least 92%, at least 93%, at least 94%, at least 95%, at least 96%, at least 97%, at least 98%, or at least 99% to a nucleotide sequence of (a), or
(c) a variant sequence of (a) or (b) within the degeneration of the genetic code.

In a preferred embodiment, the invention relates to any isolated porcine astrovirus comprising an ORFlab nucleic acid sequence selected from an ORF1b polypeptide comprising or consisting of the amino acid sequence of SEQ ID NO: 15 or SEQ ID NO: 16 or SEQ ID NO: 17, the complementary strand thereof, or a nucleotide sequence having a homology of at least 97%, at least 98%, or at least 99% thereto.

The viruses of the invention may be isolated from a physiological sample or from a tissue sample, and/or propagated or maintained on cell lines such as especially pig kidney or testis cells or cell lines, e.g., for their multiplication. They may also be produced by seeding a competent cell with a virus stock or a nucleic acid molecule containing a genome of such a virus, as defined in the present invention. In a particular embodiment, a virus is prepared by infecting a competent cell with a vector containing a nucleic acid genome of the virus and collecting the virus produced.

The NI-PoAstV-3 of the invention are typically provided in an isolated form. The term "isolated" is meant to refer to a virus strain which is not in a native environment of a wild-type virus. For example, the virus may be a component of a cell culture or other artificial medium where it can be propagated; a component or a cell culture supernatant; a component of a pharmaceutical composition; or partially or completely purified from its native environment. The viruses may be isolated or purified using techniques known per se in the art such as centrifugation, affinity purification, gel permeation techniques, chromatographic methods, and the like.

The degree of homology between two nucleic acid sequences may be determined by means of computer programs known in the art such as GAP provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453). Using GAP with the following settings for DNA sequence comparison: GAP creation penalty of 5. 0 and GAP extension penalty of 0.3. Nucleic acid molecules may be aligned to each other using the Pileup alignment software, available as part of the GCG program package, using, for instance, the default settings of gap creation penalty of 5 and gap width penalty of 0.3. When referring to a level of identity within the context of the present invention, it is typically referred to the level of identity over the entire sequence under comparison, with gaps if appropriate.

### Attenuated / killed viruses

In a particular embodiment, the invention relates to NI-PoAstV-3 viruses in attenuated or inactivated (i.e., killed) form.

The terms "killed" or "inactivated" refer to a significant or complete reduction in the infectivity of the virus(es). Typically, an inactivated virus is a virus which has been structurally altered and is unable to infect a cell susceptible to infection by a non-inactivated virus.

The term "attenuated" refers to a substantial reduction of pathogenicity, typically at least 50% reduction, more preferably at least 80% reduction. Typically, an attenuated virus can replicate and/or infect a cell, but essentially does not cause a disease in a mammal.

Methods of preparing inactivated viruses are well known in the art. Inactivation may be carried out by exposing the virus to a chemical agent such as formaldehyde, paraformaldehyde, β-propiolactone, ethyleneimine, binary ethyleneimine (BEI), thimerosal, or derivatives thereof. Alternatively, inactivation may be carried out by physical treatments such as heat treatment or sonication. Methods of inactivation are well known to those of skill in the art. The inactivated virus may be concentrated by conventional concentration techniques, in particular by ultrafiltration, and/or purified by conventional purification means, in particular using chromatography techniques including but not limited to gel-filtration, ultracentrifugation on a sucrose gradient, or selective precipitations, in particular in PEG.

Methods for attenuating a virus are also known per se in the art and comprise, e.g., the passage on cells, preferably passages on pig cells, especially lines, such as PK/15 cells (for example from 50 to 150, especially of the order of 100, passages).

### Nucleic acid molecules and vectors

The present invention relates to a nucleic acid molecule comprising a nucleotide sequence of all or part of the genome of a NI-PoAstV-3 virus, or a variant thereof. The nucleic acid molecule may comprise a sequence of an entire genome, or variants thereof. The nucleic acid may comprise a sequence of an ORF, or a fragment or variant thereof. The nucleic acid may comprise a regulatory sequence.

Particularly, the nucleic acid molecule comprises:
(a) a nucleotide sequence as set forth in SEQ ID NO: 1 or SEQ ID NO: 2 or SEQ ID NO: 3 or SEQ ID NO: 4, or
(b) a nucleotide sequence comprising or consisting of nucleotide residues 4075 to 6339 of anyone of SEQ ID NOs: 1 to 3, or nucleotide residues 31 to 2559 of anyone of SEQ ID NOs: 1 to 3, or nucleotide residues 31 to 4085 of anyone of SEQ ID NOs: 1 to 3, or nucleotide residues 2559 to 4085 of anyone of SEQ ID NOs: 1 to 3, or the complementary strand thereof, or
(c) a nucleic acid sequence encoding a polypeptide comprising or consisting of the amino acid sequence of SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15, or SEQ ID NO: 16, or SEQ ID NO: 17, or the complementary strand thereof, or
(d) a nucleotide sequence having a homology of at least 91%, at least 92%, at least 93%, at least 94%, at least 95%, at least 96%, at least 97%, at least 98%, or at least 99% to a nucleotide sequence of (a), (b) or (c), or
(e) a variant sequence of any of (a) to (d) within the degeneration of the genetic code, or
(f) a fragment of a sequence of any of (a) to (e) of at least 10 consecutive nucleotides.

In a particular embodiment, the nucleotide sequence in (d) has a homology of at least 99%, 99.1%, 99.2%, 99.3%, 99.4% 99.5%, 99.6%, 99.7%, 99.8%, or 99.9% with the reference nucleotide sequence.

In another particular embodiment, the fragment in (f) is a fragment of at least 15, at least 20, or at least 25 consecutive nucleotides of the reference nucleotide sequence, more preferably a fragment of less than 80 nucleotides, most preferably a fragment of between 20 and 70 consecutive nucleotides of the reference sequence, particularly of between 25 and 60.

The nucleic acid molecule may be double-stranded or single-stranded DNA or RNA, as well as analogues thereof such as those containing modified backbones. Single-stranded DNA or RNA may be the coding strand, also known as the sense strand, or it may be the non-coding strand, also referred to as the anti-sense strand.

The nucleic acid molecules according to the invention may be provided in the form of a nucleic acid molecule *per se* such as naked nucleic acid molecules; or incorporated in a vector, virus or host cell, either from prokaryotic or eukaryotic origin. Vectors include cloning and/or expression vectors that contain a nucleic acid molecule of the invention. The vectors of the present invention may, for example, comprise a transcription promoter, and a transcription terminator, wherein the promoter is operably linked with the nucleic acid molecule, and wherein the nucleic acid molecule is operably linked with the transcription terminator.

In a particular embodiment, the invention relates to a vector comprising a nucleotide sequence encoding an ORF2 polypeptide as defined in the present invention.

In a further particular embodiment, the invention relates to a vector comprising a nucleotide sequence encoding a fragment of an ORF2 polypeptide as defined in the present invention.

In another particular embodiment, the invention relates to a vector comprising a nucleotide sequence encoding an ORFla polypeptide as defined in the present invention.

In another particular embodiment, the invention relates to a vector comprising a nucleotide sequence encoding an ORFlab polypeptide as defined in the present invention.

The vector may be a plasmid, a cosmid, an artificial chromosome, a recombinant virus, and the like.

For the expression of such polypeptides or fragments thereof in vitro, E. coli or a baculovirus will be preferably used (U.S. Pat. No. 4,745,051). The coding sequence(s) or their fragments are integrated into the baculovirus genome (e.g. the baculovirus Autographa californica Nuclear Polyhedrosis Virus AcNPV) and the latter is then propagated on insect cells, e.g. Spodoptera frugiperda Sf9 (deposit ATCC CRL 1711). The subunits can also be produced in eukaryotic cells such as yeasts (e.g. Saccharomyces cerevisiae) or mammalian cells (e.g. CHO, BHK).

For the expression of such polypeptides in vivo, the vector may be a plasmid or a recombinant virus, such as a recombinant Swinepox virus or a recombinant adenovirus, for instance, or a baculovirus.

In a further embodiment, the invention provides a host cell transformed with a nucleic acid molecule of the invention as defined above. Suitable examples of host cells include eukaryotic and prokaryotic cells. Examples of eukaryotic cells include mammalian (*e.g.*, pig), fungal (e.g. Saccharomyces cerevisiae), insect and plant cells. Prokaryotic cells include, for example, bacteria such as E. coli.

Such cells may be maintained or cultivated in any suitable medium. They may be frozen or lyophilized, for storage or maintenance.

### Polypeptides

The invention also relates to polypeptides (or proteins) encoded by or derived from a virus or a nucleic acid molecule of the invention, as well as variants and fragments thereof. Polypeptides (including proteins or peptides) of the invention are preferably in isolated (or purified form). They may be isolated or obtained by purification from natural sources, or obtained by recombinant techniques or by chemical synthesis, or combinations thereof. When produced by chemical synthesis, they can contain unnatural amino acids. They may be further modified, e.g., to improve their stability or activity.

In a particular embodiment, the invention relates to a polypeptide encoded by ORF1a, ORF1b, ORFlab, or ORF2 of a NI-PoAstV-3 of the invention, or variants or fragments thereof.

A particular object of the invention is a polypeptide selected from:
(a) a polypeptide comprising or consisting of the amino acid sequence of SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15 or SEQ ID NO: 16 or SEQ ID NO: 17,
(b) a polypeptide having a homology of at least 90%, at least 91%, at least 92%, at least 93%, at least 94%, at least 95%, at least 96%, at least 97%, at least 98%, or at least 99% to an amino acid sequence of (a), or
(c) a fragment of a sequence of any of (a) or (b) of at least 5, preferably at least 6, at least 7, at least 8, at least 9, or at least 10 consecutive amino acids.

A polypeptide fragment designates typically a polypeptide containing at least 5 and up to 100 consecutive amino acids, preferably between 10 and 80 consecutive amino acids, even more preferably between 10 and 50, or between 10 and 30 consecutive amino acids. Preferred fragments are immunogenic, i.e., are able to induce an immune response (preferably an anti-NI-PoAstV-3 immune response) when administered to a mammal. In this regard, preferred fragments contain an epitope, which may be a T-cell or a B-cell epitope.

Specific examples of polypeptide fragments of the invention are represented by polypeptides comprising, or consisting essentially of, amino acid residues 1 to 10, 15-25, 30-50, 55 to 80 of anyone of SEQ ID NOs: 5 to 8.

Preferred polypeptides under (b) above include polypeptides having at least 97%, at least 98%, or at least 99% sequence identity to a polypeptide of (a). Such polypeptides typically contain one or more sequence variations as compared to the reference polypeptide, such as one or more amino acid deletion, substitution, or addition. In the case of a substitution, one or more amino acids may be replaced by "conservative" amino acids, i.e., amino acids which upon substitution do not substantially alter a biological or immunological property of the polypeptide. By way of example, conservative substitutions may include the replacement, for example, of leucine by valine or isoleucine, the replacement of aspartic acid by glutamic acid, or the replacement of arginine by lysine.

In a specific embodiment, a polypeptide of the invention is a polypeptide comprising or consisting essentially of an amino acid sequence SEQ ID NOs: 5, 6, 7 or 8, or which has at least 90%, preferably at least 95% sequence identity with SEQ ID NOs: 5, 6, 7 or 8, or a fragment of at least 10 consecutive amino acids thereof.

In a particular embodiment, a polypeptide of the invention is a polypeptide comprising or consisting essentially of amino acid sequence SEQ ID NO: 5 or a sequence having at least 87%, preferably at least 97% identity to SEQ ID NO: 5, or a fragment of at least 10 consecutive amino acids thereof, preferably at least 97%.

In a particular embodiment, a polypeptide of the invention is a polypeptide comprising or consisting essentially of amino acid sequence SEQ ID NO: 6 or a sequence having at least 87%, preferably at least 97% identity to SEQ ID NO: 6, or a fragment of at least 10 consecutive amino acids thereof.

In a particular embodiment, a polypeptide of the invention is a polypeptide comprising or consisting essentially of amino acid sequence SEQ ID NO: 7 or a sequence having at least 87%, preferably at least 97% identity to SEQ ID NO: 7, or a fragment of at least 10 consecutive amino acids thereof.

In a particular embodiment, a polypeptide of the invention is a polypeptide comprising or consisting essentially of amino acid sequence SEQ ID NO: 8 or a sequence having at least 87%, preferably at least 97% identity to SEQ ID NO: 8, or a fragment of at least 10 consecutive amino acids thereof.

Preferred ORF2 polypeptides of the invention contain at least one of the following amino acids (the position being given by reference to SEQ ID NO: 5, 6, 7 or 8): R29, S34, R38, V55, T57, T61, L439, S453, F457, Y559, A570, N572, D581, 1601, S617, T628, S678, and/or 1696.

Among the polypeptides according to the invention, polypeptides that are recognized by antibodies produced during infection by NI-PoAstV-3 are particularly advantageous, as well as polypeptides which can induce or stimulate anti-NI-PoAstV-3 antibodies.

As mentioned, the polypeptides may be further modified, e.g., to improve or modify their activity, immunogenicity, stability, etc. For instance, the polypeptides may be glycosylated. They may be modified (or designed) to include non-natural amino acids or non-peptide bonds. They may also be modified by lateral or terminal groups to increase their resistance to proteases.

### Compositions and vaccines

The invention relates to compositions, such as vaccines, comprising a virus, nucleic acid, vector, polypeptide or cell as defined above.

The term "vaccine" as used herein includes an agent which may be used to stimulate the immune system of an animal against a pathogen.

The compositions and vaccines may comprise a whole NI-PoAstV-3 virus, in native (live), attenuated, or inactivated (e.g., killed) form, a subunit or a portion thereof, a recombinant vector containing an insert encoding an immunogenic polypeptide thereof, a fragment of DNA capable of inducing an immune response, a NI-PoAstV-3 polypeptide, a cell containing such elements, or a combination thereof. Such compositions and vaccines can be prepared according to standard techniques well known to those skilled in the art.

In a first embodiment, the composition or vaccine comprises an inactivated NI-PoAstV-3 virus. As mentioned above, such inactivated viruses may be obtained by treating the virus with inactivating agents such as formalin or acids, or by irradiation with ultraviolet light or X-rays, for instance.

In another embodiment, the composition or vaccine comprises an attenuated NI-PoAstV-3 virus. As mentioned above, such attenuated viruses may be obtained by serial passage through cell cultures, or by gene deletions for instance.

In another embodiment, the composition or vaccine comprises a NI-PoAstV-3 subunit. Such subunit vaccines typically contain an antigenic polypeptide of a NI-PoAstV-3, e.g., a protein encoded by a NI-PoAstV-3 virus, or a fragment thereof, or a variant thereof. Preferably, a capsid protein, such as the protein encoded by the ORF2, is employed as the antigenic component of the subunit vaccine, or a fragment or variant thereof.

In another embodiment, the composition or vaccine comprises a vector encoding a NI-PoAstV-3 antigen. Such vectorized vaccines typically contain recombinant plasmid or virus containing a nucleic acid sequence encoding an antigenic polypeptide of a NI-PoAstV-3, e.g., a protein encoded by a NI-PoAstV-3 virus, or a fragment thereof, or a variant thereof. Preferably, the antigenic polypeptide is a capsid protein, such as the protein encoded by the ORF2, or a fragment or variant thereof. The vector may be a recombinant virus such as a baculovirus, or a swinepox virus, or a circovirus.

Vaccines of the invention typically comprise an immunologically effective amount of a NI-PoAstV-3 virus or antigenic material in a pharmaceutically or veterinary acceptable vehicle. As a result of the vaccination, pigs become at least partially or completely immune to NI-PoAstV-3 infections, or resistant to developing moderate or severe NI-PoAstV-3 infections. NI-PoAstV-3 vaccines may be used to elicit a humoral and/or a cellular response.

The amount required for an immunologically effective dose may vary depending on factors such as the age and general condition of the subject, the nature of the formulation and the mode of administration. Appropriate "effective amount" may be determined by one of ordinary skill in the art using only routine experimentation. For instance, methods are known in the art for determining or titrating suitable dosages of an astrovirus vaccine to find minimal effective dosages based on the weight of the non human animal subject, concentration of the vaccine and other typical factors. The dosage of the vaccine, concentration of components therein and timing of administering the vaccine, which elicit a suitable immune response, can be determined by methods such as by antibody titrations of sera, *e.g*., by ELISA and/or seroneutralization assay analysis and/or by vaccination challenge evaluation.

In a particular embodiment, vaccines according to the present invention comprise from 10³ to 10¹⁰ TCID₅₀ NI-PoAstV-3 viral particles per mL.

In another particular embodiment, vaccines according to the present invention comprise from 0.001 µg to 1g of a NI-PoAstV-3 polypeptide per mL. The polypeptide is typically an ORF2 polypeptide or a fragment thereof

In another particular embodiment, vaccines according to the present invention comprise from 0.001 µg to 1g of a NI-PoAstV-3 nucleic acid per mL. The nucleic acid is typically a vector containing a sequence encoding a NI-PoAstV-3 ORF2 protein or a fragment thereof

Compositions and vaccines may comprise other ingredients, well known by one of ordinary skill in the art, such as pharmaceutically acceptable carriers, excipients, diluents, adjuvants, freeze drying stabilizers, wetting or emulsifying agents, pH buffering agents, gelling or viscosity enhancing additives, and preservatives, depending on the route of administration.

Examples of pharmaceutically acceptable carriers, excipients or diluents include, but are not limited to demineralised or distilled water; saline solution; vegetable based oils such as peanut oil, arachis oil, safflower oil, olive oil, cottonseed oil, maize oil, sesame oil, or coconut oil; silicone oils, including polysiloxanes, such as methyl polysiloxane, phenyl polysiloxane and methylphenyl polysolpoxane; volatile silicones; mineral oils such as light liquid paraffin oil, or heavy liquid paraffin oil; squalane; cellulose derivatives such as methyl cellulose, ethyl cellulose, carboxymethylcellulose, carboxymethylcellulose sodium salt, or hydroxypropyl methylcellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3- butylene glycol or glycerin; fatty acid esters such as isopropyl palmitate, isopropyl myristate or ethyl oleate; polyvinylpyrrolidone; agar; carrageenan; gum tragacanth or gum acacia, and petroleum jelly. Typically, the carrier or carriers will form from 10% to 99.9% by weight of the vaccine composition and may be buffered by conventional methods using reagents known in the art, such as sodium hydrogen phosphate, sodium dihydrogen phosphate, potassium hydrogen phosphate, potassium dihydrogen phosphate, a mixture thereof, and the like.

Examples of adjuvants include, but are not limited to, aluminium hydroxide (alum), immunostimulating complexes, non-ionic block polymers or copolymers, cytokines (like IL-1, IL-2, IL-7, IFN-α, IFN-β, IFN-y, etc.), saponins, monophosphoryl lipid A (MLA), muramyl dipeptides (MDP) and the like. Other suitable adjuvants include, for example, aluminium potassium sulphate, heat-labile or heat-stable enterotoxin isolated from Escherichia coli, cholera toxin or the B subunit thereof, diphtheria toxin, tetanus toxin, pertussis toxin, Freund's incomplete or complete adjuvant, etc. Toxin-based adjuvants, such as diphtheria toxin, tetanus toxin and pertussis toxin may be inactivated prior to use, for example, by treatment with formaldehyde.

Examples of freeze-drying stabilizer may be for example carbohydrates such as sorbitol, mannitol, starch, sucrose, dextran or glucose, proteins such as albumin or casein, and derivatives thereof.

Vaccines may additionally comprise at least one immunogen from at least one additional pathogen, *e.g*., a pig pathogen such as Actinobacillus pleuropneunomia; Adenovirus; Alphavirus such as Eastern equine encephalomyelitis viruses; Balantidium coli; Bordetella bronchiseptica; Brachyspira spp., preferably B. hyodyentheriae, B.pilosicoli, B. innocens, Brucella suis, preferably biovars 1,2 and 3; Classical swine fever virus, African swine fever virus; Chlamydia and Chlamydophila sp. and preferably C. pecorum and C. abortus; Circovirus (preferably PCV1 or PCV2), Clostridium spp., preferably Cl. difficile, Cl. perfringens types A,B and C, Cl.novyi, Cl.septicum, Cl.tetani; Digestive and respiratory Coronavirus; Cryptosporidium parvum; Eimeria spp; Eperythrozoonis suis currently named Mycoplasma haemosuis; Erysipelothrix rhusiopathiae; Escherichia coli; Haemophilus parasuis, preferably subtypes 1,7 and 14; Hemagglutinating encephalomyelitis virus; Isospora suis ; Japanese Encephalitis virus; Lawsonia intracellularis; Leptospira spp., preferably Leptospira australis, Leptospira canicola, Leptospira grippotyphosa, Leptospira icterohaemorrhagicae, Leptospira interrogans, Leptospira Pomona and Leptospira tarassovi; Mannheimia haemolytica ; Mycobacterium spp. preferably, M.avium, M. intracellulare and M.bovis: Mycoplasma hyponeumoniae; Parvovirus; Pasteurella multocida; Porcine cytomegolovirus; Porcine parovirus, Porcine reproductive and respiratory syndrome virus: Pseudorabies virus; Rotavirus; Sagiyama virus ; Salmonella spp. preferably, S. thyhimurium and S.choleraesuis; Staphylococcus spp. preferably, S. hyicus; Streptococcus spp., preferably Strep. suis; Swine cytomegalovirus ; Swine herpes virus; Swine influenza virus; Swine pox virus; Toxoplasma gondii; Vesicular stomatitis virus and virus of exanthema of swine; or other isolates and subtypes of porcine circovirus.

Vaccines may be liquid formulations such as an aqueous solution, water-in-oil or oil-in-water emulsion, or water-in-oil-in-water emulsion, syrup, an elixir, a tincture, a preparation for parenteral, subcutaneous, intradermal, intramuscular or intravenous administration (e.g., injectable administration), such as sterile suspensions or emulsions. Such formulations are known in the art and are typically prepared by dissolution of the antigen and other typical additives in the appropriate carrier or solvent systems. Liquid formulations also may include suspensions and emulsions that contain suspending or emulsifying agents. Preferred PCV2 vaccines comprise inactivated PCV2B-Rm according to the invention in the forms of emulsions, for example water-in-oil or oil-in-water, which may be prepared according to well-known techniques in the art.

The route of administration can be percutaneous, via mucosal administration, or via a parenteral route (intradermal, intramuscular, subcutaneous, intravenous, or intraperitoneal). Vaccines compositions according to the present invention may be administered alone, or can be co-administered or sequentially administered with other treatments or therapies.

### Method of treatment

The present invention provides a method of immunizing or inducing immune response in mammals, particularly in pigs, comprising administering a virus, nucleic acid, polypeptide, host cell, composition or vaccine as described above, as well as method of vaccination in pigs, and methods of treating and/or preventing associated diseases.

The term "immunization" includes the process of delivering an immunogen to a subject. Immunization may, for example, stimulate or induce or maintain a level of antibody and/or cellular response which can neutralize or kill or suppress a pathogen in the immunized animal, such as pig.

NI-PoAstV-3 infections or associated diseases include various stages such as, in particular:
*Stage 1: Weaned Pig with Posterior Weakness:* In milder case and/or in early stage of the disease, lesions are confined to the lumbar part of the spinal cord resulting posterior weakness with motor inco-ordination, pitching and fibrillary muscle twitching.
   Otherwise the consciousness is intact, the behavior and interest of this pig towards his environment is normal, just imitating the so-called rooting behavior.
*Stage 2: Weaned Pig with Serious Tetraplegia:* The cervical, dorsal and lumbar parts of spinal cord are equally affected producing serious movement disorder of both the front and hind legs. The consciousness is intact.
*Stage 3: Newly Weaned Pigs with End Stage Encephalomyelitis:* The brainstem and cerebellum are most likely affected which result loss of consciousness, paresis, serious spastic paralysis of muscles. The animals react to pain, their sensory functions are retained.

The invention may be used to suppress or reduce or slow or avoid any of these symptoms and/or to prevent progression from one stage to another, or to eliminate any pain and suffering, or to reduce viral load in an infected mammal.

The method comprises administering a therapeutically effective dose of the vaccine. The vaccines of the invention can conveniently be administered intranasally, transdermally (*i.e.,* applied on or at the skin surface for systemic absorption), parenterally, ocularly, etc. The parenteral route of administration includes, but is not limited to, intramuscular, intravenous, intraperitoneal routes and the like.

The dosage of the vaccine made according to the present invention depends on the species, breed, age, size, vaccination history, health status of the animal to be vaccinated, as well as of the route of administration, *i.e.,* subcutaneous, intradermal, oral intramuscular or intravenous administration.

The vaccines of the invention can be administered as single doses or in repeated doses. The vaccines of the invention can be administered alone, or can be administered simultaneously or sequentially administered with one or more further compositions, such as for example other porcine immunogenic or vaccine compositions. Where the compositions are administered at different times the administrations may be separate from one another or overlapping in time.

In one embodiment, the vaccines or compositions containing the NI-PoAstV-3 immunogen are administered to a subject susceptible to or otherwise at risk for astrovirus infection to enhance the subject own immune response capabilities. The subject to which the vaccine is administered is in one embodiment a pig (including adults or young piglets). However, the subject may be any animal (*e.g*., non human mammal) in which it may be desirable to elicit an immune response to the virus.

Vaccines of the invention are preferably administered to pigs, adult pigs, but also to young pigs, piglets or to pregnant females. Vaccination of pregnant females is particularly advantageous as it confers passive immunity to the newborns via the transmission of maternal antibodies. The pigs may be less than 7, 6, 5, 4, 3, 2 or 1 week old; 1 to 6 weeks old; 2 to 5 weeks old; or 3 to 4 weeks old. Desirably, the vaccine is administered to a subject who has not yet been exposed to the NI-PoAstV-3 virus. Preferably, the subject is a pig which is in need of vaccination against NI-PoAstV-3-associated diseases.

The present invention provides a container comprising an immunologically effective amount the vaccine as described above. The invention also provides vaccination kits comprising an optionally sterile container comprising an immunologically effective amount of the vaccine, means for administering the vaccine to pigs, and optionally an instruction manual including information for the administration of the immunologically effective amount the composition into pigs for treating and/or preventing NI-PoAstV-3 associated diseases.

The invention provides a method of immunizing a pig against astroviral infection, or for preventing or treating an astrovirus-associated neurological disorder in a pig, comprising administering to the pig an immunogenically effective amount of a composition comprising any one or more of the following:
a) a NI-PoAstV-3 virus, preferably in attenuated or killed form;
b) a nucleic acid molecule as defined above;
c) a polypeptide as defined above; or
d) a host cell as defined above).

In one embodiment, the invention provides methods for immunizing or protecting a pig against at least one strain of porcine astrovirus, or for vaccinating a pig against a porcine astrovirus, comprising administering a vaccine or composition comprising an attenuated NI-PoAstV-3 strain.

In another embodiment, the invention provides methods for immunizing or protecting a pig against at least one strain of porcine astrovirus, or for vaccinating a pig against a porcine astrovirus, comprising administering a vaccine or composition comprising a NI-PoAstV-3 polypeptide, particularly an ORF2 protein or peptide.

In another embodiment, the invention provides methods for immunizing or protecting a pig against at least one strain of porcine astrovirus, or for vaccinating a pig against a porcine astrovirus, comprising administering a vaccine or composition comprising a vector comprising a nucleic acid encoding a NI-PoAstV-3 polypeptide, particularly an ORF2 protein or peptide.

Typically, the methods of the invention result in an amelioration of one or more clinical symptoms of an infection, such as a reduction of viral load, a reduction of microscopic lesions, a reduction in the level of viral nucleic acid in tissues. The method also can result in a reduction or prevention of viral infection, as well as in an improvement in the condition of the treated animal, such as a reduction of neurological affection and improvement in mobility.

### Detection and diagnosis

According to another aspect, the invention relates to a method of diagnosing the presence of an astrovirus (or of an anti-astrovirus antibody) in a mammal or in a sample, reagents used therefor, and diagnostic kits therefor.

Based on the nucleotide and amino acid sequences as described above, it is possible to produce reagents capable of recognizing porcine astroviruses of the invention in any sample. In particular, based on such sequences, the skilled artisan can prepare primers or probes or epitopes or antibodies characteristic of such sequences, and which may be used to identify a NI-PoAstV-3 in a sample, e.g., by specific amplification, hybridization, ligand binding, or the like.

In a particular embodiment, an aliquot of a biological sample is contacted with a nucleic acid probe, a nucleic acid primer, or a ligand, characteristic of at least one NI-PoAstV-3, and the presence or amount of complexes formed between said probe, primer, or ligand and analytes in the aliquot is determined. In such methods, the probe or ligand may be in solution or immobilized on a support, such as a microarray. Also, the biological sample may be treated prior to determination, e.g., diluted or concentrated or enriched for viruses or viral nucleic acid or anti-viral antibodies.

In a particular embodiment, detection is performed by selective hybridisation using nucleic probes, preferably immobilised on a support, such as a solid or semi-solid support having at least one surface, flat or not, for immobilising nucleic probes. Such supports are, for example, a slide, bead, membrane, filter, column, plate, etc. They can be made out of any compatible material, such as in particular glass, silica, plastic, fiber, metal, polymer, etc. The nucleic probes can be any nucleic acid (DNA, RNA, PNA, etc.), preferably single-stranded, with a sequence characteristic of (i.e., complementary and specific of a target region of) a viral RNA or DNA molecule. The probes typically comprise from 5 to 400 bases, preferably from 8 to 200, more preferentially less than 100, and even more preferentially less than 75, 60, 50, 40 or even 30 bases. The probes can be synthetic oligonucleotides, produced on the basis of the sequences of the target virus, according to standard synthesis techniques. In a preferred embodiment, the probe comprises all or part of a nucleic acid sequence selected from anyone of SEQ ID NOs: 1-4 or of a sequence complementary thereto. In a preferred mode, the nucleic acid probes has a length between 15 and 50 bases, more preferentially between 15 and 40 bases, and a sequence identical to a fragment of a sequence selected from anyone of SEQ ID NOs: 1-4 or the complementary strand thereof. The probe can also be designed in the opposite orientation. Such probes constitute another object of the present application, as well as the use thereof for diagnosing viral infection in a mammal.

In a particular embodiment, the method comprises contacting a sample to be analyzed to a support (such as a microarray) comprising at least one nucleic acid probe characteristic of a NI-PoAstV-3 nucleic acid under conditions allowing hybridization to occur, and detecting the presence of hybrids.

In another embodiment, the detection is performed by selective amplification using a primer or a pair of primers that amplifies all or part of a NI-PoAstV-3 nucleic acid. The primer can be specific for a target sequence within anyone of SEQ ID NOs: 1-4. The primer typically comprises a single-strand nucleic acid, with a length advantageously between 5 and 50 bases, preferably between 5 and 30 bases. Such a primer constitutes another object of the present application, as well as the use thereof (primarily *in vitro*) for diagnosing viral infection in a mammal.

In this respect, another object of the invention resides in the use of a set of nucleotide primers comprising at least 2 primers complementary to and specific of a target sequence in a nucleic acid selected from anyone of SEQ ID NOs: 1-4, or the complementary strand thereof, for *in vitro* or *ex vivo* diagnosis of viral infection in a mammal.

Another particular embodiment comprises contacting a sample with a set of at least two nucleic acid primers, said at least two nucleic acid primers specifically amplifying a sequence characteristic of a NI-PoAstV-3 viral nucleic acid, and determining the presence of an amplicon.

In another particular embodiment, the method comprises contacting the sample with an anti-NI-PoAstV-3 virus antibody, and detecting the presence of immune complexes. The present invention further relates to a method of generating an antibody which is capable of binding to a NI-PoAstV-3 virus or sub-units thereof. The method may comprise immunizing an animal, such as a rabbit, guinea pig, or rodent and harvesting the antibody produced thereby. The antibodies of the invention may be polyclonal or monoclonal antibody preparations, monospecific antisera, human antibodies, or may be derivatives thereof such as hybrid or chimeric antibodies, such as humanized antibodies, altered antibodies (Fab')2 fragments, F(ab) fragments, Fc fragments, single- domain antibodies, dimeric or trimeric antibody fragments or constructs, or functional fragments thereof which bind to the antigen in question. Antibodies may be produced using techniques well known to those of skill in the art and disclosed in "A Laboratory Manual, Harlow and Lane, eds., Cold Spring Harbor Laboratory, N.Y. (1988)".

A particular embodiment of the invention is an antibody, or a derivative thereof having essentially the same antigen specificity, which binds an ORF2 polypeptide as defined in the present invention.

Diagnostic kit thus may comprise nucleic acid probes or primers, antigens and/or antibodies specific for a NI-PoAstV-3 virus, and diagnostic testing may be performed on a sample of physiological fluid (blood, plasma, serum and the like) or a sample of tissue (ganglia, liver, lungs, kidneys and the like) obtained from a pig to be tested.

Further aspects and advantages of the invention are disclosed in the following experimental section, which illustrates the invention.

### Examples

### Materials and methods

### Collection and handling of the samples

Multiple tissue samples were collected from 6 paraplegic pigs and five asymptomatic animals (Table 1, 2), nasal and anal swab pairs from another 5 paraplegic and 13 healthy animals were also tested. The tissue samples were washed twice in 0.1 M phosphate buffered saline (PBS) to remove the excess blood and held in -80oC until total RNA extraction. The nasal swabs were acquired using two polyester-tipped swabs. For the FFPE blocks the dissected samples (Table 2) were fixed freshly with buffered 8% of formaldehyde, dehydrated and embedded into paraffin.
FFPE specimens from paraplegic pigs of past posterior paraplegia outbreaks in Tázlár and in Balmazújváros, were also analyzed (Table 2).
The three swine farms are located in the central and eastern parts of Hungary, approximately 100 km distance from each other without known connection.

### Previous laboratory diagnostics

CNS homogenates from the index farm were negative by PCR for the following pathogens: porcine reproductive and respiratory syndrome virus (*Arteriviridae*)*,* PCV2 (*Circoviridae*)*,* Hemagglutinating encephalitis virus (*Coronaviridae*)*,* porcine parvovirus 1, 2, 4 and porcine bocavirus (*Parvoviridae*)*.* Immunohistochemical detection of *Toxoplasma gondii* and West Nile virus were also negative. Bacterial cultivation attempts from the CNS samples were negative. Virus isolation attempts using brain homogenates of affected animals in swine kidney (PK-15) and caucasian colon adenocarcinoma (Caco-2) cell lines were not successful (no visible CPE was seen). There was no detectable PoAstV-3 in the cell culture supernatants by RT nested-PCR with RdRp primer pairs.

### Total RNA extraction and RT-PCR screening

The pretreatment of FFPE samples which include the deparaffination and rehydration steps and Proteinase K digestion as well as the total RNA extraction, the reaction conditions and reagents used in the RT and (nested) PCR reactions were the same as described previously with minor modifications *(24-26)*. For the RT-PCR screening of CNS samples for the presence of pestiviruses (*Flaviviridae*), and swine picornaviruses (*Picornaviridae*) including teschovirus, enterovirus, sapelovirus, Seneca Valley virus, pasivirus, kobuvirus and encephalomyocarditis virus specific primer pairs as well as the outer and inner primer pairs targeting the RdRp or the capsid regions of PoAstV-3 (Fig. 1) were used.

### Absolute quantification using RT-qPCR

For the absolute quantification of viral RNA present in different tissue, urine and faecal samples SYBR-Green based RT-qPCR (Maxima SYBR Green qPCR Master Mix, Thermo Scientific, USA) method was used. For the generation of standard curve tenfold dilution series of purified and spectrophotometrically quantified RNA transcripts were used in the RT reactions. The qPCR assays contained three technical repeats of every samples and standards. The slope of the standard curve was -3.4228 and the calculated PCR efficiency was 99.96%.
For the detailed protocol see the technical appendix.

### Long-range amplification, 5'/3' RACE-PCR and Sanger sequencing

For the complete genome (or complete 3'ORF1b-ORF2-3'UTR) acquisitions of the PoAstVs different long-range and 5'/3' RACE RT-PCR reactions were used according to the protocols described previously. The sequence specific primers used for the amplification of overlapping genome fragments were designed based on the genome of porcine astrovirus 3 strain US-MO123 (JX556691) and/or closely related sequences downloaded from the GenBank database. The PCR products were sequenced directly with the BigDye Terminator v1.1 Cycle Sequencing Ready Reaction Kit (Applied Biosystems, UK) using primer-walking method with an automated sequencer (ABI Prism 310 Genetic Analyzer; Applied Biosystems, Stafford, TX).

### Sequence and phylogenetic analyses

The astrovirus sequences were aligned using MUSCLE web tool of EMBL-EBI. The pairwise nucleotide (nt) and amino acid (aa) identity calculations of the aligned sequences were performed using GeneDoc ver.2.7. Phylogenetic trees of deduced amino acid sequence alignments were constructed using MEGA software ver. 6.06. The Neighbor-Joining method with the Jones-Taylor-Thornton matrix-based model was used for the construction of the trees. Bootstrap values were set up to 1000 replicates and only likelihood percentages of more than 50% were indicated.

### Histology and in situ hybridization

Chromogenic (with DAB) *in situ* hybridization in FFPE slides using thirty probe pairs generated at Advanced Cell Diagnostics (Newark, CA) designed to hybridize native viral PoAstV-3 RNA was carried out according to the manufacturer's instructions for nucleic acid detection (RNAscope 2.0, Brown Kit). Negative controls included 1) Dap-B 2) an unrelated viral probe, and 3) normal porcine brain region-matched sections.

### Example 1: Virus detection, isolation and structure

### Clinical observations

There are approximately 2000 sows and their offsprings in the investigated highly prolific index farm (GD). Episodes of neurologic disease of unknown etiology have persisted in the last 2 years with variable number of cases. The syndrome affects an average of 30-40 weaned pigs monthly (1.5-2% of total) although the number of monthly cases infrequently rose to around 80 per month (4%) in the autumn-winter seasons. The clinical signs of posterior leg weakness/paraplegia, pitching (Stage 1) and later paralysis of both legs, skin pain (Stage 2), loss of consciousness, paresis and serious spastic paralysis of muscles (Stage 3) typically appears among 25-35 days-old weaned pigs, one week after the weaning procedure. Gastroenteric symptoms were not observed. All of the affected pigs were unable to eat or drink and died due to exsiccosis (dehydration) or were euthanized. Signs persisted typically for 1 week prior to death or euthanasia. The post-mortem examination was negative showed no signs of mechanic damage (fractures, abscesses, or hemivertebrae). Pigs are vaccinated against porcine circovirus 2 (PCV2), *Mycoplasma hyopneumoniae* and *Actinobacillus pleuropneumoniae.* Preventive amoxicillin treatment of the piglets routinely was done every weaned period. Due to the preventive measures take into effect in spring, 2017 which includes extensive decontamination of the piggeries and the physical separation of the newly weaned pigs originated from different litters the number of encephalomyelitis cases among weaned pigs were decreased significantly, only monthly 1-2 cases were observable in the index farm.

The two additionally examined swine farms located in Tázlár and Balmazújváros each held 171 500 sows and their offsprings. Similar symptoms of staggering and paralysis described above 172 appeared among pigs between the age of 3 and 5 weeks in a 2011 (Tázlár) and a 2014 173 outbreak (Balmazújváros).

### Detection and analysis of astroviruses from CNS samples of affected animals

Brainstem (BS), spinal cord (SC), nasal swab (NS) and faecal samples were collected from a newly weaned pig (GD-1, index animal) showing signs of encephalomyelitis and posterior paraplegia (Stage 1) from index GD farm. The BS and SC samples tested negative by RT-PCR for the presence of pestivirus (family *Flaviviridae*), and several swine-infecting picornaviruses (family *Picornaviridae*)*. T*he presence of astrovirus was investigated using pan-astrovirus (Pan-AstV) PCR primers *(31).* The BS and SC samples showed strong RT-PCR positivity. The Pan-AstV PCR products were sequenced and compared to each other and to the available astroviruses using BLASTn. The 397-nt-long sequences of BS and SC were identical and showed 89% nt identity to a porcine astrovirus 3 strain US-MO123 (JX556691) as the closest match (*32*).

The full-length genome of the neuroinvasive astrovirus strain NI-Brain/9-2016a/HUN (KY073229) from the BS sample and the complete capsid-encoding ORF2 from the SC sample NI-SC/9-2016a/HUN (KY073230) both from the index animal were sequenced (see SEQ ID NO: 1 and SEQ ID NO: 2). The 6393-nt-long (without the poly(A)-tail) complete genome showed the typical astrovirus genome organization with three, 2529 nt, 1527 nt and 2265 nt long putative open reading frames (ORF1a, ORF1b and ORF2) flanked by short 5' and 3' untranslated regions (Fig. 1). The conserved proteolytic cleavage site (V561HQ↓TNT) of serine protease (ORF1a) and the conserved Y358GDD motif of the RdRp (ORF1b) were identified *(33).* The non-structural proteins of ORF1a (842 aa), ORF1b (508 aa) and the capsid protein of ORF2 (754 aa) showed 93%, 95% and 93% aa identity to the corresponding genome parts of the closest known relative PoAstV-3 strain US-MO123 (JX556691). All of the conserved genomic features of mamastroviruses were present in strain NI-Brain/9-2016a/HUN including (i) the conserved C1CAAA pentamer at the 5' end of the genome, (ii) the frame-shift heptamer motif (A2511AAAAAC) followed by a stem-loop structure at the 3' end of ORF1a, (iii) the conserved sgRNA promoter sequence motif of U4048UUGGAGgGGaGGACCaAAN8AUGgC (variable nts in lower case, start codon of ORF2 is underlined) at the junction of ORF1b/ORF2 and (iv) the stem loop II-like motif (s2m) in the 3' end of the genome between nt positions 6322 and 6353).
The 3'UTR of NI-Brain/9-2016a/HUN is 27 nt shorter and does not contain the short sequence repeat found at the 3' end of strain US-MO123 (G6381/6392AUUUCUUUNA).

Based on the sequence and the similar genomic features, it is concluded that NI-Brain/9-2016a/HUN strain most likely belongs to the PoAstV-3 genotype.

The ORF2 of NI-Brain/9-2016a/HUN shares 99% nt/aa identity to the corresponding capsid gene of NI-SC/9-2016a/HUN from the spinal cord of the same animal suggests that the same virus was present in both regions of the CNS.

PoAstV-3 was also detectable using RT-PCR in all CNS samples collected from another four affected newly weaned pigs held in the index farm (Table 1). All of the samples from the asymptomatic control animals were NI-PoAstV-3 negative.

The complete genomes of two additional PoAstV-3 strains (NI-Brain/173-2016a/HUN, KY073231 and NI-Brain/386-2015/HUN, KY073232) originated from another two affected animals (GD-3 and GD-5) in *Stage 3* of the disease were also determined (SEQ ID NO: 3 and 4). The two strains from GD-3 and GD-5 show 99.5%, 100% and 98.7-99.2% aa identities to the strain NI-Brain/9-2016a/HUN in the ORF1a, ORF1b and ORF2 (capsid) regions.

Most of the aa differences between the Ni-PoAstV-3 study strains and the other "enteric" PoAstV-3 strains are located (i) in the N-terminal part of ORF1a and (ii) the C-terminal part of ORF2 (Table 3).

### Example 2: Detection of NI-poAst-3 in samples from diseased pigs

### Detection of NI-PoAstV-3 in non-CNS samples

PoAstV-3 was detectable in multiple non-CNS samples from the respiratory system, the lymphoid system, the circular system and salivary glands of affected animals (Table 1). PoAstV-3 was detected only in a single ileum sample and two of the three analyzed faecal samples using RT- nested (n) PCR (Table 1). Samples from internal organs such as spleen, and kidney as well as the urine samples tested negative by RT-nPCR (Table 1).

The copy number of PoAstV-3 was determined using SYBR Green-based RT-qPCR. All of the PoAstV-3 RT-nPCR positive samples were negative by qPCR indicating low copy number (< ca.100 copies/µg total RNA) of the virus in that tissue samples. The highest copy number was detected in the brainstem followed by the samples of spinal cord (Fig. 2). Interestingly, relatively high copy numbers were detected in the tonsil and nasal mucosa samples (Fig. 2). The sera of GD-3 and GD-4 animals contained 2.07E+06 and 1.64E+03 copies/ml of PoAstV-3.
To validate the general presence of PoAstV-3 in the respiratory system and the absence of the virus in the faeces during the acute phase of the illness additional nasal and anal swab pairs were collected from N=5 affected and N=13 clinically healthy pigs of the same age (c.a. 25-35 days) from the index farm. Four of the five nasal swabs (80%) were positive while all of the anal swabs of the affected animals were negative using RT-nPCR with primers the following primers targeting the RdRp region of PoAstV-3 : first round: PoAstV3-Screen-R 5'-AAC CYT CTC CAC ATA ATT GGC-3' (SEQ ID NO: 18) / PoAstV3-Screen-F 5'- GAA GTG TTT ATG CAC ATC AAG A -3' (SEQ ID NO: 19); second/nested round: PoAstV3-Screen-R-in 5'- GGC GTA CTC AAA TGC TTG AA-3' (SEQ ID NO: 20) / PoAstV3-Screen-F-in 5'- GCG AAG GAG TTT AGG ACG AA-3' (SEQ ID NO: 21). The nasal and anal swabs of the asymptomatic animals were all RT-nPCR negative. Due to the variable amounts of samples collectable by polyester-tipped swabs which were unable to measure accurately the absolute quantification of PoAstV-3 by RT-qPCR of nasal and anal swabs were not performed.

### Detection of NI-PoAstV-3 in FFPE samples

All but one FFPE samples from Tázlár and Balmazújváros were RT-nPCR positive for PoAstV-3 using two sets of primer pairs targeting the RdRp and capsid genes of PoAstV-3 (Fig. 1; Table 2). The RdRp nested primers are the same as provided above. The capsid genes primers are: first round: PoAstV3-CAP-R-out 5'- GGC CCA GTR TTA GGB GCA TT-3' (SEQ ID NO: 22) /PoAstV3-CAP-F-out 5'- ACC ATY TGG CAG ATA GTK GA-3' (SEQ ID NO: 23); second/nested round: PoAstV3-CAP-R-in 5'- CCA GTR TTA GGB GCA TTA ATC TG-3' (SEQ ID NO: 24) / PoAstV3-CAP-Fin 5'- CAT YTG GCA GAT AGT KGA TGA-3' (SEQ ID NO:25). The spinal cord FFPE sample from Balmazújváros was negative using both RT-nPCR primer sets. The RT-nPCR positive samples were also positive by *in situ* hybridization (ISH) (data not shown).

### Histology and In Situ Hybridization (ISH)

Histologically, shared lesions among the cases examined included moderate to marked lympho-histiocytic cell perivascular cuffing with marked vasculitis and neuronal degeneration, necrosis and neurophagia with multifocal microgliosis and satellitosis (Fig. 3). The neuronal necrosis was especially evident in the dorsal and ventral horns of the cervical spinal cord gray matter although it was also detected in neurons of the Purkinje layer (cerebellum), the medulla oblongata, cerebellar peduncles and midbrain (Fig. 4). Necrotic neurons were variously swollen and hypereosinophilic or shrunken with tinctorial changes including faded, amphophilic, or eosinophilic cytoplasm (Fig. 4). Nuclei of affected neurons are pyknotic, karyorrhectic, or sometimes losing border definition with the cytoplasm. ISH was performed on 5 cases (Table 2). PoAstV-3 hybridization was predominantly restricted to neurons, including those with visible necrosis and, in the cerebellum in particular, some that were histologically unaffected, although there are some regions of gliosis (presumed inflammation after neuronal necrosis) that also contained viral RNA (Fig. 4M). Hybridization was distinct, with punctate to diffuse cytoplasmic staining throughout the cytoplasm. The unique microarchitecture of the Purkinje layer of the cerebellum offered the clear demonstration that viral nucleic acid was present within dendritic processes coursing through the molecular layer (Fig. 4G,J). There were no pathological lesions found in other samples from kidneys, liver, gastrointestinal tract or immune system. The samples from the latter were also negative by PoAstV-3 ISH (Table 2).

### Discussion

Astrovirus RNA was detected in multiple tissues from newly weaned pigs in Hungary with encephalomyelitis and posterior paraplegia of unknown origin with the highest viral load detected in brain and spinal cord. The same virus was also detected in brain and spinal cord FFPE samples from similarly affected animals from two additional swine herds collected between 2011 and 2014. These data define a genetically similar, neurovirulent astrovirus circulating in multiple swine farms in Hungary.

According to the refined classification for the assessment of causation by Lipkin and Anthony (2015) the Ni-PoAstV-3 and the observed encephalitis and paraplegia are in a probable causal relationship (Level 2).

Interestingly, neurological signs were observable mainly among newly weaned pigs. The time of weaning which include nutritional- (from milk to solid feed), social-, (mixing with different litters without the sow) and environmental changes (moving to a new pen) is known to be the most stressful period in the lifetime of pigs, and is associated with dysfunction of the immune system. Furthermore due to highly prolific sows with relatively large litters in the index farm, the inadequate quantity/quality of clostrum intake of sucking piglets and therefore the presumably low level of specific maternal antibodies might also be contributed to the emergence of the clinical disease. Decreased immune status was also frequently present with extra-intestinal dissemination of astroviruses in humans and in mice.

Based on the results of the sequence analyses, the identified astrovirus strains belong to the PoAstV-3 genotype which clusters within the VA/HMO phylogenetic clade.
At the molecular level, one important difference between the genomes of neuroinvasive and the "enteric" PoAstV-3 strain US-MO123 is the 27nt deletions found in the 3'UTR of the CNS associated astroviruses. This 3'UTR deletion may account for the nervous tropism of the viruses.
In aa level one of the most divergent region between the neuroinvasive and other PoAstV-3 was found at the Receptor-Interaction domain (RID) of ORF2 (Table 3) which contains potential receptor binding sites this could indicate an altered receptor spectra therefore altered tissue tropism of "neuroinvasive" and "enteric" PoAstV-3 strains.

Until now, PoAstV-3 strains were detected only from faecal samples of healthy or diarrhoeic piglets, worldwide. Interestingly, the PoAstV-3 was either undetectable or present only at low viral loads in the analyzed faecal samples while the virus was generally detectable in the respiratory system of paraplegic pigs. This may indicate that CNS infection/replication occur at a later date than enteric replication or that initial replication occurs extra-intestinal, e.g. in the respiratory tract.

The highest viral loads of PoAstV-3 were measured in brain and spinal cord samples similar as found in diseased ovine as well as human patients with astrovirus-associated encephalitis. PoAstV-3 was also detectable in the sera and multiple organs of the respiratory-, lymphoid- and cardiovascular systems of diseased swine. These results indicate that PoAstV-3 can result in viremia and disseminated infection involving the brain, spinal cord and multiple organs during the acute phase of encephalomyelitis and posterior paraplegia. Astrovirus there seem to plays a role in a common and severe disease (encephalomyelitis and paralysis) in pigs.

The observable histopathological changes as well as the neuronal localizations of PoAstV-3 RNA in CNS samples of paraplegic pigs are comparable to astrovirus-associated encephalitic cases of minks, humans and bovine. Similar neuronal degeneration/necrosis with microgliosis in the brain, cerebellum as well as inflammation changes affecting the grey matter of the spinal cord were also described in bovines with astrovirus-associated non-suppurative encephalitis which suggests the presence of general course of NI-AstV neuroinfection.

While some astroviruses are known to cause outbreaks of gastroenteritis astrovirus-associated encephalitis cases have been reported only sporadically among humans, bovine and ovine. The constant presence with recurrent increases of neurological disease cases in swine farms indicates that natural neuroinvasive astrovirus infections may cause common, severe, persistent epidemics among domestic pigs and constitute an economically important agent threatening livestock and - considering the possible zoonotic and recombinant potential of astroviruses - even humans.

### Example 3: Virus inactivation using chemical agent

The synthesis of the inactivating agent ethylenimine (further referred as EI) is performed in 0.8 % final concentration by the reaction of 0.2M 2-bromethylamin-hydrobromide and 0.4M sodium hydroxide at 37°C for one hour.
Inactivation of the NI-poAst-3 virus suspension is done with the concentration of 0.05% (w/v) and 0.075% (w/v) at the temperature of 23°C and 37°C for 24 hours and 48 hours. The pH value of the EI stock solution is adjusted to 8.0-8.5 before being used for inactivation.
Inactivation is stopped by adding 50% (w/v) sodium-thiosulphate solution to the virus suspension in 1.5M excess then on the following day the pH is adjusted by adding glycin-HCl buffer to 7.2-7.4.

As prepared herein, the NI-PoAstV-3 virus can be fully inactivated according to the appropriate inactivation control method irrespectively of the concentration, temperature and time applied during the inactivation.

### Example 4: Preparation of a composition with adjuvant (10% oil)

A NI-PoAstV-3 virus vaccine is prepared as follow:
First Step: A high shear rotor stator emulsifier is used to produce the formulations. To produce an emulsion, one volume of oily phase is emulsified at 25°C with one volume of aqueous phase #1. The aqueous phase is added to the oily phase under agitation, 5000 rpm (rotation per minute) for 1 minute. The rotation speed is progressively increased with the augmentation of the volume to 10000 rpm during 1 minute. During this step the emulsion is a water-in-oil emulsion.
   For the emulsion, phase composition is as follows:
   Oily phase (200 ml):
      - Sorbitan monooleate: 1.8% v/v,
      - Sorbitan trioleate (20 OE): 10.2% v/v,
      - Light liquid paraffin oil: 88% v/v,
   Aqueous phase #1 (200 ml):
      - 20% (w/v) solution of sorbitan monooleate (20 OE): 11.25% v/v
      - Phosphate disodic and monopotassic 0.02M isotonic buffer (pH 7.2-7.6): 88.75% v/v Sorbitan monooleate and sorbitan trioleate (20 OE) are introduced in the oily phase.
         A 20% (w/v) solution of sorbitan monooleate (20 OE) is prepared in the same buffer as the vaccine, for example, in phosphate disodic and monopotassic 0.02M isotonic buffer. When agitation is stopped, the emulsion changes to an oil-in-water emulsion. The emulsion is then placed in a cold chamber at 5°C for at least 4 hours. At this stage, the emulsion is a pre-emulsion containing 50% of oily phase.
Second Step: The aqueous phase #2 is prepared with 200 ml of phosphate disodic and monopotassic 0.02M isotonic buffer pH 7.2-7.6 with NI-PoAstV-3. The pre-emulsion as prepared in the first step is cooled to about 5°C, diluted by adding half the volume of the aqueous phase #2 at the same temperature, and mixed by the rotation of a magnetic bar for 1 minute.

### Example 5: Immunization of swine

Piglets are vaccinated with the NI-PoAstV-3 vaccine with the adjuvant as prepared in Example 5 (Group A) on day 0 (7-week-old pigs). A first control group receives the adjuvant as described in Example 5, only (Group B). A second control group receives PBS only (Group C). A 2 ml volume is injected by intramuscular route. A second injection of vaccine or PBS is performed on day 21 (10-week-old pigs). On day 42, three weeks after the second vaccination, 7 pigs from each group are challenged by intranasal administration of 2 ml NI-PoAstV-3 virus suspension containing 10³ TCID₅₀ of NI-PoAstV-3 virus/ml. After challenge, piglets are weighed on days 55 and 72. Rectal temperatures are recorded. Blood samples are taken 7 days before the first vaccination (day -7), at the time of the second vaccination (day 21), at challenge (day 42) and on days 55, 64 and 72 for immunofluorescence serology. Sera are tested individually, diluted twofold, starting with 1:2 in PBS supplemented with 0.5% bovine serum albumin.

### LIST OF SEQUENCES

### KY073229 [NI-Brain/9-2016a/HUN]

SEQ ID NO: 12: KY073229 ORFlab (amino acid) 
MALPFDDTLKFGSATARMKGRRLDMVARTKLKDLLGDGPYYYDFGPLEVVDSSSKQSTVKMTSVQTVYVS
SVVEDNSYVTYKFVPGVNDWVETEPVLHKPTALVGVLWREYKRYKDRTETLTQELSQLRLEHSLLRHDYE
RVRPQQPVAPKGRLGFITKLLIGLLIGSLLAHSTLAHKLPGTGLMGECLDTDVIDGKQVCVNFIPRNDTE
ADSPDKPATTVWDPEGLLELLILVAPYLVSWPMIATMVGFFYVATAEQPAYMLVSLLLATYSKAQVLALA
GLPFMDMPSVVTLWVSMLIHSYDAVLSLWVALLLAAFILSAGVFMPDVRYSELVRGQVVVFLVLVLNYLV
VMMSLPNWLVFSLVVGYRILRVLTFLVAEKVEVRGPDGKVVETRTSMPAWINKASNFFQTRFHQKIRTGI
SPTARVIPNGVIVIETQDGSGTGFRCRNYLVTAGHVVTGTETPKIQWAGVTAYSKVVHRVSDKDIAFLAI
PQELQDLPSYRLAKKVEDGPVVITSLEDSGALSVAVTEGVIVTDSITYAVQTRNGMSGSPVTNLDGRILA
VHQTNTGFTGGAVILSDEDFPQPKKSAREQQLEARIKELEAAMNQSSGHEDIVELVRAAVAREFKVLRTE
LSATTFTQAKGKNKKNHRRRGGKKRRAVWSEEEYKELLEKGFSKSQLREMAEVLRSHEEDPFGSDTESEG
GFPEWSDISDTESIEREWFGQSWEDCKPVKEEPQDTLPVHLKDKYTLDAYVISKEELKSFAKEFKDYVDK
VEALIDKTVQQGKWLPSVNPTAIIEELNDLWFGLNMLMWEKGLVPFTQRKKIKRKVQKKLQGGPQEGPIT
YSMMDLKAWRRSLCEVERRLVPEGYPLIGGVPLDRPISDWDEPIDDLLLMLPAFKENLNYTPAVWGPEAY
AKSFDKFTYREPCPNIAATYPREWSFATKALKREYSFLDGSVLTDITATSKNADSTPAYPKSLYWKTEAD
YLGERGYQDYIKQLDAIYSGERPDVLWYLFLKKEILKVEKVEQADIRQIVCSDPIFARIGCHFEEDQNIR
MKNHTRTRSGQCGWSPFFGGFNDRIKRLCACDPDCYIEFDWTRFDGTIPNEVFMHIKKVRYSFFAKEFRT
KRVKEAYQWYCEQLLYRHVVLPSGEVTLQTRGNPSGQISTTMDNNMVNVFLQAFEYAYLHPDCTDEELEA
NWSRMDTLVYGDDRLSAYIGLPANYVEKVVIMYSNVFGMWVKPEKVIVSPSIIGLTFCGLRITSDGTIFL
PVPAETNKLISALIKPTKKLQDVMTLYGKLLCYRILGHNLPDDNKFKAYILVALEVVARHIRTRCGDEPL
RFTDEMLDRLWRGGPKVGYGW
SEQ ID NO: 9: KY073229 ORF1a (amino acid) 
MALPFDDTLKFGSATARMKGRRLDMVARTKLKDLLGDGPYYYDFGPLEVVDSSSKQSTVKMTSVQTVYVS
SVVEDNSYVTYKFVPGVNDWVETEPVLHKPTALVGVLWREYKRYKDRTETLTQELSQLRLEHSLLRHDYE
RVRPQQPVAPKGRLGFITKLLIGLLIGSLLAHSTLAHKLPGTGLMGECLDTDVIDGKQVCVNFIPRNDTE
ADSPDKPATTVWDPEGLLELLILVAPYLVSWPMIATMVGFFYVATAEQPAYMLVSLLLATYSKAQVLALA
GLPFMDMPSVVTLWVSMLIHSYDAVLSLWVALLLAAFILSAGVFMPDVRYSELVRGQVVVFLVLVLNYLV
VMMSLPNWLVFSLVVGYRILRVLTFLVAEKVEVRGPDGKVVETRTSMPAWINKASNFFQTRFHQKIRTGI
SPTARVIPNGVIVIETQDGSGTGFRCRNYLVTAGHVVTGTETPKIQWAGVTAYSKVVHRVSDKDIAFLAI
PQELQDLPSYRLAKKVEDGPVVITSLEDSGALSVAVTEGVIVTDSITYAVQTRNGMSGSPVTNLDGRILA
VHQTNTGFTGGAVILSDEDFPQPKKSAREQQLEARIKELEAAMNQSSGHEDIVELVRAAVAREFKVLRTE
LSATTFTQAKGKNKKNHRRRGGKKRRAVWSEEEYKELLEKGFSKSQLREMAEVLRSHEEDPFGSDTESEG
GFPEWSDISDTESIEREWFGQSWEDCKPVKEEPQDTLPVHLKDKYTLDAYVISKEELKSFAKEFKDYVDK
VEALIDKTVQQGKWLPSVNPTAIIEELNDLWFGLNMLMWEKGLVPFTQRKKIKRKVQKNSKGAPKRGPLP
TP
SEQ ID NO: 15: KY073229 ORF1b (amino acid) 
MDLKAWRRSLCEVERRLVPEGYPLIGGVPLDRPISDWDEPIDDLLLMLPAFKENLNYTPAVWGPEAYAKS
FDKFTYREPCPNIAATYPREWSFATKALKREYSFLDGSVLTDITATSKNADSTPAYPKSLYWKTEADYLG
ERGYQDYIKQLDAIYSGERPDVLWYLFLKKEILKVEKVEQADIRQIVCSDPIFARIGCHFEEDQNIRMKN
HTRTRSGQCGWSPFFGGFNDRIKRLCACDPDCYIEFDWTRFDGTIPNEVFMHIKKVRYSFFAKEFRTKRV
KEAYQWYCEQLLYRHVVLPSGEVTLQTRGNPSGQISTTMDNNMVNVFLQAFEYAYLHPDCTDEELEANWS
RMDTLVYGDDRLSAYIGLPANYVEKVVIMYSNVFGMWVKPEKVIVSPSIIGLTFCGLRITSDGTIFLPVP
AETNKLISALIKPTKKLQDVMTLYGKLLCYRILGHNLPDDNKFKAYILVALEVVARHIRTRCGDEPLRFT
DEMLDRLWRGGPKVGYGW
SEQ ID NO: 5: KY073229 ORF2 (amino acid) 
MAGDKPNASAKAAPLAKEVAKEVVKEEKRNQARSRKWRKPRRQQNQPQQSKQRAVVTRQVTKKLKQEGLE
GPRPRFSVRVSATIGKIGPNKKQGPELQISTFLRPSLMKEPNDGTNFGPLQAASAQWGLWRLAYLKIRCT
 
PLVGPSAVTGSVYRLSLNLTQSPGNTSWGGLGARKHIDVPVGKQSAWSLHRGDLAGPRQTWWMTDTNEEG
GQSCGPMVEIHGLGKTSSTYKDADWVGDLFIVEVVGRWEFTNYNAKPALGTLDRVTEDVQAGIEVGSDGI
MTMTLPQRSNLAKHMSTAYEKLPLNALTVGETIWQIVDEGAGLISSAVPPPFGWLIKGGWWFVKKAIGRS
NTDETYYVYASLADAQNNKPVEATPFSRIPVQTTIASTQINAPNTGPSSSLVISTPSGLLPEDLPSTATT
FEVVGYVSAMFSVNQTTPLSPVINNVFPVLGQSASDFAFSIMQNSTQGAGKGMGCLIFQSEPVFLRLNLD
TSSSTVDNIFLLDDTPGIGWTVNFNLPASPITNCADVLAYKHEKWGDNLKFSSWLVRFTKVIPESAGNYY
CLVSAPVISAVNQAPVTIVFDKVATPKLSTSSSLSQMRVSISSVQPGTYGVIYTLGSYNVTDATGVATAP
TLTGPVNDRVVPGPFRAVTGITPISLTTQLPTSFPLWANFFRPASRPSMIDTLMEQIQQRFGLKPIEPDY
NSDDESDVEPTAASKFAKHGMYDALRDSDWDHVDADALIHFISKASNRPRGHAE
SEQ ID NO: 1 Genome

ccaaatagagctacccgttggtccggccctatggcgttgccattcgacgacactcttaaatttggctcag
ccacggcccgtatgaagggtcgtcggttggacatggttgcacgcaccaagttaaaagacttacttggtga
tggtccttactactatgactttggaccccctggaagttgtcgactcttcttctaagcagtcaactgttaaa
atgacttccgtgcaaactgtttatgtgtcgtcggttgttgaggacaactcctacgtaacctacaagtttg
tgcctggcgtcaatgattgggttgaaactgaacctgttcttcataaacccacggctttggttggtgtcct
atggcgtgagtataaacgctacaaggatagaactgaaactcttacacaagagctttcccaactcaggctt
gaacattcattattgcgccatgattatgaacgtgtacgtcctcagcaaccagtagctcctaagggccggt
tagggttcatcacaaagttactcattggcttacttattggctctctgttggctcactctactcttgcaca
taagttacctgggactggcttaatgggtgaatgtttagacactgatgttattgatggtaagcaggtgtgt
gtgaattttatccctcgaaatgataccgaggctgactcaccagataaacctgctaccactgtctgggatc
ctgaaggtcttcttgagttgcttattctagtggctccttatttagtgtcttggcccatgattgcaacaat
ggttggctttttctatgttgctacagctgaacagcctgcctatatgctagtatctttactcttagcaaca
tactcaaaagcccaggtcttagcccttgctggccttcccttcatggatatgccatcagttgtcacgctct
gggtttctatgcttatccattcatatgatgctgttctttcattatgggtggctctgctcctggccgcgtt
catcttgtctgctggcgtcttcatgcccgatgtacgctactctgagttggtgcgtggtcaggtggtagtc
ttccttgtccttgtcctgaactaccttgttgtaatgatgtctttgcccaactggcttgtcttctcccttg
tcgttggctacaggatactgcgtgtttaacatttcttgtggctgagaaagttgaagtccgtggtcctga
tggcaaggttgtagagacacgtacttccatgcctgcatggattaacaaggcgtcaaatttctttcagact
aggtttcatcaaaagataaggacaggtatctcgcccacagcacgggtgattccaaatggggtcatcgtta
ttgaaactcaggatggttcaggaacgggttttaggtgtcgaaactaccttgtcactgcaggacatgttgt
gacaggtacggaaacaccaaaaatccagtgggctggtgtcactgcttactcaaaagttgtccatcgtgta
tcagataaagatattgcttttcttgccattcctcaagaacttcaagatttaccttcttatcgcttggcta
agaaggttgaggatggtcccgtggttattacatccctagaggattcaggtgctctgtcagttgctgtcac
cgaaggtgtcatagtgactgattctataacttatgccgtccagacacggaatggcatgagtggttcacct
gttactaaccttgatggtaggattcttgcggtgcatcaaaccaacactggtttcactgggggcgctgtga
tactatctgatgaagactttccacagcctaagaaatcagcccgtgaacaacaacttgaggcacgtattaa
ggaacttgaggccgcaatgaatcagtcttctggccatgaggacattgtggagcttgtgagagccgcagtg
gcccgtgaattcaaagtgctgcgcactgaactttctgccaccacttttactcaggcaaaggtaagaata
agaaaaatcatcgtcggcgtggtggtaagaaacgccgcgctgtctggtcagaagaagaatataaggaact
cctagagaaaggcttttccaatcacaactacgtgagatggctgaggttcttagatctcatgaggaagac
ccgtttggctctgacactgaatcggaagggggtttccccgagtggtccgatattagtgatactgagtcaa
ttgaacgggagtggtttggccagtcctgggaagactgtaagcctgttaaggaagagccccaggacacact
tcctgtgcaccttaaagataaatacaccttggatgcctatgtcattagtaaggaagagttgaaatctttt
gccaaggagttcaaagactatgttgacaaggttgaagcattaattgataaaactgtccaacaggtaagt
ggttgccatcagttaatcccacagccataatcgaggagcttaatgacttatggtttggcctaaatatgtt
gatgtgggagaaaggcttggttcctttacacaaaggaagaaaatcaagcgtaaggttcaaaaaaactcc
Aagggggcccccaagagggggcccattacctactccatgatggacctcaaagcatggcggcgctccctgtg
tgaggttgagcgccgcttagtaccagagggttacccgcttattggtggggtgcctctggacaggcctatc
tctgattgggatgaacctattgatgacttactcctaatgttaccagcatttaaagagaaccttaactaca
ctcctgctgtctgggggcctgaggcctatgctaaatcatttgataagttcacttatagagagccttgccc
taacatagccgccacgtatccacgtgagtggtcttttgcaacaaagctttgaagagggagtactctttc
cttgatggctctgtcctgaccgatattactgccacatccaaaaatgctgactccacccctgcttatccta
agtctctttactggaaaactgaagcagactatcttggtgagcggggctatcaggattacattaagcaatt
ggacgccatctactctggggaacgcccagatgttttatggtacctcttcttgaagaaggaaatcctgaaa
gtggaaaaagtcgagcaggccgacattagacaaattgtttgttctgaccctatatttgcacgtataggtt
gtcactttgaggaggatcaaaacatcaggatgaagaaccatactaggacacggtctggtcagtgtggctg
gtcgcctttctttggtggcttcaatgacaggatcaaacgcttatgcgcttgtgaccctgattgttacata
gaatttgattggacacgctttgatggtactatacctaatgaagtgtttatgcacatcaagaaagtccgct

actctttcttcgcgaaggagtttaggacgaagcgtgttaaagaagcctaccagtggtactgcgaacagct
cctgtacaggcacgtcgtgctcccttcgggtgaggtaacattgcagacccgtggaaacccctctggacaa
atctccaccactatggacaacaacatggtgaatgtttttcttcaagcatttgagtacgcctacttacacc
cagattgtacagatgaggaactcgaggcaattggtctcgtatggacaccctggtttatggtgatgatag
attatccgcatatattggcttgcccgccaattatgtggagaaggttgtaataatgtactcaaatgttttt
ggtatgtgggtgaaaccagaaaaagttatcgtttcccccagcattataggtctcaccttctgtggtctca
gaattaccagcgacggtaccatcttttacctgttccagcagagactaacaagcttatttctgcgctcat
taagccaaccaagaaacttcaagatgtgatgaccctctatggaaactcctttgctaccgcatccttggc
cacaacctccctgatgacaacaagtttaaggcctacattctggtggccttggaggttgttgctaggcaca
tcagaacacgttgtggtgacgagccactccgctttactgatgagatgcttgaccggctttggagggagg
accaaaagtcggatatggctggtgataagcccaatgcgtctgccaaagcggcacctcttgctaaggaggt
tgccaaggaggttgttaaggaggagaagagaaatcaggcacgtagtcggaagtggcgtaagcctcgccga
cagcaaaatcaacctcaacaatcaaaacaacgagccgttgtgactaggcaagttacaaagaaattaaagc
aagagggcttggaaggaccacgaccccgcttttctgtacgagtgtcagctaccattggaaagattggcc
caataaaaaacaggggcccgaacttcaaatttcaactttcttacgcccatctcttatgaaggaaccaaat
gatggcaccaatttgggcctttgcaagcagcctcagcacagtggggtctgtggcgtcttgcatacttaa
agatacgctgtacacccttagttgggccatcggcggttacaggctcagtctataggctttccctaaatct
cactcagtcgcccggcaatacctcttggggtggcctaggtgcacgcaaacatattgatgtaccagtggga
aagcaatcagcttggagccttcacaggggcgaccttgctggccctcgccaaacctggtggatgactgaca
ctaatgaagaaggaggccaatcttgtggacctatggttgagattcatggcttgggaaagacttcctccac
ttataaagacgccgactgggttggcgatttgtttatagtggaggtggtaggacgttgggagtttaccaat
tacaatgctaagcccgccctcggcaccttagatcgtgtgaccgaggatgtccaagccggcattgaggttg
gttcagatgggatcatgactatgacccttcctcaacgctccaacctagctaagcacatgtcaacagctta
tgaaaaattaccattaaatgcccttacagtgggtgagaccatttggcagatagttgatgaaggtgctggc
cttatctcatctgcagtacctccgccttttggctggctcattaaaggtggctggtggtttgttaagaagg
ccattggtaggtctaatacagatgagacctattatgtttatgcctctcttgcagatgcgcaaaacaataa
acctgttgaggcgactcccttctcacgtatacctgtccaaaccaccattgcatccacccagattaatgcg
cctaacactgggccctcctcttctctggtcatttccacgccttctggcttgttaccagaggatcttccat
ctactgctaccacctttgaggtcgttggctatgtgtcagctatgttttcagttaatcagaccacaccgct
ttctccagttattaataatgtgttcccagttctgggcagagtgcaagtgattttgcattttccatcatg
caaaattctacacagggggctgggaaaggcatgggttgcctaatctttcagtccgagccggtgttcctta
ggctaaatctagatacatcatcatctacagttgataacatcttcttgcttgatgataccccctggtattgg
ctggactgttaacttcaatcttccagctagtccaatcaccaactgtgcagatgtcttggcgtacaagcat
gaaaaatggggtgataacctcaagtttttcatcttggcttgtccgctttacaaaagtaatccctgagtcag
ctggcaactactattgtcttgtttctgctccagttatttcagctgtgaaccaggctccagttactatagt
ctttgataaagtggcaactccaaagttgtctacttcttcttcattgtcacagatgcgtgtttctatctca
tctgtgcagcctggtacgtatggtgttatctatacccttggcagttataatgtgactgatgccactggag
tagctactgcaccgaccctgacaggccccgttaatgacagggtggtgcctggcccctttcgtgcagtaac
tgggattacacctatctcgctcacaacccagctgccgacatcttttcctttgtgggcaactttttcagg
ccggcttcccggccgagcatgattgacactcttatggagcaaatccaacagaggtttggtctgaagccaa
ttgaacctgactataattcagatgatgagagcgatgtagagcctacagctgcttctaaatttgccaagca
tggcatgtatgatgctctccgtgattctgactggatcacgttgatgccgacgctcttatccatttcatt
tcaaaggcatccaatcgcccccgaggccacgccgagtaggaacgagggtacaggggctcagatctctttt
aaattgatctttctctttcaggc

### KY073230 [NI-SC/9-2016a/HUN]

SEQ ID NO: 4: **KY073230** ORF2 (nucleotide) 
ATGGCTGGTGATAAGCCCAATGCGTCTGCCAAAGCGGCACCTCTTGCTAAGGAGGTTGCCAAGGAGGTTG
TTAAGGAGGAGAAGAGAAATCAGGCACGTAGTCGGAAGTGGCGTAAGCCTCGCCGACAGCAAAATCAACC
TCAACAATCAAAACAACGAGCCGTTGTGACTAGGCAAGTTACAAAGAAATTAAAGCAAGAGGGCTTGGAA
GGACCACGACCCCGCTTTTCTGTACGAGTGTCAGCTACCATTGGAAAGATTGGGCCCAATAAAAAACAGG
GGCCCGAACTTCAAATTTCAACTTTCTTACGCCCATCTCTTATGAAGGAACCAAATGATGGCACCAATTT
TGGGCCTTTGCAAGCAGCCTCAGCACAGTGGGGTCTGTGGCGTCTTGCATACTTAAAGATACGCTGTACA
CCCTTAGTTGGGCCATCGGCGGTTACAGGCTCAGTCTATAGGCTTTCCCTAAATCTCACTCAGTCGCCCG
GCAATACCTCTTGGGGTGGCCTAGGTGCACGCAAACATATTGATGTACCAGTGGGAAAGCAATCAGCTTG
GAGCCTTCACAGGGGCGACCTTGCTGGCCCTCGCCAAACCTGGTGGATGACTGACACTAATGAAGAAGGA
 
GGCCAATCTTGTGGACCTATGGTTGAGATTCATGGCTTGGGAAAGACTTCCTCCACTTATAAAGACGCCG
ACTGGGTTGGCGATTTGTTTATAGTGGAGGTGGTAGGACGTTGGGAGTTTACCAATTACAATGCTAAGCC
CGCCCTCGGCACCTTAGATCGTGTGACCGAGGATGTCCAAGCCGGCATTGAGGTTGGTTCAGATGGGATC
ATGACTATGACCCTTCCTCAACGCTCCAACCTAGCTAAGCACATGTCAACAGCTTATGAAAAATTACCAT
TAAATGCCCTTACAGTGGGTGAGACCATTTGGCAGATAGTTGATGAAGGTGCTGGCCTTATCTCATCTGC
AGTACCTCCGCCTTTTGGCTGGCTCATTAAAGGTGGCTGGTGGTTTGTTAAGAAGGCCATTGGTAGGTCT
AATACAGATGAGACCTATTATGTTTATGCCTCTCTTGCAGATGCGCAAAACAATAAACCTGTTGAGGCGA
CTCCCTTCTCACGTATACCTGTCCAAACCACCATTGCATCCACCCAGATTAATGCGCCTAACACTGGGCC
CTCCTCTTCTCTGGTCATTTCCACGCCTTCTGGCTTGTTACCAGAGGATCTTCCATCTACTGCTACCACC
TTTGAGGTCGTTGGCTATGTGTCAGCTATGTTTTCAGTTAATCAGACCACACCGCTTTCTCCAGTTATTA
ATAATGTGTTCCCAGTTCTGGGGCAGAGTGCAAGTGATTTTGCATTTTCCATCATGCAAAATTCTACACA
GGGGGCTGGGAAAGGCATGGGTTGCCTAATCTTTCAGTCCGAGCCGGTGTTCCTTAGGCTAAATCTAGAT
ACATCATCATCTACAGTTGATAACATCTTCTTGCTTGATGATACCCCTGGTATTGGCTGGACTGTTAACT
TCAATCTTCCAGCTAGTCCAATCACCAACTGTGCAGATGTCTTGGCGTACAAGCATGAAAAATGGGGTGA
TAACCTCAAGTTTTCATCTTGGCTTGTCCGCTTTACAAAAGTAATCCCTGAGTCAGCTGGCAACTACTAT
TGTCTTGTTTCTGCTCCAGTTATTTCAGCTGTGAACCAGGCTCCAGTTACTATAGTCTTTGATAAAGTGG
CAACTCCAAAGTTGTCTACTTCTTCTTCATTGTCACAGATGCGTGTTTCTATCTCATCTGTGCAGCCTGG
TACGTATGGTGTTATCTATACCCTTGGCAGTTATAATGTGACTGATGCCACTGGAGTAGCTACTGCACCG
ACCCTGACAGGCCCCGTTAATGACAGGGTGGTGCCTGGCCCCTTTCGTGCAGTAACTGGGATTACACCTA
TCTCGCTCACAACCCAGCTGCCGACATCTTTTCCTTTGTGGGCAAACTTTTTCAGGCCGGCTTCCCGGCC
GAGCATGATTGACACTCTTATGGAGCAAATCCAACAGAGGTTTGGTCTGAAGCCAATTGAACCTGACTAT
AATTCAGATGATGAGAGCGATGTAGAGCCTACAGCTGCTTCTAAATTTGCCAAGCATGGCATGTATGATG
CTCTCCGTGATTCTGACTGGGATCACGTTGATGCCGACGCTCTTATCCATTTCATTTCAAAGGCATCCAA
TCGCCCCCGAGGCCACGCCGAGTAG
SEQ ID NO: 6: **KY073230** ORF2 (aminoacid)

MAGDKPNASAKAAPLAKEVAKEVVKEEKRNQARSRKWRKPRRQQNQPQQSKQRAVVTRQVTKKLKQEGLE
GPRPRFSVRVSATIGKIGPNKEQGPELQISTFLHPSLMKEPNDGTNFGPLQAASAQWGLWRLADLKIRCT
PLVGPSAVTGSVYRLSLNLTQSPGNTSWGGLGARKHIDVPVGKQSTWSLHRGDLAGPRQTWWMTDTNEEG
GQSCGPMVEIHGLGKTSSTYKDADWVGDLFIVEVVGRWEFTNYNAKPALGTLDRVTEDVQAGIEVGSDGI
MTMTLPQRSNLAKHMSTAYEKLPLNALTVGETIWQIVDEGAGLISSAVPPPFGWLIKGGWWFVKKAIGRS
NTDETYYVYASLADAQNNKPVEATPFSRIPVQTTIASTQINAPNTGPSSSLVISTPSGLLPEDLPSTATT
FEVVGYVSAMFSVNQTTPLSPVINNVFPVLGQSASDFAFSIMQNSTQGAGKGMGCLIFQSEPVFLRLNLD
TSSSTVDNIFLLDDTPGIGWTVNFNLPASPITNCADVLAYKHEKWGDNLKFSSWLVRFTKVIPESAGNYY
CLVSAPVISAVNQAPVTIVFDKVATPKLSTSSSLSQMRVSISSVQPGTYGVIYTLGSYNVTDATGVATAP
TLTGPVNDRVVPGPFRAVTGITPISLTTQLPTSFPLWANFFRPASRPSMIDTLMEQIQQRFGLKPIEPDY
NSDDESDVEPTAASKFAKHGMYDALRDSDWDHVDADALIHFISKASNRPRGHAE

### KY073231 [NI-Brain/173-2016a/HUN]

SEQ ID NO: 13: KY073231 ORFlab (aminoacid) 
MALPFDDTLKFGSATARMKGRRLDMVARTKLKDLLGDGPYYYDFGPLEVVDSSSKQSTVKMTSVQTVYVS
SVVEDNSYVTYKFVPGVNDWVETEPVLHKPTALVGVLWREYKRYKDRTETLTQELSQLKLEHSLLRHDYE
RVRPQQPVAPKGRLGFITKLLIGLLIGSLLAHSTLAHKLPGTGLMGECLDTDVIDGKQVCVNFIPRNDTE
ADSPDKPATTAWDPEGLLELLILVAPYLVSWPMIATMVGFFYVATAEQPAYMLVSLLLATYSKAQVLALA
GLPFMDMPSVVTLWVSMLIHSYDAVLSLWVALLLAAFILSAGVFMPDVRYSELVRGQVVVFLVLVLNYLV
VMMSLPNWLVFSLVVGYRILRVLTFLVAEKVEVRGPDGKVVETRTSMPAWINKASNFFQTRFHQKIRTGI
SPTARVIPNGVIVIETQDGSGTGFRCRNYLVTAGHVVTGTETPKIQWAGVTAYSKVVHRVSDKDIAFLAI
PQELQDLPSYRLAKKVEDGPVVITSLEDSGALSVAVTEGVIVTDSITYAVQTRNGMSGSPVTNLDGRILA
VHQTNTGFTGGAVILSDEDFPQPKKSAREQQLEARIKELEAAMNQSSGHEDIVELVRAAVAREFKVLRTE
LSATTFTQAKGKNKKNHRRRGGKKRRAVWSEEEYKELLEKGFSKSQLREMAEVLRSHEEDPFGSDTESEG
GFPEWSDISDTESIEREWFGQSWEDCKPVKEEPQDTLPVHLKDKYTLDAYVISKEELKSFAKEFKDYVDK
VEALIDKTVQQGKWLPSVNPTAIIEELNDLWFGLNILMWEKGLVPFTQRKKIKRKVQKKLQGGPQEGPIT
YSMMDLKAWRRSLCEVERRLVPEGYPLIGGVPLDRPISDWDEPIDDLLLMLPAFKENLNYTPAVWGPEAY
AKSFDKFTYREPCPNIAATYPREWSFATKALKREYSFLDGSVLTDITATSKNADSTPAYPKSLYWKTEAD
YLGERGYQDYIKQLDAIYSGERPDVLWYLFLKKEILKVEKVEQADIRQIVCSDPIFARIGCHFEEDQNIR
 
MKNHTRTRSGQCGWSPFFGGFNDRIKRLCACDPDCYIEFDWTRFDGTIPNEVFMHIKKVRYSFFAKEFRT
KRVKEAYQWYCEQLLYRHVVLPSGEVTLQTRGNPSGQISTTMDNNMVNVFLQAFEYAYLHPDCTDEELEA
NWSRMDTLVYGDDRLSAYIGLPANYVEKVVIMYSNVFGMWVKPEKVIVSPSIIGLTFCGLRITSDGTIFL
PVPAETNKLISALIKPTKKLQDVMTLYGKLLCYRILGHNLPDDNKFKAYILVALEVVARHIRTRCGDEPL
RFTDEMLDRLWRGGPKVGYGW
SEQ ID NO: 10: KY073231 ORFla (amino acid) 
MALPFDDTLKFGSATARMKGRRLDMVARTKLKDLLGDGPYYYDFGPLEVVDSSSKQSTVKMTSVQTVYVS
SVVEDNSYVTYKFVPGVNDWVETEPVLHKPTALVGVLWREYKRYKDRTETLTQELSQLKLEHSLLRHDYE
RVRPQQPVAPKGRLGFITKLLIGLLIGSLLAHSTLAHKLPGTGLMGECLDTDVIDGKQVCVNFIPRNDTE
ADSPDKPATTAWDPEGLLELLILVAPYLVSWPMIATMVGFFYVATAEQPAYMLVSLLLATYSKAQVLALA
GLPFMDMPSVVTLWVSMLIHSYDAVLSLWVALLLAAFILSAGVFMPDVRYSELVRGQVVVFLVLVLNYLV
VMMSLPNWLVFSLVVGYRILRVLTFLVAEKVEVRGPDGKVVETRTSMPAWINKASNFFQTRFHQKIRTGI
SPTARVIPNGVIVIETQDGSGTGFRCRNYLVTAGHVVTGTETPKIQWAGVTAYSKVVHRVSDKDIAFLAI
PQELQDLPSYRLAKKVEDGPVVITSLEDSGALSVAVTEGVIVTDSITYAVQTRNGMSGSPVTNLDGRILA
VHQTNTGFTGGAVILSDEDFPQPKKSAREQQLEARIKELEAAMNQSSGHEDIVELVRAAVAREFKVLRTE
LSATTFTQAKGKNKKNHRRRGGKKRRAVWSEEEYKELLEKGFSKSQLREMAEVLRSHEEDPFGSDTESEG
GFPEWSDISDTESIEREWFGQSWEDCKPVKEEPQDTLPVHLKDKYTLDAYVISKEELKSFAKEFKDYVDK
VEALIDKTVQQGKWLPSVNPTAIIEELNDLWFGLNILMWEKGLVPFTQRKKIKRKVQKNSKGAPKRGPLP
TP
SEQ ID NO: 16: KY073231 ORF1b (amino acid) 
MDLKAWRRSLCEVERRLVPEGYPLIGGVPLDRPISDWDEPIDDLLLMLPAFKENLNYTPAVWGPEAYAKS
FDKFTYREPCPNIAATYPREWSFATKALKREYSFLDGSVLTDITATSKNADSTPAYPKSLYWKTEADYLG
ERGYQDYIKQLDAIYSGERPDVLWYLFLKKEILKVEKVEQADIRQIVCSDPIFARIGCHFEEDQNIRMKN
HTRTRSGQCGWSPFFGGFNDRIKRLCACDPDCYIEFDWTRFDGTIPNEVFMHIKKVRYSFFAKEFRTKRV
KEAYQWYCEQLLYRHVVLPSGEVTLQTRGNPSGQISTTMDNNMVNVFLQAFEYAYLHPDCTDEELEANWS
RMDTLVYGDDRLSAYIGLPANYVEKVVIMYSNVFGMWVKPEKVIVSPSIIGLTFCGLRITSDGTIFLPVP
AETNKLISALIKPTKKLQDVMTLYGKLLCYRILGHNLPDDNKFKAYILVALEVVARHIRTRCGDEPLRFT
DEMLDRLWRGGPKVGYGW
SEQ ID NO: 7: KY073231 ORF2 (amino acid) 
MAGDKPNASAKAAPLAKEVAKEVVKEEKRNQARSRKWRKPRRQQNQPQQSKQRAVVTRQVTKKLKQEGLE
GPRPRFSVRVSATIGKIGPNKEQGPELQISTFLHPSLMKEPNDGTNFGPLQAASAQWGLWRLADLKICCT
PLVGPSAVTGSVYRLSLNLTQSPGNTSWGGLGARKHIDVPVGKQSTWSLHRGDLAGPRQTWWMTDTNEEG
GQSCGPMVEIHGLGKTSSTYKDADWVGDLFIVEVVGRWEFTNYNAKPALGTLDRVTEDVQAGIEVGSDGI
MTMTLPQRSNLAKHMSTAYEKLPLNALTVGETIWQIVDEGAGLISSAVPPPFGWLIKGGWWFVKKAIGRS
NTDETYYVYASLADAQNNKPVEATPFSRIPVQTTIASTQINAPNTGPSSSLVISTPSGLLPEDLPSTATT
FEVVGYVSAMFSVNQTTPLSPVINNVFPVLGQSASDFAFSIMQNSTQGAGKGMGCLIFQSEPVFLRLNLD
TSSSTVDNIFLLDDTPGIGWTVNFNLPASPITNCADVLAYKHEKWGDNLKFSSWLVRFTKVIPESAGNYY
CLVSAPVISAVNQAPVTIVFDKVATPKLSTSSSLSHMRVSISSVQPGTYGVIYTLGSYNVTDATGVATAP
TLTGPVNDRVVPGPFRAVTGITPISLTTQLPTSFPLWANFFRPASRPSMIDTLMEQIQQRFGLKPIEPDY
NSDDESDVEPTAASKFAKHSMYDALRDSDWDHVDADALIHFISKASNRPRGHAE
SEQ ID NO: 2: KY073231 Genome(nucleic acid)

ccaaatagagctacccgttggtccggccctatggcgttgccattcgacgacactcttaaatttggctcag
ccacggcccgtatgaagggtcgtcggttggacatggttgcacgcaccaagttaaaagacttacttggtga
tggtccttactactatgactttggacccctggaagttgtcgactcttcttctaagcagtcaactgttaaa
atgacttccgtgcaaactgtttatgtgtcgtcggttgttgaggacaactcctacgtaacctacaagtttg
tgcctggcgtcaatgattgggttgaaactgaacctgttcttcataaacccacggctttggttggtgtcct
atggcgtgagtataaacgctacaaggatagaactgaaactcttacacaagagctttcccaactcaagctt
gaacattcattattgcgccatgattatgaacgtgtacgtcctcagcaaccagtagctcctaagggccggt
tagggttcatcacaaagttactcattggcttacttattggctctctgttggctcactctactcttgcaca
taagttacctggactggcttaatgggtgaatgtttagacactgatgttattgatggtaagcaggtgtgt
gtgaattttatccctcgaaatgataccgaggctgactcaccagataaacctgctaccactgcctgggatc
ctgaaggtcttcttgagttgcttattctagtggctccttatttagtgtcttggcccatgattgcaacaat
ggttggcttttctatgttgctacagctgaacagcctgcctatatgctagtatctttactcttagcaaca

tactcaaaagcccaggtcttagcccttgctggccttcccttcatggatatgccatcagttgtcacgctct
gggtttctatgcttatccattcatatgatgctgttctttcattatgggtggctctgctcctggccgcatt
catcttgtctgctggcgtcttcatgcccgatgtacgctactctgagttggtgcgtggtcaggtggtagtc
ttccttgtccttgtcctgaactaccttgttgtaatgatgtctttgcccaactggcttgtcttctcccttg
tcgttggctacaggatactgcgtgttttaacatttcttgtggctgagaaagttgaagtccgtggtcctga
tggcaaggttgtagagacacgtacttccatgcctgcatggattaacaaggcgtcaaatttctttcagact
aggtttcatcaaaagataaggacaggtatctcgcccacagcacgggtgattccaaatggggtcatcgtta
ttgaaactcaggatggttcaggaacgggttttaggtgtcgaaactaccttgtcactgcaggacatgttgt
gacaggtacggaaacaccaaaaatccagtgggctggtgtcactgcttactcaaaagttgtccatcgtgta
tcagataaagatattgcttttcttgctattcctcaagaacttcaagatttaccttcttaccgcttggcta
agaaggttgaggatggtcccgtggttattacatccctagaggattcaggtgctctgtcagttgctgtcac
cgaaggtgtcatagtgactgattctataacttatgccgtccagacacggaatggcatgagtggttcacct
gttactaaccttgatggtaggattcttgcagtgcatcaaaccaacactggtttcactgggggcgctgtga
tactatctgatgaagactttccacagcctaagaaatcagcccgtgaacaacaacttgaggcacgtattaa
ggaacttgaggccgcaatgaatcagtcttctggccatgaggacattgtggagcttgtgagagccgcagtg
gcccgtgaattcaaagtgctgcgcactgaactttctgccaccacttttactcaggcaaaggcaagaata
agaaaaatcatcgtcggcgtggtggtaagaaacgccgcgctgtctggtcagaagaagaatataaggaact
cctagagaaaggcttttccaaatcacaactacgtgaaatggctgaggttcttaggtctcatgaggaagac
ccgtttggctctgacactgaatcggaaggggggtttccccgagtggtccgatattagtgatactgagtcaa
ttgaacgggagtggtttggccagtcctgggaagactgtaagcctgttaaggaagagccccaggacacact
tcctgtgcaccttaaagataaatacaccttggatgcctatgtcattagtaaggaagagttgaaatctttt
gccaaggagttcaaagactatgttgacaaggttgaagcactaattgataaaactgtccaacagggtaagt
ggttgccatcagttaatcccacagccataatcgaggagcttaatgacttatggtttggcctaaatatctt
gatgtgggagaaaggcttggttcctttacacaaaggaagaaaatcaagcgtaaggttcaaaaaaactcc
Aaggggggcccccaagagggggcccattacctactccatgatggacctcaaagcatggcggcgctccctgtg
tgaggttgagcgccgcttagtaccagagggttacccgcttattggtggggtgcctctggacaggcctatc
tctgattgggatgaacctattgatgacttactcctaatgttaccagcatttaaagagaatcttaactaca
ctcctgctgtttggggggcctgaggcctatgctaaatcatttgataagttcacttatagagagccttgccc
taacatagccgccacgtatccacgtgagtggtcttttgcaacaaaagctttgaagagggagtactctttc
cttgatggctctgtcctgaccgatattactgccacatccaaaaatgctgactccacccctgcttatccta
agtctctttactggaaaactgaagcagactatcttggtgagcggggctatcaggattacattaagcaatt
ggacgccatctactctggggaacgcccagatgttttatggtacctcttcttgaagaaggaaatcctgaaa
gtggaaaaagtcgagcaggccgacattagacaaattgtttgttctgaccctatatttgcacgtataggtt
gtcactttgaggaggatcaaaacatcaggatgaagaaccatactaggacacggtctggtcagtgtggctg
gtcgcctttctttggtggcttcaatgacaggatcaaacgcttatgcgcttgtgaccctgattgttacata
gaatttgattggacacgctttgatggtactatacctaatgaagtgtttatgcacatcaagaaagtccgtt
actctttcttcgcgaaggagtttaggacgaagcgtgttaaagaagcctaccagtggtactgcgaacagct
cctgtacaggcacgtcgtgctcccttcgggtgaggtaacattgcagacccgtggaaacccctctggacaa
atctccaccactatggacaacaacatggtgaatgtttttcttcaagcatttgagtacgcctacttacacc
cagattgtacagatgaggaactcgaggcaaattggtctcgtatggataccctggtttatggtgatgatag
attatccgcatatattggcttgcccgccaattatgtggagaaggttgtaataatgtactcaaatgttttt
ggtatgtgggtgaaaccagaaaaagttatcgtttcccccagcattataggtctcaccttctgtggtctca
gaattaccagtgacggtaccatctttttacctgttccagcagagactaacaagcttatttctgcgctcat
taagccaaccaagaaacttcaagatgtgatgaccctctatggaaactcctttgctaccgcatccttggc
cacaacctccctgatgacaacaagtttaaggcctacattctggtggccttggaggttgttgctaggcaca
tcagaacacgttgtggtgacgagccactccgctttactgatgagatgcttgaccggctttggagggagg
accaaaagtcggatatggctggtgataagcccaatgcgtctgccaaagcggcacctcttgctaaggaggt
tgccaaggaggttgttaaggaggagaagagaaatcaggcacgtagtcgaaagtggcgtaagcctcgccga
cagcaaaatcaacctcaacaatcaaaacaacgagccgttgtgactaggcaagttacaaagaaattaaagc
aagagggcttggaaggaccacgaccccgcttttctgtgcgagtgtcagctaccattggaaagattgggcc
caataaagaacaggggcctgaacttcaaatttcaactttcttacatccatctcttatgaaggaaccaaat
gatggcaccaattttgggcctttgcaagcagcctcagcacagtggggtctgtggcgtcttgcagacttaa
agatatgctgtacacccttagttgggccatcggcagttacaggctcagtctataggctttccctaaatct
cactcaatcacccggcaacacctcttggggtggcctaggtgcacgcaaacatattgatgtaccagtggga
aagcaatcaacttggagccttcacagggcgaccttgctggccctcgccaaacctggtggatgactgaca
ctaatgaagaaggaggccaatcttgtggacctatggttgagattcatggcttgggaaagacttcctccac
ttataaagacgccgactgggttggcgatttgtttatagtggaggtggtaggacgttgggagtttaccaat
tacaatgctaagcccgccctcggcaccttagatcgtgtgaccgaggatgttcaagccggcattgaggttg
gttcagatgggatcatgactatgacccttcctcaacgctccaacctagctaagcacatgtcaacagctta

tgaaaattaccattaaatgcccttacagtgggtgagaccatttggcagatagttgatgaaggtgctggc
cttatctcatctgcagtacctccgcctttggctggctcattaaaggtggctggtggttcgttaagaagg
ccattggtaggtctaatacagatgagacctattatgtttatgcctctcttgcagatgcgcaaaacaataa
acctgttgaggcgactcccttctcacgtatacctgtccaaaccaccattgcatccacccagattaatgcg
cctaacactgggccctcctcttctctggtcatttccacgccttctggcttgttaccagaggatcttccat
ctactgctaccacctttgaggttgttggctatgtgtcagctatgttttcagtcaatcagaccacaccgct
ttctccagttattaacaatgtgttcccagttctgggcagagtgcaagtgatttgcattttccatcatg
caaaattctacacaggggctgggaaaggcatgggttgcctaatttttcagtccgagccggtgttcctta
ggctaaatctagatacatcatcatctacagttgataacatcttcttgcttgatgataccccctggtattgg
ctggactgttaacttcaatcttccagctagtccaatcaccaactgtgcagatgtcttggcgtacaagcat
gaaaaatgggtgacaacctcaagtttcatcttggcttgtccgctttacaaaagtaatccctgagtcag
ctggcaactactattgccttgtttctgctccagttatttcagctgtgaaccaggctccagttactatagt
ctttgataaagtggcaactccaaagttgtctacttcttcttcattgtcacacatgcgtgtttctatctca
tctgtgcagcctggtacgtatggtgttatctatacccttggcagttataatgtgactgatgccactggag
tagctactgcaccgaccctgacaggccccgttaatgacagggtggtgcctggcccctttcgtgcagtaac
tgggattacacctatctcgctcacaacccagctgccgacatctttcctttgtgggcaaactttttcagg
ccggcttcccggccgagcatgattgacactcttatggagcaaatccaacagaggtttggtctgaagccaa
ttgaacctgactataattcagatgatgagagcgatgtagagcctacagctgcttctaaatttgccaagca
tagcatgtatgatgctctccgtgattctgactgggatcacgttgatgccgacgctcttatccatttcatt
tcaaaggcatccaatcgcccccgaggccacgccgagtaggaacgagggtacaggggctcagatctctttt
aaattgatctttctctttcaggc

### KY073232 [NI-Brain/386-2015/HUN]

SEQ ID NO: 14: KY073232 ORFlab (amino acid) 
MALPFDDTLKFGSATARMKGRRLDMVARTKLKDLLGDGPYYYDFGPLEVVDSSSKQSTVKMTSVQTVYVS
SVVEDNSYVTYKFVPGVNDWVETEPVLHKPTALVGVLWREYKRYKDRTETLTQELSQLKLEHSLLRHDYE
RVRPQQPIAPKGRLGFITKLLIGLLIGSLLAHSTLAHKLPGTGLMGECLDTDVIDGKQVCVNFIPRNDTE
ADSQDKPATTVWDPEGLLELLILVAPYLVSWPMIATMVGFFYVATAEQPAYMLVSLLLATYSKAQVLALA
GLPFMDMPSVVTLWVSMLIHSYDAVLSLWVALLLAAFILSAGVFMPDVRYSELVRGQVVVFLVLVLNYLV
VMMSLPNWLVFSLVVGYRILRVLTFLVAEKVEVRGPDGKVVETRTSMPAWINKASNFFQTRFHQKIRTGI
SPTARVIPNGVIVIETQDGSGTGFRCRNYLVTAGHVVTGTETPKIQWAGVTAYSKVVHRVSDKDIAFLAI
PQELQDLPSYRLAKKVEDGPVVITSLEDSGALSVAVTEGVIVTDSITYAVQTRNGMSGSPVTNLDGRILA
VHQTNTGFTGGAVILSDEDFPQPKKSAREQQLEARIKELEAAMNQSSGHEDIVELVRAAVAREFKVLRTE
LSATTFTQAKGKNKKNHRRRGGKKRRAVWSEEEYKELLEKGFSKSQLREMAEVLRSHEEDPFGSDTDSEG
GFPEWSDISDTESIEREWFGQSWEDCKPVKEEPQDTLPVHLKDKYTLDAYVISKEELKSFAKEFKDYVDK
VEALIDKTVQQGKWLPSVNPTAIIEELNDLWFGLNMLMWEKGLVPFTQRKKIKRKVQKKLQGGPQEGPIT
YSMMDLKAWRRSLCEVERRLVPEGYPLIGGVPLDRPISDWDEPIDDLLLMLPAFKENLNYTPAVWGPEAY
AKSFDKFTYREPCPNIAATYPREWSFATKALKREYSFLDGSVLTDITATSKNADSTPAYPKSLYWKTEAD
YLGERGYQDYIKQLDAIYSGERPDVLWYLFLKKEILKVEKVEQADIRQIVCSDPIFARIGCHFEEDQNIR
MKNHTRTRSGQCGWSPFFGGFNDRIKRLCACDPDCYIEFDWTRFDGTIPNEVFMHIKKVRYSFFAKEFRT
KRVKEAYQWYCEQLLYRHVVLPSGEVTLQTRGNPSGQISTTMDNNMVNVFLQAFEYAYLHPDCTDEELEA
NWSRMDTLVYGDDRLSAYIGLPANYVEKVVIMYSNVFGMWVKPEKVIVSPSIIGLTFCGLRITSDGTIFL
PVPAETNKLISALIKPTKKLQDVMTLYGKLLCYRILGHNLPDDNKFKAYILVALEVVARHIRTRCGDEPL
RFTDEMLDRLWRGGPKVGYGW
SEQ ID NO: 11: KY073232 ORFla (aminoacid) 
MALPFDDTLKFGSATARMKGRRLDMVARTKLKDLLGDGPYYYDFGPLEVVDSSSKQSTVKMTSVQTVYVS
SVVEDNSYVTYKFVPGVNDWVETEPVLHKPTALVGVLWREYKRYKDRTETLTQELSQLKLEHSLLRHDYE
RVRPQQPIAPKGRLGFITKLLIGLLIGSLLAHSTLAHKLPGTGLMGECLDTDVIDGKQVCVNFIPRNDTE
ADSQDKPATTVWDPEGLLELLILVAPYLVSWPMIATMVGFFYVATAEQPAYMLVSLLLATYSKAQVLALA
GLPFMDMPSVVTLWVSMLIHSYDAVLSLWVALLLAAFILSAGVFMPDVRYSELVRGQVVVFLVLVLNYLV
VMMSLPNWLVFSLVVGYRILRVLTFLVAEKVEVRGPDGKVVETRTSMPAWINKASNFFQTRFHQKIRTGI
SPTARVIPNGVIVIETQDGSGTGFRCRNYLVTAGHVVTGTETPKIQWAGVTAYSKVVHRVSDKDIAFLAI
PQELQDLPSYRLAKKVEDGPVVITSLEDSGALSVAVTEGVIVTDSITYAVQTRNGMSGSPVTNLDGRILA
VHQTNTGFTGGAVILSDEDFPQPKKSAREQQLEARIKELEAAMNQSSGHEDIVELVRAAVAREFKVLRTE
LSATTFTQAKGKNKKNHRRRGGKKRRAVWSEEEYKELLEKGFSKSQLREMAEVLRSHEEDPFGSDTDSEG
GFPEWSDISDTESIEREWFGQSWEDCKPVKEEPQDTLPVHLKDKYTLDAYVISKEELKSFAKEFKDYVDK
 
VEALIDKTVQQGKWLPSVNPTAIIEELNDLWFGLNMLMWEKGLVPFTQRKKIKRKVQKNSKGAPKRGPLP
TP
SEQ ID NO: 17: KY073232 ORF1b (aminoacid) 
MDLKAWRRSLCEVERRLVPEGYPLIGGVPLDRPISDWDEPIDDLLLMLPAFKENLNYTPAVWGPEAYAKS
FDKFTYREPCPNIAATYPREWSFATKALKREYSFLDGSVLTDITATSKNADSTPAYPKSLYWKTEADYLG
ERGYQDYIKQLDAIYSGERPDVLWYLFLKKEILKVEKVEQADIRQIVCSDPIFARIGCHFEEDQNIRMKN
HTRTRSGQCGWSPFFGGFNDRIKRLCACDPDCYIEFDWTRFDGTIPNEVFMHIKKVRYSFFAKEFRTKRV
KEAYQWYCEQLLYRHVVLPSGEVTLQTRGNPSGQISTTMDNNMVNVFLQAFEYAYLHPDCTDEELEANWS
RMDTLVYGDDRLSAYIGLPANYVEKVVIMYSNVFGMWVKPEKVIVSPSIIGLTFCGLRITSDGTIFLPVP
AETNKLISALIKPTKKLQDVMTLYGKLLCYRILGHNLPDDNKFKAYILVALEVVARHIRTRCGDEPLRFT
DEMLDRLWRGGPKVGYGW
SEQ ID NO: 8: KY073232 ORF2 (amino acid) 
MAGDKPNASAKAAPLAKEVAKEVVKEEKRNQARSRKWRKPRRQQNQPQQSKQRAVVTRQVTKKLKQEGLE
GPRPRFSVRVSATIGKIGPNKEQGPELQISTFLHPSLMKEPNDGTNFGPLQAASAQWGLWRLADLKIRCT
PLVGPSAVTGSVYRLSLNLTQSPGNTSWGGLGARKHIDVPVGKQSTWSLHRGDLAGPRQTWWMTDTNEEG
GQSCGPMVEIHGLGKTSSTYKDADWVGDLFIVEVVGRWEFTNYNAKPALGTLDRVTEDVQAGIEVGSDGI
MTMTLPQRSNLAKHMSTAYEKLPLNALTVGETIWQIVDEGAGLISSAVPPPFGWLIKGGWWFVKKAIGRS
NTDETYYVYASLADAQNNKPVEATPFSRIPVQTTIASTQINAPNTGPSSSLVISTPSGLLPEDLPSTATT
FEVVGYVSAMFSVNQTTPLSPVINNVFPVLGQSASDFAFSIMQNSTQGAGKGMGCLIFQSEPVFLRLNLD
TSSSTVDNIFLLDDTPGIGWTVNFNLPASPITNCADVLAYKHEKWGDNLKFSSWLVRLTKVIPESAGNYY
CLVPAPVISPVYQAPVTIVFDKVATPKLSTSSSLSQMRVSISSVQPGTYGVIYTLGSYNVTDATGVATAP
TLTGPVNDRVVPGPFRAVTGITPISLTTQLPTSFPLWANFFRPASRPSMIDTLMEQIQQRFGLKPIEPDY
NSDDESDVEPTAASKFAKHSMYDALRDSDWDHVDADALIHFISKASNRPRGHAE
SEQ ID NO: 3: KY073232 Genome (nucleic acid)

CCAAATAGAGCTACCCGTTGGTCCGGCCCTATGGCGTTGCCATTCGACGACACTCTTAAATTTGGCTCAG
CCACGGCCCGTATGAAGGGTCGTCGGTTGGACATGGTTGCACGCACCAAGTTAAAAGACTTACTTGGTGA
TGGTCCTTACTACTATGACTTTGGACCCCTGGAAGTTGTCGACTCTTCTTCTAAGCAGTCAACTGTTAAA
ATGACTTCCGTGCAAACTGTTTATGTGTCGTCGGTTGTTGAGGACAACTCCTACGTAACCTACAAGTTTG
TGCCTGGCGTCAATGATTGGGTTGAAACTGAACCTGTTCTTCATAAACCCACGGCTTTGGTTGGTGTCCT
ATGGCGTGAGTATAAACGCTACAAGGATAGAACTGAAACTCTTACACAAGAGCTTTCCCAACTCAAGCTT
GAACATTCATTATTGCGCCATGATTATGAACGTGTACGTCCTCAGCAACCAATAGCTCCTAAGGGCCGGT
TAGGGTTCATCACAAAGTTACTCATTGGCTTACTTATTGGCTCTCTGTTGGCTCACTCTACTCTTGCACA
TAAGTTACCTGGGACTGGCTTAATGGGTGAATGTTTAGACACTGATGTTATTGATGGTAAGCAGGTGTGT
GTGAATTTTATCCCTCGAAATGATACCGAGGCTGACTCACAAGATAAACCTGCTACCACTGTCTGGGATC
CTGAAGGTCTTCTTGAGTTGCTTATTCTAGTGGCTCCTTATTTAGTGTCTTGGCCCATGATTGCAACAAT
GGTTGGCTTTTTCTATGTTGCTACAGCTGAACAGCCTGCCTATATGCTAGTATCTTTACTCTTAGCAACA
TACTCAAAAGCCCAGGTCTTAGCCCTTGCTGGCCTTCCCTTCATGGATATGCCATCAGTTGTCACGCTCT
GGGTTTCTATGCTTATCCATTCATATGATGCTGTTCTTTCATTATGGGTGGCTCTGCTCCTGGCCGCGTT
CATCTTGTCTGCTGGCGTCTTCATGCCCGATGTACGCTACTCTGAGTTGGTGCGTGGTCAGGTGGTAGTC
TTCCTTGTCCTTGTCCTGAACTACCTTGTTGTAATGATGTCTTTGCCCAACTGGCTTGTCTTCTCCCTTG
TCGTTGGCTACAGGATACTGCGTGTTTTAACATTTCTTGTGGCTGAGAAAGTTGAAGTCCGTGGTCCTGA
TGGCAAGGTTGTAGAGACACGTACTTCCATGCCTGCATGGATTAACAAGGCGTCAAATTTCTTTCAGACT
AGGTTTCATCAAAAGATAAGGACAGGTATCTCGCCCACAGCACGGGTGATTCCAAATGGGGTCATCGTTA
TTGAAACTCAGGATGGTTCAGGAACGGGTTTTAGGTGTCGAAACTACCTTGTCACTGCAGGACATGTTGT
GACAGGTACGGAAACACCAAAAATCCAGTGGGCTGGTGTCACTGCTTACTCAAAAGTTGTCCATCGTGTA
TCAGATAAAGATATTGCTTTTCTTGCCATTCCTCAAGAACTTCAAGATTTACCTTCTTACCGCTTGGCTA
AGAAGGTTGAGGATGGTCCCGTGGTTATTACATCCCTAGAGGATTCAGGTGCTCTGTCAGTTGCTGTCAC
CGAAGGTGTCATAGTGACTGATTCTATAACTTATGCCGTCCAGACACGGAATGGCATGAGTGGTTCACCT
GTTACTAACCTTGATGGTAGGATTCTTGCAGTGCATCAAACCAACACTGGTTTCACTGGGGGCGCTGTGA
TACTATCTGATGAAGACTTTCCACAGCCTAAGAAATCAGCCCGTGAACAACAACTTGAGGCACGTATTAA
GGAACTTGAGGCCGCAATGAATCAGTCTTCTGGCCATGAGGACATTGTGGAGCTTGTGAGAGCCGCAGTG
GCCCGTGAATTCAAAGTGCTGCGCACTGAACTTTCTGCCACCACTTTTACTCAGGCAAAAGGCAAGAATA
AGAAAAATCATCGTCGGCGTGGTGGTAAGAAACGCCGCGCTGTCTGGTCAGAAGAAGAATATAAGGAACT
CCTAGAGAAAGGCTTTTCCAAATCACAACTACGTGAAATGGCTGAGGTTCTTAGATCTCATGAGGAAGAC

CCGTTTGGCTCTGACACTGACTCGGAAGGGGGTTTCCCCGAGTGGTCCGATATTAGTGATACTGAGTCAA
TTGAACGGGAGTGGTTTGGCCAGTCCTGGGAAGACTGTAAGCCTGTTAAGGAAGAGCCCCAGGACACACT
TCCTGTGCACCTTAAAGATAAATACACCTTGGATGCCTATGTCATTAGTAAGGAAGAGTTGAAATCTTTT
GCCAAGGAGTTCAAAGACTATGTTGACAAGGTTGAAGCATTAATTGATAAAACTGTCCAACAGGGTAAGT
GGTTGCCATCAGTTAATCCCACAGCCATAATCGAGGAGCTTAATGACTTATGGTTTGGCCTAAATATGTT
GATGTGGGAGAAAGGCtTGGTTCCTTTTACACAAAGGAAGAAAATCAAGCGTAAGGTTCAAAAAAACTCC
AAGGGGGCCCCCAAGAGGGGCCCATTACCTACTCCATGATGGACCTCAAAGCATGGCGGCGCTCCCTGTG
TGAGGTTGAGCGCCGCTTAGTACCAGAGGGTTACCCGCTTATTGGTGGGGTGCCTCTGGACAGGCCTATC
TCTGATTGGGATGAACCTATTGATGACTTACTCCTAATGTTACCAGCATTTAAAGAGAATCTTAACTACA
CTCCTGCTGTTTGGGGGCCTGAGGCCTATGCTAAATCATTTGATAAGTTCACTTATAGAGAGCCTTGCCC
TAACATAGCCGCCACGTATCCACGTGAGTGGTCTTTTGCAACAAAAGCTTTGAAGAGGGAGTACTCTTTC
CTTGATGGCTCTGTCCTGACCGATATTACTGCCACATCCAAAAATGCTGACTCCACCCCTGCTTATCCTA
AGTCTCTTTACTGGAAAACTGAAGCAGACTATCTTGGTGAGCGGGGCTATCAGGATTACATTAAGCAATT
GGACGCCATCTACTCTGGGGAACGCCCAGATGTTTATGGTACCTCTTCTTGAAGAAGGAAATCCTGAAA
GTGGAAAAAGTCGAGCAGGCCGACATTAGACAAATTGTTTGTTCTGACCCTATATTTGCACGTATAGGTT
GTCACTTTGAGGAGGATCAAAACATCAGGATGAAGAACCATACTAGGACACGGTCTGGTCAGTGTGGCTG
GTCGCCTTTCTTTGGTGGCTTCAATGACAGGATCAAACGCTTATGCGCTTGTGACCCTGATTGTTACATA
GAATTTGATTGGACACGCTTTGATGGTACTATACCTAATGAAGTGTTTATGCACATCAAGAAAGTCCGCT
ACTCTTTCTTCGCGAAGGAGTTTAGGACGAAGCGTGTTAAAGAAGCCTACCAGTGGTACTGCGAACAGCT
CCTGTACAGGCACGTCGTGCTCCCTTCGGGTGAGGTAACATTGCAGACCCGTGGAAACCCCTCTGGACAA
ATCTCCACCACTATGGACAACAACATGGTAAATGTTTTTCTTCAAGCATTTGAGTACGCCTACTTACACC
CAGATTGTACAGATGAGGAACTCGAGGCAAATTGGTCTCGTATGGATACCCTGGTTTATGGTGATGATAG
ATTATCCGCATATATTGGCTTGCCCGCCAATTATGTGGAGAAGGTTGTAATAATGTACTCAAATGTTTTT
GGTATGTGGGTGAAACCAGAAAAAGTTATCGTTTCCCCCAGCATTATAGGTCTCACCTTCTGTGGTCTCA
GAATTACCAGCGACGGTACCATCTTTTTACCTGTTCCAGCAGAGACTAACAAGCTTATTTCTGCGCTCAT
TAAGCCAACCAAGAAACTTCAAGATGTGATGACCCTCTATGGAAACTCCTTTGCTACCGCATCCTTGGC
CACAACCTCCCTGATGACAACAAGTTTAAGGCCTATATTCTGGTGGCCTTGGAGGTTGTTGCTAGGCACA
TCAGAACACGTTGTGGTGACGAGCCACTCCGCTTTACTGATGAGATGCTTGACCGGCTTTGGAGGGGAGG
ACCAAAAGTCGGATATGGCTGGTGATAAGCCCAATGCGTCTGCCAAAGCGGCACCTCTTGCTAAGGAGGT
TGCCAAGGAGGTTGTTAAGGAGGAGAAGAGAAATCAGGCACGTAGTCGGAAGTGGCGTAAGCCTCGCCGA
CAGCAAAATCAACCTCAACAATCAAAACAACGAGCCGTTGTGACTAGGCAAGTTACAAAGAAATTAAAGC
AAGAGGGCTTGGAAGGACCACGACCCCGCTTTTCTGTACGAGTGTCAGCTACCATTGGAAAGATTGGGCC
CAATAAAGAACAGGGGCCCGAACTTCAAATTTCAACTTTCTTACACCCATCTCTTATGAAGGAACCAAAT
GATGGCACCAATTTTGGGCCTTTGCAAGCAGCCTCAGCACAGTGGGGTCTGTGGCGTCTTGCAGACTTAA
AGATACGCTGTACACCCTTAGTTGGGCCATCGGCAGTTACAGGCTCAGTCTATAGGCTTTCCCTAAATCT
CACTCAATCACCCGGCAATACCTCTTGGGGTGGCCTAGGTGCACGCAAACATATTGATGTACCAGTGGGA
AAGCAATCAACTTGGAGCCTTCACAGGGGCGACCTTGCTGGCCCTCGCCAAACCTGGTGGATGACTGACA
CTAATGAAGAAGGAGGCCAATCTTGTGGACCTATGGTTGAGATTCATGGCTTGGGAAAGACTTCCTCCAC
TTATAAAGACGCCGACTGGGTTGGCGATTTGTTTATAGTGGAGGTGGTAGGACGTTGGGAGTTTACCAAT
TACAATGCTAAGCCCGCCCTCGGCACCTTAGATCGTGTGACCGAGGATGTCCAAGCCGGCATTGAGGTTG
GTTCAGATGGGATCATGACTATGACCCTTCCTCAACGCTCCAACCTAGCTAAGCACATGTCAACAGCTTA
TGAAAAATTACCATTAAATGCCCTTACAGTGGGTGAGACCATTTGGCAGATAGTTGATGAAGGTGCTGGC
CTTATCTCATCTGCAGTACCTCCGCCTTTTGGCTGGCTCATTAAAGGTGGCTGGTGGTTTGTTAAGAAGG
CCATTGGTAGGTCTAATACAGATGAGACCTATTATGTTTATGCCTCTCTTGCAGATGCGCAAAACAATAA
ACCTGTTGAGGCGACTCCCTTCTCACGTATACCTGTCCAAACCACCATTGCATCCACCCAGATTAATGCG
CCTAACACTGGCCCTCCTCTTCTCTGGTCATTTCCACGCCTTCTGGCTTGTTACCAGAGGATCTTCCAT
CTACTGCTACCACCTTTGAGGTCGTTGGCTATGTGTCAGCTATGTTTTCAGTTAATCAGACCACACCGCT
TTCTCCAGTTATTAATAATGTGTTCCCAGTTCTGGGGCAGAGTGCAAGTGATTTTGCATTTTCCATCATG
CAAAATTCTACACAGGGGGCTGGGAAAGGCATGGGTTGCCTAATCTTTCAGTCCGAGCCGGTGTTCCTTA
GGCTAAATCTAGATACATCATCATCTACAGTTGATAACATCTTCTTGCTTGATGATACCCCTGGTATTGG
CTGGACTGTTAACTTCAATCTTCCAGCTAGTCCAATCACCAACTGTGCAGATGTCTTGGCGTACAAGCAT
GAAAAATGGGGTGATAACCTCAAGTTTCATCTTGGCTTGTCCGCTTAACAAAAGTAATCCCTGAGTCAG
CTGGCAACTACTATTGCCTTGTTCCTGCTCCAGTTATTTCACCTGTGTACCAGGCTCCAGTTACTATAGT
CTTTGATAAAGTGGCAACTCCAAAGTTGTCTACTTCTTCTTCATTGTCACAGATGCGTGTTTCTATCTCA
TCTGTGCAGCCTGGTACGTATGGTGTTATCTATACCCTTGGCAGTTATAATGTGACTGATGCCACTGGAG
TAGCTACTGCACCGACCCTGACAGGCCCCGTTAATGACAGGGTGGTGCCTGGCCCCTTTCGTGCAGTAAC
TGGGATTACACCTATCTCGCTCACAACCCAGCTGCCGACATCTTTTCCTTTGTGGCAAACTTTTTCAGG
CCGGCTTCCCGGCCGAGCATGATTGACACTCTTATGGAGCAAATCCAACAGAGGTTTGGTCTGAAGCCAA
TTGAACCTGACTATAATTCAGATGATGAGAGCGATGTAGAGCCTACAGCTGCTTCTAAATTTGCCAAGCA

TAGCATGTATGATGCTCTCCGTGATTCTGACTGGATCACGTTGATGCCGACGCTCTTATCCATTTCATT
TCAAAGGCATCCAATCGCCCCCGAGGCCACGCCGAGTAGGAACGAGGGTACAGGGGCTCAGATCTCTTTC
AAATTGATCTTTCTCTTTTCAGGC

### Primers

AAC CYT CTC CAC ATA ATT GGC (SEQ ID NO: 18)
GAA GTG TTT ATG CAC ATC AAG A (SEQ ID NO: 19)
GGC GTA CTC AAA TGC TTG AA (SEQ ID NO: 20)
GCG AAG GAG TTT AGG ACG AA (SEQ ID NO: 21)
GGC CCA GTR TTA GGB GCA TT (SEQ ID NO: 22)
ACC ATY TGG CAG ATA GTK GA (SEQ ID NO: 23)
CCA GTR TTA GGB GCA TTA ATC TG (SEQ ID NO: 24)
CAT YTG GCA GAT AGT KGA TGA (SEQ ID NO: 25)

**Table 1: Results of the porcine astrovirus 3 RT-nested (n)PCR screening of different tissues collected from 5 affected pigs with encephalomyelitis and posterior paraplegia (PP) from the index farm. + or - signs indicate the results of the first-; (+) or (-) signs indicate second (nested) reactions. The screening RT-(n)PCR primers are designed to the RdRp region of porcine astrovirus 3 (Fig.1). n.a.: No 494 available sample. LN: lymph node.*: Nasal swab samples**

| Animal ID Age Symptoms | GD-1 25 day PP (Stage 1) | GD-2 25 day PP (stage 1) | GD-3 25 day PP (stage 3) | GD-4 25 day PP (stage 3) | GD-5 35 day PP (stage 3) |
|---|---|---|---|---|---|
| Brainstem | + | + | + | + | + |
| Spinal cord (cervical) | n.a. | n.a. | + | + | n.a. |
| Spinal cord (thoracic) | n.a. | n.a. | + | + | n.a. |
| Spinal cord (lumbar) | + | n.a. | + | + | + |
| Nasal mucosa | - (+)* | +* | + | + | n.a. |
| Lung | n.a. | n.a. | + | + | n.a. |
| Tonsils | n.a. | - (-) | + | + | + |
| Salivary glands | n.a. | n.a. | - (+) | + | n.a. |
| Myocardium | n.a. | + | n.a. | n.a. | + |
| Faeces | - (-) | n.a. | - (+) | - (+) | n.a. |
| Ileum | n.a. | n.a. | - (-) | - (+) | - (-) |
| Mesenterial LN | n.a. | - (-) | - (-) | - (-) | n.a. |
| Submandibular LN | n.a. | n.a. | - (+) | + | n.a. |
| Urine | n.a. | n.a. | - (-) | - (-) | n.a. |
| Kidney | n.a. | n.a. | - (-) | - (-) | n.a. |
| Liver | n.a. | n.a. | - (+) | - (+) | n.a. |
| Spleen | n.a. | n.a. | - (-) | - (-) | n.a. |
| Serum | n.a. | n.a. | + | + | n.a. |

**Table 2: List of FFPE samples used for porcine astrovirus 3 (PoAstV-3) detection, histology and in situ hybridization (ISH) analyses. GD: index farm. RdRp: RNA-dependent RNA polymerase, RdRp and capsid RT-nested (n) PCR: + or - signs indicate the results of the first-; (+) or (-) signs indicate second (nested) round of RT-PCR screening reactions. The screening RT-PCR primers are designed to either the RdRp or the capsid region of porcine astrovirus 3 (Fig.1). The results of RT-(n)PCR screening reactions refer to a mixture of tissues embedded into the total of seven paraffin blocks. ISH +: PoAstV-3 ISH positive tissue samples, ISH -: PoAstV-3 ISH negative samples.**

| Farm ID | Animal ID | FFPE block ID | RdRp RT-(n)PCR | Capsid RT-(n)PCR | Tissue samples | ISH |
|---|---|---|---|---|---|---|
| GD | GD-1 | GD-1A | -(+) | -(+) | spinal cord | + |
| | | | | | brainstem | + |
| | | | | | cerebellum | + |
| | | | | | medulla | - |
| | | | | | oblongata | |
| | GD-2 | GD-2A | -(-) | -(-) | lymph node | - |
| | | | | | tonsil | - |
| | | | | | myocardium | - |
| | | | | | spleen | - |
| | | | | | thymus | - |
| | GD-7 | GD-7A | -(+) | -(+) | brainstem | + |
| | | | | | cerebellum | + |
| Tázlár | TAZ-1 | TAZ-1A | -(+) | -(+) | hippocampus | - |
| | | | | | brainstem | + |
| | | TAZ-1B | -(+) | -(+) | spinal cord | + |
| Balmazújváros | BAM-1 | BAM-1A | -(-) | -(-) | spinal cord | - |
| | | BAM-1B | -(-) | -(-) | brainstem | + |
| | | | | | cerebellum | + |

**Table 3: Amino acid (aa) differences of the "neuroinvasive" ("Ni") study strains of porcine astrovirus 3 (PoAstV-3): NI-Brain/9-2016a/HUN (KY073230), NI-Brain/173-2016a/HUN (KY073231) and NI-Brain/386-2015/HUN (KY073232) compared to the available PoAstV-3 strains detectable only from faecal samples (referred as "enteric" or "Ent" strains): JX556691, LC201595-7, LC201599. AD: Presumed particle assembly domain, RID: Presumed Receptor-Interaction domain.**

| genomic region | ORF1a | ORF1a | ORF1a | ORF1a | ORF1b | ORF1b | ORF2 | ORF2 | ORF2 | ORF2 |
|---|---|---|---|---|---|---|---|---|---|---|
| aa position | 1-400 | 1-400 | 401-844 | 401-844 | 1-508 | 1-508 | 1-415 (AD) | 1-415 (AD) | 416-754 (RID) | 416-754 (RID) |
| PoAstV-3 type | "Ni" | "Ent" | "Ni" | "Ent" | "Ni" | "Ent" | "Ni" | "Ent" | "Ni" | "Ent" |
| aa changes | M25 | S/L | F408 | L | N54 | D | R29N | KT[I/A/ V] | L439 | H[P/V] |
| | Y41 | F | I434 | V | D106G | [A/E]D | S34 | R | S453 | D |
| | R117 | K | S481 | P | A181 | S | R38 | Y | F457 | Y |
| | T120 | S/L | S576 | T/V | I206 | V | V55 | T | Y559 | F |
| | T122 | S/L | G608 | N | R213 | K | T57R | SK | A570[P] | N |
| | K151G | RC | N646 | H | Y293 | H/N | T61 | A | N572[Y] | D |
| | L170 | M | E679 | D | E343 | D | | | D581 | N |
| | L179 | M | | | K375 | R | | | I601 | V |
| | M185 | L | | | I378 | T | | | S617 | N |
| | D208 | E/N | | | N382 | D | | | T628 | S |
| | D202S[P/ Q]- | NPTD | | | I415 | A/T | | | S678 | T |
| | - | G | | | | | | | | |
| | P217a | TT | | | | | | | 1696 | V |
| | T220[V/A ] | IS | | | | | | | | |
| | P224 | H/R | | | | | | | | |
| | 1299 | V | | | | | | | | |
| | E332 | D | | | | | | | | |
| | V338 | L/I | | | | | | | | |
| | L346 | F | | | | | | | | |
| | 1369 | V | | | | | | | | |

## Claims

1. An isolated porcine astrovirus comprising
(a) a nucleotide sequence as set forth in SEQ ID NO: 1 or SEQ ID NO: 2 or SEQ ID NO: 3 or SEQ ID NO: 4, or the complementary strand thereof, and/or
(b) an ORF2 nucleic acid sequence selected from nucleotide residues nucleotide residues 4075 to 6339 of anyone of SEQ ID NOs: 1 to 3, SEQ ID NO: 4, or the complementary strand thereof, and/or
(c) an ORF2 nucleic acid sequence encoding an ORF2 polypeptide comprising or consisting of the amino acid sequence of anyone of SEQ ID NOs: 5 to 8 or the complementary strand thereof, and/or
(d) an ORF1a nucleic acid sequence selected from nucleotide residues 31 to 2559 of anyone of SEQ ID NOs: 1 to 3 or the complementary strand thereof, and/or
(e) an ORF1a nucleic acid sequence encoding an ORF1a polypeptide comprising or consisting of the amino acid sequence of anyone of SEQ ID NOs: 9 to 11 or the complementary strand thereof, and/or
(f) an ORFlab nucleic acid sequence selected from nucleotide residues 31 to 4085 of anyone of SEQ ID NOs: 1 to 3 or the complementary strand thereof, and/or
(g) an ORFlab nucleic acid sequence encoding an ORFlab polypeptide comprising or consisting of the amino acid sequence of anyone of SEQ ID NOs: 12 to 14 or the complementary strand thereof, or
(h) an ORF1b nucleic acid sequence selected from nucleotide residues 2559 to 4085 of anyone of SEQ ID NOs: 1 to 3 or the complementary strand thereof, and/or
(i) an ORF1b nucleic acid sequence encoding an ORF1b polypeptide comprising or consisting of the amino acid sequence of anyone of SEQ ID NOs: 15 to 17 or the complementary strand thereof, or
(j) a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% to a nucleotide sequence of anyone of (a) to (i), or
(k) a variant sequence of anyone of (a) to (j) within the degeneration of the genetic code.

2. The virus of claim 1, which comprises SEQ ID NO: 1 or SEQ ID NO: 2 or SEQ ID NO: 3 or SEQ ID NO: 4, or the complementary strand thereof, or a nucleotide sequence having at least 98% sequence homology therewith.

3. The virus of claim 1, which comprises a nucleotide sequence encoding a polypeptide as set forth in anyone of SEQ ID NOs: 5 to 8, or the complementary strand thereof, or a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% thereto, or a variant thereof within the degeneration of the genetic code.

4. The virus according to any one of claims 1 to 3, which is in attenuated form.

5. The virus according to any one of claims 1 to 3, which is in inactivated form.

6. A method of preparing a vaccine comprising a step of attenuating or killing a virus according to any one of claims 1 to 3 and, optionally, formulating the attenuated or inactivated virus in a suitable excipient.

7. A host cell containing a virus of any one of claims 1 to 4.

8. Use of a host cell of claim 7 for propagating a porcine astrovirus of any one of claims 1 to 4.

9. A method of producing a porcine astrovirus, comprising seeding a competent host cell with a seed of culture of a virus of any one of claims 1 to 4, growing the cells under suitable conditions in a manner allowing production of the virus, and harvesting the porcine astrovirus from said cells.

10. The porcine astrovirus obtained or obtainable by the method of claim 9.

11. A nucleic acid molecule comprising
(a) a nucleotide sequence as set forth in SEQ ID NO: 1 or SEQ ID NO: 2 or SEQ ID NO: 3 or SEQ ID NO: 4, or
(b) a nucleotide sequence comprising or consisting of nucleotide residues 4075 to 6339 of anyone of SEQ ID NOs: 1 to 3, or nucleotide residues 31 to 2559 of anyone of SEQ ID NOs: 1 to 3, or nucleotide residues 31 to 4085 of anyone of SEQ ID NOs: 1 to 3, or nucleotide residues 2559 to 4085 of anyone of SEQ ID NOs: 1 to 3, or the complementary strand thereof, or
(c) a nucleic acid sequence encoding a polypeptide comprising or consisting of the amino acid sequence of SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15, or SEQ ID NO: 16, or SEQ ID NO: 17, or the complementary strand thereof, or
(d) a nucleotide sequence having a homology of at least 87%, at least 88%, at least 89%, at least 90%, at least 91%, at least 92%, at least 93%, at least 94%, at least 95%, at least 96%, at least 97%, at least 98%, or at least 99% to a nucleotide sequence of (a), (b) or (c), or
(e) a variant sequence of any of (a) to (d) within the degeneration of the genetic code, or
(f) a fragment of a sequence of any of (a) to (e) of at least 10 consecutive nucleotides.

12. The nucleic acid molecule of claim 11, wherein the nucleotide sequence is functionally linked to a promoter sequence, such as a homologous or a heterologous promoter, and eventually a cis-acting transcription regulatory sequence.

13. A polypeptide encoded by a nucleic acid molecule according to claim 11.

14. A polypeptide selected from:
(a) a polypeptide comprising or consisting of the amino acid sequence of anyone of SEQ ID NOs: 5 to 17,
(b) a polypeptide having a homology of at least 90%, at least 95%, at least 96%, at least 97%, at least 98%, or at least 99% to an amino acid sequence of (a), or
(c) a variant sequence of any of (a) or (b) containing conservative amino acid modification(s), or
(d) a fragment of a sequence of any of (a) to (c) of at least 5, preferably at keast 6, at least 7, at least 8, at least 9, or at least 10 consecutive amino acids.

15. A polypeptide comprising at least 95% amino acid sequence identity to anyone of SEQ ID NOs 5 to 8.

16. A vector, virus, plasmid or host cell comprising a nucleic acid molecule according to claim 11 or 12.

17. A composition comprising a virus according to any one of claims 1 to 5 or 10, a subunit thereof, a polypeptide of anyone of claims 13 to 15, a vector, virus, plasmid or host cell of claim 16, or a cell of claim 7, and a veterinary acceptable vehicle or excipient.

18. The composition of claim 17, further comprising an adjuvant, such as a light paraffin oil, carbomer, aluminium hydroxyde, saponin, avridine (N,N-dioctadecyl-N',N'-bis(2-hydroxyethyl)-propanediamine), DMRIE or DDA, a pharmaceutically acceptable carrier, and/or a cytokine.

19. The composition of claim 17 or 18, for use for treating and/or preventing a porcine astrovirus associated disease.

20. The composition of claim 17 or 18, for use for vaccinating a porcine.

21. The composition of any one of claims 17 or 18, wherein the composition further comprises an additional immunogenic active component effective against another disease-causing organism in swine.

22. The composition of claim 21, further comprising an effective amount of PCV2 subtype A virus.

23. The composition of claim 21, further comprising at least one immunogen from at least one additional pig pathogen chosen among Actinobacillus pleuropneunomia; Adenovirus; Alphavirus such as Eastern equine encephalomyelitis viruses; Balantidium coli; Bordetella bronchiseptica; Brachyspira spp., preferably B. hyodyentheriae, B.pilosicoli, B. innocens, Brucella suis, preferably biovars 1, 2 and 3; Classical swine fever virus, African swine fever virus; Chlamydia and Chlamydophila sp. and preferably C. pecorum and C. abortus; Clostridium spp., preferably Cl. difficile, Cl. perfringens types A,B and C, Cl.novyi, Cl.septicum, Cl.tetani; Digestive and respiratory Coronavirus; Cryptosporidium parvum; Eimeria spp; Eperythrozoonis suis currently named Mycoplasma haemosuis; Erysipelothrix rhusiopathiae; Escherichia coli; Haemophilus parasuis, preferably subtypes 1,7 and 14; Hemagglutinating encephalomyelitis virus; Isospora suis ; Japanese Encephalitis virus; Lawsonia intracellularis; Leptospira spp., preferably Leptospira australis, Leptospira canicola, Leptospira grippotyphosa, Leptospira icterohaemorrhagicae, Leptospira interrogans, Leptospira Pomona and Leptospira tarassovi; Mannheimia haemolytica ; Mycobacterium spp. preferably, M.avium, M. intracellulare and M.bovis: Mycoplasma hyponeumoniae; Parvovirus ; Pasteurella multocida; Porcine cytomegolovirus; Porcine parovirus, Porcine reproductive and respiratory syndrome virus: Pseudorabies virus; Rotavirus; Sagiyama virus ; Salmonella spp. preferably, S. thyhimurium and S.choleraesuis; Staphylococcus spp. preferably, S. hyicus; Streptococcus spp., preferably Strep. suis; Swine cytomegalovirus ; Swine herpes virus; Swine influenza virus; Swine pox virus; Toxoplasma gondii; Vesicular stomatitis virus and virus of exanthema of swine; or other isolates and subtypes of porcine circovirus.

24. A container comprising an immunologically effective amount the composition according to any one of claims 17 to 18.

25. A kit comprising the container of claim 24 and an instruction manual including information for the administration of the immunologically effective amount the composition into pigs for treating and/or preventing porcine astrovirus-associated diseases.

26. A nucleic acid probe or primer characteristic of a nucleotide sequence as set forth in anyone of SEQ ID NO: 1-4 or a fragment thereof.

27. An antibody or a derivative thereof which is capable of binding to a virus as defined in any one of claims 1-5 or sub-units thereof.

28. A method of diagnosing the presence of a virus in an animal comprising contacting a tissue or fluid sample with a probe, primer or antibody as defined in anyone of claims 26 to 27, and detecting the presence of a virus.
